# EUROPEAN PATENT APPLICATION

(11) **EP 2 684 878 A1**
(43) Date of publication of application: **15.01.2014**
(21) Application number: 12754741.2
(22) Date of filing: 08.03.2012
(51) Int. Cl.: C07D 401/14, A61K 31/444, A61K 31/506, A61P 3/10, A61P 9/00, A61P 9/10, A61P 13/12, A61P 25/02, A61P 27/02, A61P 43/00, C07D 413/14

(54) **DIPYRIDYLAMINE DERIVATIVE**

(30) Priority: 09.03.2011 JP 2011051178; 04.11.2011 JP 2011242421
(71) Applicant: Daiichi Sankyo Company, Limited, Tokyo 103-8426 (JP)
(72) Inventor: KANEKO, Satoru, Tokyo 140-8710 (JP); TSURUOKA, Hiroyuki, Tokyo 140-8710 (JP); HONZUMI, Masatoshi, okyo 140-8710 (JP); ABE, Manabu, Tokyo 140-8710 (JP); YOSHIDA, Taishi, Tokyo 140-8710 (JP)
(74) Representative: Fairbairn, Angus Chisholm
(86) International application number: PCT/JP2012/055905
(87) International publication number: WO 2012/121314

(57) **Abstract**

The present invention relates to a compound or a pharmacologically acceptable salt thereof having superior glucokinase activating activity, and is a compound represented by general formula (I), or pharmacologically acceptable salt thereof: 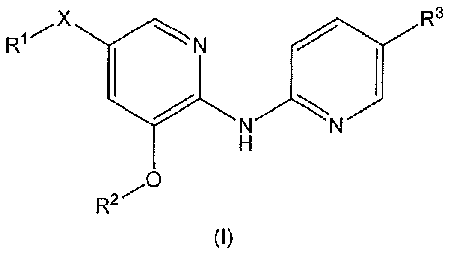 [wherein,
R² represents a C₆-C₁₀ aryl group that may be substituted with 1 to 5 group(s) independently selected from Substituent Group A, a C₁-C₆ alkyl group that may be substituted with 1 to 5 group(s) independently selected from Substituent Group B, or the like; X represents a single bond, an oxygen atom, a sulfur atom, or the like; R² represents a C₆-C₁₀ aryl group that may be substituted with 1 to 5 group(s) independently selected from Substituent Group A, or the like; R³ represents a 1H-tetrazol-5-yl group or a 5-oxo-4,5-dihydro-[1,2,4]oxadiazol-3-yl group; Substituent Group A represents the group of substituents selected from a halogen atom, a C₁-C₆ alkyl group, a C₁-C₆ halogenated alkyl group, a hydroxy group, a C₁-C₆ hydroxyalkyl group, or the like; and, Substituent Group B represents the group of substituents selected from a halogen atom, a C₃-C₆ cycloalkyl group that may be substituted with one C₁-C₆ hydroxyalkyl group, a hydroxy group, a C₁-C₆ alkoxy group, a C₂-C₇ alkylcarbonyl group, a C₂-C₇ alkoxycarbonyl group, or the like].

## Description

### TECHNICAL FIELD

The present invention relates to a compound, or a pharmacologically acceptable salt thereof, that has superior glucokinase activating activity and is useful as a therapeutic for diabetes and the like.

### BACKGROUND ART

Glucokinase (abbreviated as GK in the present description; EC 2.7.1.1) is one of the four types of hexokinases (hexokinase IV) found in mammals. Hexokinases are enzymes that catalyze the conversion of glucose to glucose-6-phosphate in the first stage of the glycolysis system in cells, and the expression of GK is localized mainly in the liver and pancreatic beta cells. In pancreatic beta cells, GK functions as a detection mechanism of extracellular glucose concentration that regulates glucose-stimulated insulin secretion, while in the liver, the enzymatic reaction of GK serves as the rate-limiting step to regulate subsequent reactions such as glycolysis and glycogen synthesis. Although GK found in the liver and that found in pancreatic beta cells differ in the sequence consisting of 15 amino acids from the N-terminal due to a difference in splicing, their enzymatic properties are identical. In contrast to the enzymatic activities of the three types of hexokinases other than GK (types I, II and III) becoming saturated at a glucose concentration of 1 mM or less, GK exhibits low affinity for glucose, and the Km value thereof is near that of the physiological level of glucose in the blood at 8 to 15 mM. Thus, acceleration of GK-mediated intracellular glucose metabolism occurs in response to changes in blood glucose levels ranging from normal blood glucose levels (about 5 mM) to postprandial blood glucose levels (10 to 15 mM).

The hypothesis that GK functions as a glucose sensor in the liver and pancreatic beta cells has long been advocated (Non-Patent Documents 1 to 3). Recent research findings have demonstrated that GK actually plays an important role in maintaining systemic glucose homeostasis, thereby verifying this hypothesis. For example, mice in which glucokinase gene had been disrupted exhibited prominent hyperglycemia symptoms and died soon after birth, while GK hetero-deficient mice were observed to have defective glucose tolerance and impaired glucose-stimulated insulin secretion (Non-Patent Document 4). On the other hand, normal mice excessively expressing GK were observed to demonstrate decreased blood glucose levels and increased glycogen content in the liver, and these phenomena were similar to those in mice in which diabetes was artificially induced (Non-Patent Document 5).

In addition, GK also functions as a glucose sensor in humans, and has been demonstrated by recent research to play an important role in maintaining glucose homeostasis. Abnormalities have been discovered in the GK genes of family lineages exhibiting a form of juvenile-onset diabetes referred to as maturity onset diabetes of the young (MODY2), and a correlation was clearly observed between these cases and GK activity (Non-Patent Document 6). On the other hand, family lineages have also been found that possess a mutation that increases GK activity, and symptoms of fasting hypoglycemia accompanied by elevated plasma insulin concentrations have been observed in such family lineages (Non-Patent Document 7). On the basis of these reports, GK plays an important role in blood glucose regulation by functioning as a glucose sensor in mammals, including humans. Thus, substances having GK activating activity are considered to be useful as drugs for treatment of glycometabolic diseases including type II diabetes mellitus. Since GK activating substances can be expected to simultaneously demonstrate glucose uptake promoting activity and glucose release inhibitory activity in the liver as well as insulin secretion promoting activity in pancreatic beta cells in particular, they are predicted to be able to demonstrate potent therapeutic effects unable to be attained with existing drugs.

Pancreatic beta cell type GK has recently been determined to be expressed, being localized in the ventromedial hypothalamus (VMH) of the rat brain. The VMH has been conventionally known to be the site of neurons that respond to glucose concentration. In contrast to food intake decreasing when glucose is administered to rat ventricle in the brain, food intake is accelerated when glucose metabolism is inhibited by administration of the glucose analogue, glucosamine (Non-Patent Document 8). Electrophysiological experiments have demonstrated that glucose-responsive neurons are activated by responding to physiological changes in glucose concentrations (5 to 20 mM), and glucokinase has been determined to similarly function as a glucose sensor in peripheral tissue (Non-Patent Document 9). Thus, substances that give rise to glucokinase activation not only in the liver and pancreatic beta cells, but also in the VMH can be expected to demonstrate blood glucose lowering activity as well as activity that corrects obesity, which is considered to be a problem associated with numerous patients of type II diabetes mellitus.

On the basis of the aforementioned descriptions, substances having GK activating activity are useful as diabetes therapeutics and preventives, or as therapeutics and preventives of chronic complications of diabetes, including diabetic retinopathy, diabetic nephropathy, diabetic neuropathy, ischemic heart disease and arteriosclerosis.

As a 2-aminopyridine-containing compound having GK activating ability, those having a 2-(pyridin-2-yl)aminopyridine moiety, and the like are described in Patent Document 1. In these compounds, one pyridine ring has a substituent(s) at the 5-position (and 3-position), while the other pyridine ring does not have a substituent. On the other hand, in the compounds included in the present application, one pyridine ring has substituents at the 3-position and 5-position, while the other pyridine ring has a substituent at the 5-position. In addition, Patent Documents 2 to 7 describe compounds having a 2-(thiazol-2-yl)aminopyridine or 2-([1,2,4]thiadiazol-5-yl)aminopyridine moiety, and the like.

### Prior art documents

### [Patent Documents]

[Patent Document 1] International Publication No. WO2009/046784 Pamphlet
[Patent Document 2] International Publication No. WO2007/053345 Pamphlet
[Patent Document 3] International Publication No. WO2007/089512 Pamphlet
[Patent Document 4] International Publication No. WO2007/117381 Pamphlet
[Patent Document 5] International Publication No. WO2008/091770 Pamphlet
[Patent Document 6] International Publication No. WO2008/118718 Pamphlet
[Patent Document 7] International Publication No. WO2009/042435 Pamphlet

### [Non-Patent Documents]

[Non-Patent Document 1] Am J Physiol. 1984 Sep; 247(3 Pt 2):R527-36.
[Non-Patent Document 2] Diabetes. 1986 Jan; 35(1):61-7.
[Non-Patent Document 3] Diabetes. 1986 Oct; 35(10):1163-73.
[Non-Patent Document 4] Cell. 1995 Oct 6; 83(1):69-78.
[Non-Patent Document 5] Proc Natl Acad Sci U S A. 1996 Jul 9; 93(14):7225-30.
[Non-Patent Document 6] Nature. 1992 Apr 23; 356(6371):721-2.
[Non-Patent Document 7] N Engl J Med. 1998 Jan 22; 338(4):226-30.
[Non-Patent Document 8] Life Sci. 1985 Dec 30; 37(26):2475-82.
[Non-Patent Document 9] Diabetes. 2006 Feb; 55(2):412-20. Erratum in: Diabetes. 2006 Mar; 55(3):862.

### SUMMARY OF THE INVENTION

### [Problems to be Solved by the Invention]

An object of the present invention is to provide a dipyridylamine derivative and a GK activator that uses this dipyridylamine derivative, and particularly to provide a therapeutic and preventive of diabetes and impaired glucose tolerance. As a result of conducting extensive studies on compounds having GK activating activity, the inventors of the present invention found that a nitrogen-containing aromatic ring compound having a specific chemical structure has superior GK activating activity. In addition, the compound of the present invention has superior GK selectivity, low toxicity and few adverse side effects. The inventors of the present invention also found that this nitrogen-containing aromatic ring compound is useful as an active ingredient of a pharmaceutical for the treatment and/or prevention of diseases selected from the group consisting of diabetes, impaired glucose tolerance, gestational diabetes, chronic complications of diabetes (including diabetic peripheral neuropathy, diabetic nephropathy, diabetic retinopathy and diabetic macroangiopathy) and metabolic syndrome. The present invention was completed based on the aforementioned findings.

In addition, the present compound was also found to be superior in terms of having a high degree of safety.

### [Means for Solving the Problems]

The present invention is:
(1) a compound represented by the general formula (I): 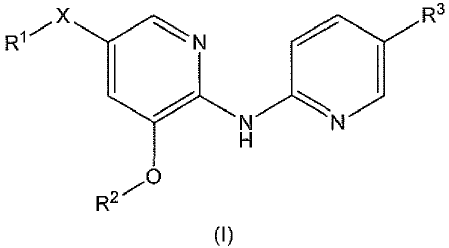 [wherein,
   R¹ represents a C₆-C₁₀ aryl group that may be substituted with 1 to 5 group(s) independently selected from Substituent Group A, a heterocyclic group that may be substituted with 1 to 4 group(s) independently selected from Substituent Group A, a C₃-C₆ cycloalkyl group that may be substituted with 1 to 4 group(s) independently selected from Substituent Group A or a C₁-C₆ alkyl group that may be substituted with 1 to 5 group(s) independently selected from Substituent Group B,
   X represents a single bond, an oxygen atom, a sulfur atom or a group represented by the formula -N(R⁴)- (wherein R⁴ represents a hydrogen atom or a C₁-C₆ alkyl group),
   with the proviso that the case in which X represents a single bond, and in which R¹ represents a C₆-C₁₀ aryl group that may be substituted with 1 to 5 group(s) independently selected from Substituent Group A or a heterocyclic group that may be substituted with 1 to 4 group(s) independently selected from Substituent Group A is excluded,
   R² represents a C₆-C₁₀ aryl group that may be substituted with 1 to 5 group(s) independently selected from Substituent Group A or a heterocyclic group that may be substituted with 1 to 4 group(s) independently selected from Substituent Group A,
   R³ represents a 1H-tetrazol-5-yl group or a 5-oxo-4,5-dihydro-[1,2,4]oxadiazol-3-yl group,

Substituent Group A represents the group of substituents selected from a halogen atom, a C₁-C₆ alkyl group, a C₁-C₆ halogenated alkyl group, a hydroxy group, a C₁-C₆ hydroxyalkyl group, a C₁-C₆ alkoxy group, a carboxyl group, a C₂-C₇ alkylcarbonyl group, a C₂-C₇ alkoxycarbonyl group, a C₂-C₇ alkylcarbonyloxy group, a cyano group, a nitro group, an amino group, a mono-C₁-C₆ alkylamino group, a di-(C₁-C₆ alkyl)amino group, a C₁-C₆ alkylsulfanyl group, a C₁-C₆ alkylsulfinyl group, a C₁-C₆ alkylsulfonyl group, a group represented by the formula-C(=O)-NR⁵R⁶ (wherein R⁵ and R⁶ may be the same or different and respectively represent a hydrogen atom or a C₁-C₆ alkyl group), and a group represented by the formula -NR⁷R⁸ (wherein R⁷ represents a hydrogen atom or a C₁-C₆ alkyl group, and R⁸ represents a C₂-C₇ alkylcarbonyl group, a C₁-C₆ alkylsulfinyl group or a C₁-C₆ alkylsulfonyl group), and

Substituent Group B represents the group of substituents selected from a halogen atom, a C₃-C₆ cycloalkyl group that may be substituted with one C₁-C₆ hydroxyalkyl group, a hydroxy group, a C₁-C₆ alkoxy group, a C₂-C₇ alkylcarbonyl group, a C₂-C₇ alkoxycarbonyl group, an amino group, a mono-C₁-C₆ alkylamino group, a di-(C₁-C₆ alkyl)amino group, a C₆-C₁₀ aryl group that may be substituted with 1 to 5 group(s) independently selected from Substituent Group A, and a heterocyclic group that may be substituted with 1 to 4 group(s) independently selected from Substituent Group A]
or a pharmacologically acceptable salt thereof.

The present invention may include preferably those matters indicated below.
(2) The compound or pharmacologically acceptable salt thereof in (1) above, wherein
   Substituent Group A is the group of substituents selected from a halogen atom, a C₁-C₆ alkyl group, a C₁-C₆ halogenated alkyl group, a C₁-C₆ hydroxyalkyl group, a C₂-C₇ alkoxycarbonyl group, a C₁-C₆ alkylsulfonyl group, and a group represented by the formula -C(=O)-NR⁵R⁶.
(3) The compound or pharmacologically acceptable salt thereof in (1) or (2) above, wherein
   Substituent Group B is the group of substituents selected from a halogen atom, a C₃-C₆ cycloalkyl group that may be substituted with one C₁-C₆ hydroxyalkyl group, a hydroxy group, a C₁-C₆ alkoxy group, a C₂-C₇ alkoxycarbonyl group, and a di-(C₁-C₆ alkyl)amino group.
(4) The compound or pharmacologically acceptable salt thereof in any one selected from (1) to (3) above, wherein R¹ is a pyridyl group that may be substituted with 1 to 3 group(s) independently selected from Substituent Group A, a pyrimidinyl group that may be substituted with 1 to 3 group(s) independently selected from Substituent Group A or a C₁-C₆ alkyl group that may be substituted with 1 to 5 group(s) independently selected from Substituent Group B.
(5) The compound or pharmacologically acceptable salt thereof in any one selected from (1) to (3) above, wherein R¹ is a 2-pyridyl group that may be substituted with 1 or 2 group(s) independently selected from the group consisting of (a C₁-C₆ alkyl group, a C₁-C₆ halogenated alkyl group, a C₁-C₆ hydroxyalkyl group and a C₂-C₇ alkoxycarbonyl group), a 2-pyrimidinyl group, or a C₁-C₆ alkyl group that may be substituted with 1 to 3 group(s) independently selected from the group consisting of (a halogen atom, a C₃-C₆ cycloalkyl group which may be substituted with one C₁-C₆ hydroxyalkyl group, a hydroxy group, a C₁-C₆ alkoxy group, a C₂-C₇ alkoxycarbonyl group and a di-(C₁-C₆ alkyl) amino group).
(6) The compound or pharmacologically acceptable salt thereof in any one selected from (1) to (3) above, wherein R¹ is a 2-pyridyl group, a 5-hydroxymethyl-2-pyridyl group, a 5-ethoxycarbonyl-2-pyridyl group, a 2-pyrimidinyl group, an isopropyl group, a cyclopropylmethyl group, a trifluoromethyl group, a 3-hydroxypropyl group, a 3-methoxypropyl group, a 3-methoxy-1-methylpropyl group, a 2-fluoro-3-methoxypropyl group or a 4-methoxybutyl group.
(7) The compound or pharmacologically acceptable salt thereof in any one selected from (1) to (3) above, wherein R¹ is a 2-pyridyl group, a 5-hydroxymethyl-2-pyridyl group, a 5-ethoxycarbonyl-2-pyridyl group, a 2-pyrimidinyl group, an isopropyl group, a cyclopropylmethyl group, a trifluoromethyl group, a 3-hydroxypropyl group, a 3-methoxypropyl group, a 3-methoxy-1-methylpropyl group or a 2-fluoro-3-methoxypropyl group.
(8) The compound or pharmacologically acceptable salt thereof in any one selected from (1) to (3) above, wherein R¹ is a cyclopropyl group or a 2-cyclopropylethyl group.
(9) The compound or pharmacologically acceptable salt thereof in any one selected from (1) to (8) above, wherein X is a single bond or a sulfur atom.
(10) The compound or pharmacologically acceptable salt thereof in any one selected from (1) to (9) above, wherein R² is a phenyl group that may be substituted with 1 to 3 group(s) independently selected from Substituent Group A.
(11) The compound or pharmacologically acceptable salt thereof in any one selected from (1) to (9) above, wherein R² is a phenyl group that is substituted with 1 or 2 group(s) independently selected from the group consisting of (a halogen atom, a C₁-C₆ halogenated alkyl group, a C₁-C₆ alkylsulfonyl group and a group represented by the formula -C(=O)-NR⁵R⁶).
(12) The compound or pharmacologically acceptable salt thereof in any one selected from (1) to (9) above, wherein R² is a 4-fluorophenyl group, a 2,4-difluorophenyl group, a 3,4-difluorophenyl group or a 2-chloro-4-fluorophenyl group.
(13) The compound or pharmacologically acceptable salt thereof in any one selected from (1) to (9) above, wherein R² is a 4-fluorophenyl group, a 2,4-difluorophenyl group or a 3,4-difluorophenyl group.
(14) The compound or pharmacologically acceptable salt thereof in any one selected from (1) to (13) above, wherein R³ is a 1H-tetrazol-5-yl group.
(15) The compound or pharmacologically acceptable salt thereof in (1) above, wherein
   R¹ is a pyridyl group that may be substituted with 1 to 3 group(s) independently selected from Substituent Group A, a pyrimidinyl group that may be substituted with 1 to 3 group(s) independently selected from Substituent Group A or a C₁-C₆ alkyl group that may be substituted with 1 to 5 group(s) independently selected from Substituent Group B, X is an oxygen atom or a sulfur atom, R² is a phenyl group that may be substituted with 1 to 3 group(s) independently selected from Substituent Group A, R³ is a 1H-tetrazol-5-yl group or a 5-oxo-4,5-dihydro-[1,2,4]oxadiazol-3-yl group, Substituent Group A is the group of substituents selected from a halogen atom, a C₁-C₆ alkyl group, a C₁-C₆ halogenated alkyl group, a C₁-C₆ hydroxyalkyl group, a C₂-C₇ alkoxycarbonyl group, a C₁-C₆ alkylsulfonyl group, and a group represented by the formula -C(=O)-NR⁵R⁶, and Substituent Group B is the group of substituents selected from a halogen atom, a C₃-C₆ cycloalkyl group which may be substituted with one C₁-C₆ hydroxyalkyl group, a hydroxy group, a C₁-C₆ alkoxy group, a C₂-C₇ alkoxycarbonyl group, and a di-(C₁-C₆ alkyl)amino group.
(16) The compound or pharmacologically acceptable salt thereof in (1) above, wherein R¹ is a 2-pyridyl group that may be substituted with 1 or 2 group(s) independently selected from the group consisting of (a C₁-C₆ alkyl group, a C₁-C₆ halogenated alkyl group, a C₁-C₆ hydroxyalkyl group and a C₂-C₇ alkoxycarbonyl group), a 2-pyrimidinyl group, or a C₁-C₆ alkyl group that may be substituted with 1 to 3 group(s) independently selected from the group consisting of (a halogen atom, a C₃-C₆ cycloalkyl group which may be substituted with one C₁-C₆ hydroxyalkyl group, a hydroxy group, a C₁-C₆ alkoxy group, a C₂-C₇ alkoxycarbonyl group and a di-(C₁-C₆ alkyl)amino group), X is a sulfur atom, R² is a phenyl group that is substituted with 1 or 2 group(s) independently selected from the group consisting of (a halogen atom, a C₁-C₆ halogenated alkyl group, a C₁-C₆ alkylsulfonyl group and a group represented by the formula -C(-O)-NR⁵R⁶), and R³ is a 1H-tetrazol-5-yl group.
(17) The compound or pharmacologically acceptable salt thereof in (1) above, wherein
   R¹ is a 2-pyridyl group, a 5-hydroxymethyl-2-pyridyl group, a 5-ethoxycarbonyl-2-pyridyl group, a 2-pyrimidinyl group, an isopropyl group, a cyclopropylmethyl group, a trifluoromethyl group, a 3-hydroxypropyl group, a 3-methoxypropyl group, a 3-methoxy-1-methylpropyl group or a 2-fluoro-3-methoxypropyl group, X is a single bond or a sulfur atom, R² is a 4-fluorophenyl group, a 2,4-difluorophenyl group or a 3,4-difluorophenyl group, and R³ is a 1H-tetrazol-5-yl group.
(18) The compound or pharmacologically acceptable salt thereof in (1) above, wherein
   R¹ is a 2-pyridyl group, a 5-hydroxymethyl-2-pyridyl group, a 5-ethoxycarbonyl-2-pyridyl group, a 2-pyrimidinyl group, an isopropyl group, a cyclopropylmethyl group, a trifluoromethyl group, a 3-hydroxypropyl group, a 3-methoxypropyl group, a 3-methoxy-1-methylpropyl group, a 2-fluoro-3-methoxypropyl group or a 4-methoxybutyl group, X is a single bond or a sulfur atom, R² is a 4-fluorophenyl group, a 2,4-difluorophenyl group, a 3,4-difluorophenyl group, a 2-chloro-4-fluorophenyl group, a cyclopropyl group or a 2-cyclopropylethyl group, and R³ is a 1H-tetrazol-5-yl group.
(19) The compound or pharmacologically acceptable salt thereof in (1) above, wherein
   R¹ is a 2-pyridyl group, a 5-hydroxymethyl-2-pyridyl group, a 5-ethoxycarbonyl-2-pyridyl group, a 2-pyrimidinyl group, an isopropyl group, a cyclopropylmethyl group, a trifluoromethyl group, a 3-hydroxypropyl group, a 3-methoxypropyl group, a 3-methoxy-1-methylpropyl group, a 2-fluoro-3-methoxypropyl group or a 4-methoxybutyl group, X is a single bond or a sulfur atom, R² is a 4-fluorophenyl group, a 2,4-difluorophenyl group, a 3,4-difluorophenyl group or a 2-chloro-4-fluorophenyl group, and R³ is a 1H-tetrazol-5-yl group.
(20) A compound or a pharmacologically acceptable salt thereof, which is:
   [3-(3,4-difluorophenoxy)-5-(pyridin-2-ylsulfanyl)pyridin-2-yl]-[5-(1H-tetrazol-5-yl)pyridin-2-yl]amine,
   [3-(4-fluorophenoxy)-5-(pyridin-2-ylsulfanyl)pyridin-2-yl]-[5-(1H-tetrazol-5-yl)pyridin-2-yl]amine,
   [3-(2,4-difluorophenoxy)-5-(pyridin-2-ylsulfanyl)pyridin-2-yl]-[5-(1H-tetrazol-5-yl)pyridin-2-yl]amine,
   [3-(3,4-difluorophenoxy)-5-(pyrimidin-2-ylsulfanyl)pyridin-2-yl]-[5-(1H-tetrazol-5-yl)pyridin-2-yl]amine,
   ethyl 6-{5-(3,4-difluorophenoxy)-6-[5-(1H-tetrazol-5-yl)pyridin-2-ylamino]pyridin-3-ylsulfanyl}nicotinate,
   6-{5-(3,4-difluorophenoxy)-6-[5-(1H-tetrazol-5-yl)pyridin-2-ylamino]pyridin-3-ylsulfanyl}pyridin-3-ylmethanol,
   [3-(4-fluorophenoxy)-5-[(3-methoxypropylsulfanyl)pyridin-2-yl]-[5-(1H-tetrazol-5-yl)pyridin-2-yl]amine,
   [5-(cyclopropylmethylsulfanyl)-3-(4-fluorophenoxy)pyridin-2-yl]-[5-(1H-tetrazol-5-yl)pyridin-2-yl]amine,
   [5-(2-fluoro-3-methoxypropylsulfanyl)-3-(4-fluorophenoxy)pyridin-2-yl]-[5-(1H-tetrazol-5-yl)pyridin-2-yl]amine,
   [3-(3,4-difluorophenoxy)-5-(3-methoxypropylsulfanyl)pyridin-2-yl]-[5-(1H-tetrazol-5-yl)pyridin-2-yl]amine,
   3-{5-(3,4-difluorophenoxy)-6-[5-(1H-tetrazol-5-yl)pyridin-2-ylamino]pyridin-3-ylsulfanyl}propan-1-ol,
   [3-(3,4-difluorophenoxy)-5-(1-methylethylsulfanyl)pyridin-2-yl]-[5-(1H-tetrazol-5-yl)pyridin-2-yl]amine,
   [3-(2,4-difluorophenoxy)-5-trifluoromethylpyridin-2-yl]-[5-(1H-tetrazol-5-yl)pyridin-2-yl]amine,
   [3-(2,4-difluorophenoxy)-5-(3-methoxypropylsulfanyl)pyridin-2-yl]-[5-(1H-tetrazol-5-yl)pyridin-2-yl]amine, or
   [3-(2,4-difluorophenoxy)-5-(3-methoxy-1-methylpropylsulfanyl)pyridin-2-yl]-[5-(1H-tetrazol-5-yl)pyridin-2-yl] amine.
(21) A compound among the compounds or the pharmacologically acceptable salt thereof described in (20) above.
(22) A compound or a pharmacologically acceptable salt thereof, which is:
   [3-(2,4-difluorophenoxy)-5-(pyridin-2-ylsulfanyl)pyridin-2-yl]-[5-(1H-tetrazol-5-yl)pyridin-2-yl]amine,
   ethyl 6-{5-(3,4-difluorophenoxy)-6-[5-(1H-tetrazol-5-yl)pyridin-2-ylamino]pyridin-3-ylsulfanyl}nicotinate,
   6-{5-(3,4-difluorophenoxy)-6-[5-(1H-tetrazol-5-yl)pyridin-2-ylamino]pyridin-3-ylsulfanyl}pyridin-3-ylmethanol,
   [3-(4-fluorophenoxy)-5-[(3-methoxypropylsulfanyl)pyridin-2-yl]-[5-(1H-tetrazol-5-yl)pyridin-2-yl]amine,
   [5-(cyclopropylmethylsulfanyl)-3-(4-fluorophenoxy)pyridin-2-yl]-[5-(1H-tetrazol-5-yl)pyridin-2-yl]amine,
   [3-(3,4-difluorophenoxy)-5-(3-methoxypropylsulfanyl)pyridin-2-yl]-[5-(1H-tetrazol-5-yl)pyridin-2-yl]amine,
   [3-(2,4-difluorophenoxy)-5-trifluoromethylpyridin-2-yl]-[5-(1H-tetrazol-5-yl)pyridin-2-yl]amine,
   [3-(2,4-difluorophenoxy)-5-(3-methoxypropylsulfanyl)pyridin-2-yl]-[5-(1H-tetrazol-5-yl)pyridin-2-yl]amine, or
   [3-(2,4-difluorophenoxy)-5-(3-methoxy-1-methylpropylsulfanyl)pyridin-2-yl]-[5-(1H-tetrazol-5-yl)pyridin-2-yl]amine.
(23) A compound among the compounds or the pharmacologically acceptable salt thereof described in (22) above.
(24) A compound or a pharmacologically acceptable salt thereof, which is:
   [3-(2,4-difluorophenoxy)-5-(4-methoxybutyl)pyridin-2-yl]-[5-(1H-tetrazol-5-yl)pyridin-2-yl]amine,
   [5-(2-cyclopropylethyl)-3-(2,4-difluorophenoxy)pyridin-2-yl]-[5-(1H-tetrazol-5-yl)pyridin-2-yl]amine,
   [3-(4-fluorophenoxy)-5-(trifluoromethyl)pyridin-2-yl]-[5-(1H-tetrazol-5-yl)pyridin-2-yl]amine,
   [3-(2,4-difluorophenoxy)-5-cyclopropylpyridin-2-yl]-[5-(1H-tetrazol-5-yl)pyridin-2-yl]amine,
   [3-(2-chloro-4-fluorophenoxy)-5-(trifluoromethyl)pyridin-2-yl]-[5-(1H-tetrazol-5-yl)pyridin-2-yl]amine, or
   [3-(3,4-difluorophenoxy)-5-(trifluoromethyl)pyridin-2-yl]-[5-(1H-tetrazol-5-yl)pyridin-2-yl]amine.
(25) A compound among the compounds or the pharmacologically acceptable salt thereof described in (24) above.
(26) A pharmaceutical composition containing as an active ingredient thereof a compound or pharmacologically acceptable salt thereof described in any one selected from (1) to (25) above.
(27) The pharmaceutical composition described in (26) above, wherein the pharmaceutical composition has glucokinase activating activity.
(28) The pharmaceutical composition described in (26) above, wherein the pharmaceutical composition is for treating and/or preventing a disease that is treatable and/or preventable by glucokinase activating activity.
(29) The pharmaceutical composition described in (26) above, wherein the pharmaceutical composition is for treating and/or preventing a disease for which the symptoms thereof are treated, improved, diminished and/or prevented by activation of glucokinase and maintenance of glucose homeostasis or regulation of blood glucose level.
(30) The pharmaceutical composition described in (26) above, wherein the pharmaceutical composition is for treating and/or preventing diabetes, impaired glucose tolerance, gestational diabetes, chronic complications of diabetes (including diabetic peripheral neuropathy, diabetic nephropathy, diabetic retinopathy and diabetic macroangiopathy) or metabolic syndrome.
(31) The pharmaceutical composition described in (26) above, wherein the pharmaceutical composition is for treating and/or preventing diabetes or impaired glucose tolerance.
(32) The pharmaceutical composition described in (26) above, wherein the pharmaceutical composition is for treating and/or preventing diabetes.
(33) The pharmaceutical composition described in (26) above, wherein the pharmaceutical composition is for treating and/or preventing impaired glucose tolerance.
(34) The pharmaceutical composition described in (26) above, wherein the pharmaceutical composition is for treating and/or preventing gestational diabetes.
(35) A glucokinase activator containing as an active ingredient thereof a compound or pharmacologically acceptable salt thereof described in any one selected from (1) to (25) above.
(36) A use of a compound or pharmacologically acceptable salt thereof described in any one selected from (1) to (25) above, for producing a pharmaceutical composition.
(37) The use described in (36) above, wherein the pharmaceutical composition is a composition for activating glucokinase.
(38) The use described in (36) above, wherein the pharmaceutical composition is a composition for treating and/or preventing diabetes, impaired glucose tolerance, gestational diabetes, chronic complications of diabetes (including diabetic peripheral neuropathy, diabetic nephropathy, diabetic retinopathy and diabetic macroangiopathy) or metabolic syndrome.
(39) The use described in (36) above, wherein the pharmaceutical composition is a composition for treating and/or preventing diabetes or impaired glucose tolerance.
(40) A glucokinase activation method, comprising administering a pharmacologically effective amount of a compound or pharmacologically acceptable salt thereof described in any one selected from (1) to (25) above to a warm-blooded animal.
(41) A method for treating and/or preventing a disease, comprising administering a pharmacologically effective amount of a compound or pharmacologically acceptable salt thereof described in any one selected from (1) to (25) above to a warm-blooded animal.
(42) The method described in (41) above, wherein the disease is diabetes, impaired glucose tolerance, gestational diabetes, chronic complications of diabetes (including diabetic peripheral neuropathy, diabetic nephropathy, diabetic retinopathy and diabetic macroangiopathy) or metabolic syndrome.
(43) The method described in (41) above, wherein the disease is diabetes or impaired glucose tolerance.
(44) The method described in (41) above, wherein the disease is diabetes.
(45) The method described in (41) above, wherein the disease is impaired glucose tolerance.
(46) The method described in (41) above, wherein the disease is gestational diabetes.
(47) The method described in any one selected from (40) to (46) above, wherein the warm-blooded animal is a human.

In the present invention, a "halogen atom" is a fluorine atom, a chlorine atom, a bromine atom or an iodine atom. The halogen atom is preferably a fluorine atom or a chlorine atom, and more preferably a fluorine atom.

In the present invention, a "C₁-C₆ alkyl group" is a linear or branched alkyl group having 1 to 6 carbon atom(s). Examples of the C₁-C₆ alkyl group include a methyl group, an ethyl group, a propyl group, an isopropyl group, a butyl group, an isobutyl group, an s-butyl group, a t-butyl group, a pentyl group, an isopentyl group, a 2-methylbutyl group, a neopentyl group, a 1-ethylpropyl group, a hexyl group, an isohexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group and a 1,2-dimethylbutyl group. In R¹, the C₁-C₆ alkyl group is preferably a linear or branched alkyl group having 1 to 3 carbon atom(s) (C₁-C₃ alkyl group), and more preferably a methyl group, a propyl group or an isopropyl group. In R⁴, R⁵, R⁶, R⁷ and Substituent Group A, the C₃-C₆ alkyl group is preferably a linear or branched alkyl group having 1 to 4 carbon atom(s) (C₁-C₄ alkyl group), more preferably a methyl group or an ethyl group (C₁-C₂ alkyl group), and even more preferably a methyl group.

In the present invention, a "C₁-C₆ halogenated alkyl group" is a group in which 1 to 5 of the same or different above-mentioned "halogen atom" are bonded to the above-mentioned "C₁-C₆ alkyl group". Examples of C₁-C₆ halogenated alkyl group include a trifluoromethyl group, a trichloromethyl group, a difluoromethyl group, a dichloromethyl group, a dibromomethyl group, a fluoromethyl group, a 2,2,2-trifluoroethyl group, a 2,2,2-trichloroethyl group, a 2-bromoethyl group, a 2-chloroethyl group or a 2-fluoroethyl group. The C₁-C₆ halogenated alkyl group is preferably a group in which 1 to 5 of the same or different "halogen atom" are bonded to a "C₁-C₄ alkyl group" (C₁-C₄ halogenated alkyl group), more preferably a group in which 1 to 5 of the same or different "halogen atom" are bonded to a "C₁-C₂ alkyl group" (C₁-C₂ halogenated alkyl group), and even more preferably a trifluoromethyl group.

In the present invention, a "C₁-C₆ hydroxyalkyl group" is a group in which one hydroxy group is bonded to the above-mentioned "C₁-C₆ alkyl group". Examples of the C₁-C₆ hydroxyalkyl group include a hydroxymethyl group, a 2-hydroxyethyl group, a 1-hydroxyethyl group or a 3-hydroxypropyl group. The "C₁-C₆ hydroxyalkyl group" is preferably a group in which one hydroxy group is bonded to a "C₁-C₂ alkyl group", and more preferably a hydroxymethyl group.

In the present invention, a "C₁-C₆ alkoxy group" is a group in which the above-mentioned "C₁-C₆ alkyl group" is bonded to an oxygen atom, and is a linear or branched alkoxy group having 1 to 6 carbon atom(s). Examples of the C₁-C₆ alkoxy group include a methoxy group, an ethoxy group, a propoxy group, an isopropoxy group, a butoxy group, an isobutoxy group, an s-butoxy group, a t-butoxy group, a pentoxy group, a 2-methylbutoxy group, a 3-ethylpropoxy group, a neopentoxy group, a hexyloxy group or a 2,3-dimethylbutoxy group. The "C₁-C₆ alkoxy group" is preferably a linear or branched alkoxy group having 1 to 4 carbon atom(s) (C₁-C₄ alkoxy group), more preferably a methoxy group or an ethoxy group (C₁-C₂ alkoxy group), and even more preferably a methoxy group.

In the present invention, a "C₂-C₇ alkylcarbonyl group" is a group in which one above-mentioned "C₁-C₆ alkyl group" is bonded to a carbonyl group. Examples of the C₂-C₇ alkylcarbonyl group include an acetyl group, a propionyl group, a butyryl group, an isobutyryl group, a pentanoyl group, a pivaloyl group or a valeryl group. The "C₂-C₇ alkylcarbonyl group" is preferably a group in which one "C₁-C₄ alkyl group" is bonded to a carbonyl group (C₂-C₅ alkylcarbonyl group), more preferably an acetyl group or a propionyl group (C₂-C₃ alkylcarbonyl group), and even more preferably a propionyl group.

In the present invention, a "C₂-C₇ alkoxycarbonyl group" is a group in which one above-mentioned "C₁-C₆ alkoxy group" is bonded to a carbonyl group. Examples of the C₂-C₇ alkoxycarbonyl group include a methoxycarbonyl group, an ethoxycarbonyl group, a propoxycarbonyl group, an isopropoxycarbonyl group, a butoxycarbonyl group, an isobutoxycarbonyl group, an s-butoxycarbonyl group or a t-butoxycarbonyl group. The "C₂-C₇ alkoxycarbonyl group" is preferably a group in which one "C₁-C₄ alkoxy group" is bonded to a carbonyl group (C₂-C₅ alkoxycarbonyl group), more preferably a methoxycarbonyl group or an ethoxycarbonyl group (C₂-C₃ alkoxycarbonyl group), and even more preferably an ethoxycarbonyl group.

In the present invention, a "C₂-C₇ alkylcarbonyloxy group" is a group in which a carbonyl group to which is bonded one above-mentioned "C₁-C₆ alkyl group" is bonded to an oxygen atom. Examples of the C₂-C₇ alkylcarbonyloxy group include an acetoxy group, a propionyloxy group, a butyryloxy group, an isobutyryloxy group, a pentanoyloxy group, a pivaloyloxy group, a valeryloxy group or an isovaleryloxy group. The C₂-C₇ alkylcarbonyloxy group is preferably a group in which a carbonyl group to which is bonded one "C₁-C₄ alkyl group" is bonded to an oxygen atom (C₂-C₅ alkylcarbonyloxy group), more preferably an acetoxy group or a propionyloxy group (C₂-C₃ alkylcarbonyloxy group), and even more preferably a propionyloxy group.

In the present invention, a "mono-C₁-C₆ alkylamino group" is a group in which one above-mentioned "C₁-C₆ alkyl group" is bonded to an amino group. Examples of the mono-C₁-C₆ alkylamino group include a methylamino group, an ethylamino group, a propylamino group, an isopropylamino group or a butylamino group. The mono-C₁-C₆ alkylamino group is preferably a group in which one "C₁-C₄ alkyl group" is bonded to an amino group (mono-C₁-C₄ alkylamino group), more preferably a methylamino group or an ethylamino group (mono-C₁-C₂ alkylamino group), and even more preferably a methylamino group.

In the present invention, a "di-(C₁-C₆ alkyl)amino group" is a group in which two of the same or different above-mentioned "C₁-C₆ alkyl group" is bonded to an amino group. Examples of the di-(C₁-C₆ alkyl)amino group include a dimethylamino group, a diethylamino group, a dipropylamino group, an N-ethyl-N-methylamino group, an N-methyl-N-propylamino group or an N-butyl-N-methylamino group. The "di-(C₁-C₆ alkyl)amino group" is preferably a group in which two of the same or different "C₁-C₄ alkyl group" is bonded to an amino group (di-(C₁-C₄ alkyl)amino group), more preferably a dimethylamino group, a diethylamino group or an N-ethyl-N-methylamino group (di-(C₁-C₂ alkyl)amino group), and even more preferably a dimethylamino group.

In the present invention, a "C₁-C₆ alkylsulfanyl group" is a group in which one above-mentioned "C₁-C₆ alkyl group" is bonded to a sulfur atom. Examples of the C₁-C₆ alkylsulfanyl group include a methylsulfanyl group, an ethylsulfanyl group, a propylsulfanyl group, an isopropylsulfanyl group, a butylsulfanyl group or a hexylsulfanyl group. The "C₁-C₆ alkylsulfanyl group" is preferably a linear or branched alkylsulfanyl group having 1 to 4 carbon atom(s) (C₁-C₄ alkylsulfanyl group), more preferably a methylsulfanyl group or an ethylsulfanyl group (C₁-C₂ alkylsulfanyl group), and even more preferably a methylsulfanyl group.

In the present invention, a "C₁-C₆ alkylsulfinyl group" is a group in which one above-mentioned "C₁-C₆ alkyl group" is bonded to a sulfinyl group. Examples of the C₁-C₆ alkylsulfinyl group include a methylsulfinyl group, an ethylsulfinyl group, a propylsulfinyl group, an isopropylsulfinyl group, a butylsulfinyl group or a hexylsulfinyl group. The "C₁-C₆ alkylsulfinyl group" is preferably a linear or branched alkylsulfinyl group having 1 to 4 carbon atom(s) (C₁-C₄ alkylsulfinyl group), more preferably a methylsulfinyl group or an ethylsulfinyl group (C₁-C₂ alkylsulfinyl group), and even more preferably a methylsulfinyl group.

In the present invention, a "C₁-C₆ alkylsulfonyl group" is a group in which one above-mentioned "C₁-C₆ alkyl group" is bonded to a sulfonyl group. Examples of the C₁-C₆ alkylsulfonyl group include a methylsulfonyl group, an ethylsulfonyl group, a propylsulfonyl, an isopropylsulfonyl group or a hexylsulfonyl group. The "C₁-C₆ alkylsulfonyl group" is preferably a linear or branched alkylsulfonyl group having 1 to 4 carbon atom(s) (C₁-C₄ alkylsulfonyl group), more preferably a methylsulfonyl group or an ethylsulfonyl group (C₁-C₂ alkylsulfonyl group), and even more preferably a methylsulfonyl group.

In the present invention, a "group represented by the formula -C(=O)-NR⁵R⁶ (wherein R⁵ and R⁶ may be the same or different and respectively represent a hydrogen atom or C₁-C₆ alkyl group)" is a carbamoyl group, a "mono-C₁-C₆ alkylaminocarbonyl group (a group in which an amino group to which is bonded one above-mentioned "C₁-C₆ alkyl group" is bonded to a carbonyl group)", or a "di-(C₁-C₆ alkyl)aminocarbonyl group (a group in which an amino group to which are bonded two of the same or different above-mentioned "C₁-C₆ alkyl group" is bonded to a carbonyl group)". Examples of the group represented by the formula -C(=O)-NR⁵R⁶ include a carbamoyl group, a methylaminocarbonyl group, an ethylaminocarbonyl group, a propylaminocarbonyl group, a dimethylaminocarbonyl group, a diethylaminocarbonyl group, a dipropylaminocarbonyl group, an N-ethyl-N-methylaminocarbonyl group or an N-methyl-N-propylaminocarbonyl group. The "group represented by the formula -C(=O)-NR⁵R⁶" is preferably a carbamoyl group, a methylaminocarbonyl group or a dimethylaminocarbonyl group, and more preferably a dimethylaminocarbonyl group.

In the present invention, a "group represented by the formula -NR⁷R⁸ (wherein R⁷ represents a hydrogen atom or a C₁-C₆ alkyl group, and R⁸ represents a C₂-C₇ alkylcarbonyl group, a C₁-C₆ alkylsulfinyl group or a C₁-C₆ alkylsulfonyl group)" is a "mono-C₂-C₇ alkylcarbonylamino group (a group in which a carbonyl group to which is bonded one above-mentioned "C₁-C₆ alkyl group" is bonded to an amino group)", a "mono-C₂-C₇ alkylsulfinylamino group (a group in which a sulfinyl group to which is bonded one above-mentioned "C₁-C₆ alkyl group" is bonded to an amino group)", a "mono-C₂-C₇ alkylsulfonylamino group (a group in which a sulfonyl group to which is bonded one above-mentioned "C₁-C₆ alkyl group" is bonded to an amino group)", a "C₂-C₇ alkylcarbonyl(C₁-C₆ alkyl)amino group (a group in which a carbonyl group to which is bonded one above-mentioned "C₁-C₆ alkyl group" is bonded to the above-mentioned "mono-C₁-C₆ alkylamino group")", a "C₂-C₇ alkylsulfinyl(C₁-C₆ alkyl)amino group (a group in which a sulfinyl group to which is bonded one above-mentioned "C₁-C₆ alkyl group" is bonded to the above-mentioned "mono-C₁-C₆ alkylamino group")", or a "C₂-C₇ alkylsulfonyl(C₁-C₆ alkyl)amino group (a group in which a sulfonyl group to which is bonded one above-mentioned "C₁-C₆ alkyl group" is bonded to the above-mentioned "mono-C₁-C₆ alkylamino group")". The "group represented by the formula-NR⁷R⁸" is preferably a methylcarbonylamino group, an ethylcarbonylamino group, a methylsulfinylamino group, a methylsulfonylamino group, a methylcarbonyl(methyl)amino group, an ethylcarbonyl (methyl) amino group, a methylcarbonyl(ethyl)amino group, a methylsulfinyl(methyl)amino group or a methylsulfonyl(methyl)amino group.

In the present invention, a "C₃-C₆ cycloalkyl group" is a cyclopropyl group, a cyclobutyl group, a cyclopentyl group or a cyclohexyl group. In R¹, the "C₃-C₆ cycloalkyl group" is preferably a cyclopentyl group or a cyclohexyl group. In Substituent Group B, the "C₃-C₆ cycloalkyl group" is preferably a cyclopropyl group.

In the present invention, a "C₃-C₆ cycloalkyl group which may be substituted with one C₁-C₆ hydroxyalkyl group" is the above-mentioned "C₃-C₆ cycloalkyl group", or a group in which one above-mentioned "C₁-C₆ hydroxyalkyl group" is bonded to the above-mentioned "C₃-C₆ cycloalkyl group". Examples of the C₃-C₆ cycloalkyl group which may be substituted with one C₁-C₆ hydroxyalkyl group include a cyclopropyl group, a 2-hydroxymethylcyclopropyl group, a 2-(2-hydroxyethyl)cyclopropyl group, a cyclobutyl group, a 3-hydroxymethylcyclobutyl group, a 3-hydroxymethylcyclopentyl group or a 4-hydroxymethylcyclohexyl group. The "C₃-C₆ cycloalkyl group which may be substituted with one C₁-C₆ hydroxyalkyl group" is preferably a cyclopropyl group or a 2-hydroxymethylcyclopropyl group, and more preferably a cyclopropyl group.

In the present invention, a "C₆-C₁₀ aryl group" is a group formed by the elimination of one hydrogen atom bound to a ring of an aromatic hydrocarbon having 6 to 10 carbon atoms. Examples of the C₆-C₁₀ aryl group include a phenyl group or a naphthyl group. The "C₆-C₁₀ aryl group" is preferably a phenyl group.

In the present invention, a "heterocyclic group" is a 4-to 7-membered heterocyclic group that contains 1 to 3 sulfur, oxygen and/or nitrogen atom(s), and may further contain 1 or 2 nitrogen atom(s), and said sulfur atom(s) may have 2 oxygen atoms bonded thereto. Examples of the heterocyclic group include an "aromatic heterocyclic group" such as a furyl group, a thienyl group, a pyrrolyl group, an azepinyl group, a pyrazolyl group, an imidazolyl group, an oxazolyl group, an isoxazolyl group, a thiazolyl group, an isothiazolyl group, a 1,3,4-oxadiazolyl group, a 1,3,4-thiadiazolyl group, a triazolyl group, a tetrazolyl group, a thiadiazolyl group, a pyranyl group, a pyridyl group, a pyridazinyl group, a pyrimidinyl group or a pyrazinyl group, or a "partially or completely reduced saturated heterocyclic group" such as a tetrahydropyranyl group, a tetrahydrothienyl group, a morpholinyl group, a thiomorpholinyl group, a pyrrolidinyl group, a pyrrolinyl group, an imidazolidinyl group, a pyrazolidinyl group, a piperidinyl group, a piperazinyl group, an oxazolinyl group, an oxazolidinyl group, an isoxazolidinyl group, a thiazolinyl group, a thiazolidinyl group, a pyrazolidinyl group, a dioxolanyl group, a dioxanyl group or a 5,6-dihydro-4H-1,3-oxazine group. The above heterocyclic group may be fused with another cyclic group such as a benzene group ("fused bicyclic heterocyclic group"), examples of which include a benzothienyl group, a benzothiazolyl group, a benzoxazolyl group, an isobenzofuranyl group, a 1,3-dihydroisobenzofuranyl group, a quinolyl group, a 1,3-benzodioxolanyl group, a 1,4-benzodioxanyl group, an indolyl group, an isoindolyl group or an indolinyl group. The "heterocyclic group" is preferably a 5-membered or 6-membered aromatic heterocyclic group, more preferably a triazolyl group, a pyridyl group or a pyrimidinyl group, and even more preferably a 2-pyridyl group or a 2-pyrimidinyl group.

In the present invention, a "C₆-C₁₀ aryl group that may be substituted with 1 to 5 group(s) independently selected from Substituent Group A" is the above-mentioned "C₆-C₁₀ aryl group", or the above-mentioned "C₆-C₁₀ aryl group" that is substituted with 1 to 5 group(s) independently selected from Substituent Group A. The "C₆-C₁₀ aryl group that may be substituted with 1 to 5 group(s) independently selected from Substituent Group A" is preferably a phenyl group that may be substituted with 1 to 3 group(s) independently selected from Substituent Group A, more preferably a phenyl group that is substituted with 1 or 2 group(s) independently selected from the group consisting of (a halogen atom, a C₁-C₆ halogenated alkyl group, a C₁-C₆ alkylsulfonyl group and a group represented by the formula - C(=O)-NR⁵R⁶), even more preferably a 4-fluorophenyl group, a 4-trifluoromethylphenyl group, a 4-methylsulfonylphenyl group, a 4-dimethylaminocarbonylphenyl group, a 2,4-difluorophenyl group, a 3,4-difluorophenyl group or a 2-chloro-4-fluorophenyl group, particularly preferably a 4-fluorophenyl group, a 2,4-difluorophenyl group, a 3,4-difluorophenyl group or a 2-chloro-4-fluorophenyl group, and most preferably a 4-fluorophenyl group, a 2,4-difluorophenyl group or a 3,4-difluorophenyl group.

In the present invention, a "heterocyclic group that may be substituted with 1 to 4 group(s) independently selected from Substituent Group A" is the above-mentioned "heterocyclic group", or the above-mentioned "heterocyclic group" that is substituted with 1 to 4 group(s) independently selected from Substituent Group A. The "heterocyclic group that may be substituted with 1 to 4 group(s) independently selected from Substituent Group A" is preferably a pyridyl group that may be substituted with 1 to 3 group(s) independently selected from Substituent Group A, or a pyrimidinyl group that may be substituted with 1 to 3 group(s) independently selected from Substituent Group A, more preferably a 2-pyridyl group that may be substituted with 1 or 2 group(s) independently selected from the group consisting of (a C₁-C₆ alkyl group, a C₁-C₆ halogenated alkyl group, a C₁-C₆ hydroxyalkyl group and a C₂-C₇ alkoxycarbonyl group), or a 2-pyrimidinyl group, even more preferably a 2-pyridyl group, a 3-methyl-2-pyridyl group, a 3-trifluoromethyl-2-pyridyl group, a 5-hydroxymethyl-2-pyridyl group, a 5-ethoxycarbonyl-2-pyridyl group or a 2-pyrimidinyl group, and particularly preferably a 2-pyridyl group, a 5-hydroxymethyl-2-pyridyl group or a 5-ethoxycarbonyl-2-pyridyl group.

In the present invention, a "C₃-C₆ cycloalkyl group that may be substituted with 1 to 4 group(s) independently selected from Substituent Group A" is the above-mentioned "C₃-C₆ cycloalkyl group", or the above-mentioned "C₃-C₆ cycloalkyl group" substituted with 1 to 4 group(s) independently selected from Substituent Group A. The "C₃-C₆ cycloalkyl group that may be substituted with 1 to 4 group(s) independently selected from Substituent Group A" is preferably a cyclohexyl group that may be substituted with 1 to 4 group(s) independently selected from Substituent Group A, a cyclopentyl group that may be substituted with 1 to 4 group(s) independently selected from Substituent Group A, or a cyclopropyl group that may be substituted with 1 to 4 group(s) independently selected from Substituent Group A, and more preferably a cyclopropyl group.

In the present invention, a "C₁-C₆ alkyl group that may be substituted with 1 to 5 group(s) independently selected from Substituent Group B" is the above-mentioned "C₁-C₆ alkyl group", or the above-mentioned "C₁-C₆ alkyl group" substituted with 1 to 5 group(s) independently selected from Substituent Group B. The "C₁-C₆ alkyl group that may be substituted with 1 to 5 group(s) independently selected from Substituent Group B" is preferably a C₁-C₆ alkyl group that may be substituted with 1 to 3 group(s) independently selected from the group consisting of (a halogen atom, a C₃-C₆ cycloalkyl group which may be substituted with one C₁-C₆ hydroxyalkyl group), a hydroxy group, a C₁-C₆ alkoxy group, a C₂-C₇ alkoxycarbonyl group and a di-(C₁-C₆ alkyl)amino group), more preferably a C₁-C₃ alkyl group that may be substituted with 1 to 3 group(s) independently selected from the group(s) consisting of (a halogen atom, a C₃-C₆ cycloalkyl group, a hydroxy group and a C₁-C₆ alkoxy group), even more preferably an isopropyl group, a cyclopropylmethyl group, a trifluoromethyl group, a 3-hydroxypropyl group, a 3-methoxypropyl group, a 3-methoxy-1-methylpropyl group, a 2-fluoro-3-methoxypropyl group, a 4-methoxybutyl group or a 2-cyclopropylethyl group, and particularly preferably an isopropyl group, a cyclopropylmethyl group, a trifluoromethyl group, a 3-hydroxypropyl group, a 3-methoxypropyl group, a 3-methoxy-1-methylpropyl group or a 2-fluoro-3-methoxypropyl group.

In the present invention, although a "1H-tetrazol-5-yl group" may also be represented as a 2H-tetrazol-5-yl group depending on the case, they both indicate the same group.

In the present invention, R¹ is preferably a pyridyl group that may be substituted with 1 to 3 group(s) independently selected from Substituent Group A, a pyrimidinyl group that may be substituted with 1 to 3 group(s) independently selected from Substituent Group A, or a C₁-C₆ alkyl group that may be substituted with 1 to 5 group(s) independently selected from Substituent group B, more preferably a 2-pyridyl group that may be substituted with 1 or 2 group(s) independently selected from the group consisting of (a C₁-C₆ alkyl group, a C₁-C₆ halogenated alkyl group, a C₁-C₆ hydroxyalkyl group and a C₂-C₇ alkoxycarbonyl group), a 2-pyrimidinyl group, or a C₁-C₆ alkyl group that may be substituted with 1 to 3 group(s) independently selected from the group consisting of (a halogen atom, a C₃-C₆ cycloalkyl group which may be substituted with one C₁-C₆ hydroxyalkyl group, a hydroxy group, a C₁-C₆ alkoxy group, a C₂-C₇ alkoxycarbonyl group and a di-(C₁-C₆ alkyl)amino group), even more preferably a 2-pyridyl group, a 5-hydroxymethyl-2-pyridyl group, a 5-ethoxycarbonyl-2-pyridyl group, a 2-pyrimidinyl group, an isopropyl group, a cyclopropylmethyl group, a trifluoromethyl group, a 3-hydroxypropyl group, a 3-methoxypropyl group, a 3-methoxy-1-methylpropyl group, a 2-fluoro-3-methoxypropyl group, a 4-methoxybutyl group, a cyclopropyl group or a 2-cyclopropylethyl group, particularly preferably a 2-pyridyl group, a 5-hydroxymethyl-2-pyridyl group, a 5-ethoxycarbonyl-2-pyridyl group, a 2-pyrimidinyl group, an isopropyl group, a cyclopropylmethyl group, a trifluoromethyl group, a 3-hydroxypropyl group, a 3-methoxypropyl group, a 3-methoxy-1-methylpropyl group or a 2-fluoro-3-methoxypropyl group.

In the present invention, R² is preferably a phenyl group that may be substituted with 1 to 3 group(s) independently selected from Substituent Group A, more preferably a phenyl group that may be substituted with 1 or 2 group(s) independently selected from the group consisting of (a halogen atom, a C₁-C₆ halogenated alkyl group, a C₁-C₆ alkylsulfonyl group and a group represented by the formula -C(=O)-NR⁵R⁶), even more preferably a 4-fluorophenyl group, a 2,4-difluorophenyl group, a 3,4-difluorophenyl group or a 2-chloro-4-fluorophenyl group, particularly preferably a 4-fluorophenyl group, a 2,4-difluorophenyl group or a 3,4-difluorophenyl group.

In the present invention, R³ is preferably a 1H-tetrazol-5-yl group.

In the present invention, R⁴ is preferably a hydrogen atom or a methyl group.

In the present invention, R⁵ is preferably a hydrogen atom or a methyl group.

In the present invention, R⁶ is preferably a hydrogen atom or a methyl group.

In the present invention, R⁷ is preferably a hydrogen atom or a methyl group.

In the present invention, R⁸ is preferably an acetyl group, a methylsulfinyl group or a methylsulfonyl group.

In the present invention, X is preferably a single bond or a sulfur atom.

In the present invention, Substituent Group A is preferably the group of substituents selected from a halogen atom, a C₁-C₆ alkyl group, a C₁-C₆ halogenated alkyl group, a C₁-C₆ hydroxyalkyl group, a C₂-C₇ alkoxycarbonyl group, a C₁-C₆ alkylsulfonyl group and a group represented by the formula - C(=O)-NR⁵R⁶.

In the present invention, Substituent Group B is preferably the group of substituents selected from a halogen atom, a C₃-C₆ cycloalkyl group which may be substituted with one C₁-C₆ hydroxyalkyl group, a hydroxy group, a C₁-C₆ alkoxy group, a C₂-C₇ alkoxycarbonyl group and a di-(C₁-C₆ alkyl) amino group.

The compound or pharmacologically acceptable salt thereof represented by the general formula (I) of the present invention includes all isomers (such as a keto-enol isomer, a diastereomer, an optical isomer, a rotamer, etc.).

The compound or pharmacologically acceptable salt thereof represented by the general formula (I) of the present invention has various isomers because asymmetric carbon atom(s) exist in the molecule. These isomers and mixtures of these isomers of the present invention are all represented by a single formula, specifically, the general formula (I). Accordingly, the present invention includes all of these isomers and mixtures of these isomers in arbitrary ratios.

The aforementioned stereoisomers can be obtained by synthesizing the compound of the present invention using an optically active raw material compound or using an asymmetric synthesis or asymmetric induction technique or by isolating the synthesized compound of the present invention by a common optical resolution or separation method if desired.

The compound or pharmacologically acceptable salt thereof represented by the general formula (I) of the present invention may contain unnatural proportions of atomic isotopes at one or more of the atoms that constitute such compounds. Examples of atomic isotopes include deuterium (²H) tritium (³H), iodine-125 (¹²⁵I) and carbon-14 (¹⁴C). The above-described compounds may be radiolabeled with radioisotopes such as tritium (³H), iodine-125 (¹²⁵I) or carbon-14 (¹⁴C). Radiolabeled compounds are useful as therapeutic or prophylactic agents, research reagents such as assay reagents, and diagnostic agents such as *in vivo* diagnostic imaging agents. All isotopic variants of the compounds of the present invention, whether radioactive or not, are included in the scope of the present invention.

A "pharmacologically acceptable salt thereof" refers to a salt that is free of prominent toxicity and which can be used as a pharmaceutical. The compound represented by the general formula (I) of the present invention can be converted to a salt by reacting with an acid in the case the compound has a basic group, or by reacting with a base in the case the compound has an acidic group.

Examples of salts based on a basic group include salts of hydrohalic acids such as hydrofluorides, hydrochlorides, hydrobromides or hydroiodides, salts of inorganic acids such as nitrates, perchlorates, sulfates or phosphates; C₁-C₆ alkylsulfonates such as methanesulfonates, trifluoromethanesulfonates or ethanesulfonates, arylsulfonates such as benzenesulfonates or p-toluenesulfonates; salts of organic acids such as acetates, malates, fumarates, succinates, citrates, ascorbates, tartrates, oxalates or maleates; and, salts of amino acids such as salts of glycine, lysine, arginine, ornithine, glutamic acid and aspartic acid.

On the other hand, examples of salts based on acidic groups include metal salts such as alkali metal salts such as sodium salts, potassium salts or lithium salts, alkaline earth metal salts such as calcium salts or magnesium salts, aluminum salts, or iron salts; amine salts such as inorganic salts such as ammonium salts, or organic salts such as salts of t-octylamine, dibenzylamine, morpholine, glucosamine, phenylglycine alkyl esters, ethylenediamine, N-methylglucamine, guanidine, diethylamine, triethylamine, dicyclohexylamine, N,N'-dibenzylethylenediamine, chloroprocaine, procaine, diethanolamine, N-benzylphenethylamine, piperazine, tetramethylammonium or tris(hydroxymethyl)aminomethane; and, salts of amino acids such as salts of glycine, lysine, arginine, ornithine, glutamic acid and aspartic acid.

The compound or pharmacologically acceptable salt thereof represented by the general formula (I) of the present invention may become a hydrate by absorbing moisture or by allowing adsorbed water to adhere thereto as a result of being left in the atmosphere or recrystallized, and such hydrates are also included in the salts of the present invention.

The compound or pharmacologically acceptable salt thereof represented by the general formula (I) of the present invention may become a solvate by absorbing another type of solvent, and such solvates are also included in the salts of the present invention.

The compound or pharmacologically acceptable salt thereof represented by the general formula (I) of the present invention is preferably a compound represented by the general formula (I) of the present invention.

In the present invention, "metabolic syndrome" refers to a disease state that is based on insulin resistance for which there is considerably higher risk for coronary artery disease due to accumulation of multiple coronary vessel risk factors (including lifestyle diseases such as hyperlipemia, diabetes, obesity and hypertension) (Diabetes, Obesity and Metabolism, 9, 2007, 246-258; Journal of the American Medical Association, 285, 2486-2497 (2001); Diabet. Med., 15, 539-553 (1998)).

### Effect of the Invention

The compound represented by the general formula (I) of the present invention, or a pharmacologically acceptable salt thereof, has superior GK activating activity, and is useful as a pharmaceutical for the prevention and/or treatment of a disease selected from the group consisting of diabetes, impaired glucose tolerance, gestational diabetes, chronic complications of diabetes (including diabetic peripheral neuropathy, diabetic nephropathy, diabetic retinopathy and diabetic macroangiopathy) and metabolic syndrome in warm-blooded animals (and preferably in mammals, including humans). In addition, the novel compound represented by the general formula (I) provided by the present invention, or a pharmacologically acceptable salt thereof, has superior GK activating activity and is useful as an active ingredient of a pharmaceutical for the prevention and/or treatment of the aforementioned diseases in warm-blooded animals (and preferably in mammals, including humans). Preferred examples of diseases include diabetes and impaired glucose tolerance. The compound represented by the general formula (I) of the present invention, or a pharmacologically acceptable salt thereof, can preferably be used as a pharmaceutical for treatment of the aforementioned diseases.

### MODE FOR CARRYING OUT THE INVENTION

The compound represented by the general formula (I) of the present invention can be produced according to Methods A to F described below.

There are no particular limitations on the solvent used in the reactions of each of the steps of the Methods A to F described below, provided it does not inhibit the reaction and it dissolves the starting raw material to a certain degree. For example, the solvent is selected from the group of solvents indicated below. The solvent group is composed of hydrocarbons such as pentane, hexane, octane, petroleum ether, ligroin or cyclohexane; amides such as formamide, N,N-dimethylformamide, N,N-dimethylacetamide, N-methyl-2-pyrrolidone, N-methyl-2-pyrrolidinone or hexamethylphosphoric triamide; ethers such as diethyl ether, diisopropyl ether, tetrahydrofuran, dioxane, dimethoxyethane, diethylene glycol dimethyl ether or cyclopentyl methyl ether; alcohols such as methanol, ethanol, n-propanol, i-propanol, n-butanol, 2-butanol, 2-methyl-1-propanol, t-butanol, isoamyl alcohol, diethylene glycol, glycerin, octanol, cyclohexanol or methyl cellosolve; sulfoxides such as dimethyl sulfoxide; sulfones such as sulfolane; nitriles such as acetonitrile, propionitrile, butyronitrile or isobutyronitrile; esters such as ethyl formate, ethyl acetate, propyl acetate, butyl acetate or diethyl carbonate; ketones such as acetone, methyl ethyl ketone, 4-methyl-2-pentanone, methyl isobutyl ketone, isophorone or cyclohexanone; nitro compounds such as nitroethane or nitrobenzene; halogenated hydrocarbons such as dichloromethane, 1,2-dichloroethane, chlorobenzene, dichlorobenzene, chloroform or carbon tetrachloride; aromatic hydrocarbons such as benzene, toluene or xylene; carboxylic acids such as acetic acid, formic acid, propionic acid, butyric acid or trifluoroacetic acid; amines such as N-methylmorpholine, triethylamine, tripropylamine, tributylamine, diisopropylethylamine, dicyclohexylamine, N-methylpiperidine, pyridine, 2,6-lutidine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO), 1,8-diazabicyclo[5.4.0]undec-7-ene (DBU) or piperidine; water; and, mixed solvents thereof.

Examples of bases used in the reactions of each of the steps of the following Methods A to F include inorganic bases such as alkali metal carbonates such as sodium carbonate, potassium carbonate, lithium carbonate or cesium carbonate; alkali metal hydrogencarbonates such as sodium hydrogencarbonate, potassium hydrogencarbonate or lithium hydrogencarbonate; alkali metal acetates such as sodium acetate, potassium acetate, lithium acetate or cesium acetate; alkali metal hydrides such as lithium hydride, sodium hydride or potassium hydride; alkali metal hydroxides such as sodium hydroxide, potassium hydroxide, barium hydroxide or lithium hydroxide; and, alkali metal fluorides such as sodium fluoride, or potassium fluoride; alkali metal alkoxides such as sodium methoxide, sodium ethoxide, sodium t-butoxide or potassium t-butoxide; alkali metal trialkylsiloxides such as sodium trimethylsiloxide, potassium trimethylsiloxide or lithium trimethylsiloxide; organic bases such as N-methylmorpholine, triethylamine, tripropylamine, tributylamine, diisopropylethylamine, dicyclohexylamine, N-methylpiperidine, pyridine, 2,6-lutidine, collidine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) or 1,8-diazabicyclo[5.4.0]undec-7-ene (DBU); organometallic bases such as lithium diisopropylamide or lithium bis(trimethylsilyl)amide; and, amino acids such as proline.

In the reactions of each of the steps of the following Methods A to F, the reaction temperatures vary depending on the solvent, starting raw material, reagents and the like, while the reaction times vary depending on the solvent, starting raw material, reagents, reaction temperature and the like.

In the reactions of each of the steps of the following Methods A to F, each desired compound is collected from the reaction mixture in accordance with conventional methods after completion of the reaction. For example, the reaction mixture is appropriately neutralized, and insoluble matter, if present, is removed by filtration. Then, water and an immiscible organic solvent such as ethyl acetate are added, and the organic layer containing the desired compound is separated. The organic layer is washed with water or the like, and then dried over anhydrous magnesium sulfate, anhydrous sodium sulfate or the like. After filtration, the solvent is evaporated to give the desired compound. The resulting desired compound may be isolated and purified if necessary by appropriately combining conventional methods, for example, methods usually employed for isolation and purification of organic compounds such as recrystallization, reprecipitation, or chromatography (for example, adsorption column chromatography using a carrier such as silica gel, alumina or magnesium-silica gel-based florisil; methods employing a synthetic adsorbent such as partition column chromatography, or the like using a carrier such as Sephadex LH-20 (Pharmacia Corp.), Amberlite XAD-11 (Rohm and Haas, GmbH), or Diaion HP-20 (Mitsubishi Chemical Corp.); methods employing ion exchange chromatography; or forward phase and/or reverse phase column chromatography using silica gel or alkyl-bonded silica gel (and preferably, high performance liquid chromatography), followed by eluting with a suitable eluent). The desired compound insoluble in a solvent may be purified by washing the resulting crude solid product with a solvent. Moreover, the desired compound in each step may also be used as is for the next reaction without purification.

In the reactions of each of the steps of the following Methods A to F, R¹, R² and X have the same meanings as previously described. R^{1a} and R^{2a} represent groups in which an amino group, hydroxy group and/or carboxyl group contained as a substituent in groups R¹ and R² is an optionally protected amino group, hydroxy group and/or carboxyl group, and also R^{1a} and R^{2a} represent the same groups as defined for groups R¹ and R². R^{9a}CH₂CH₂ represents R^{1a}. Z represents a halogen atom (preferably a chlorine atom, a bromine atom or an iodine atom, and more preferably a bromine atom). Z¹ represents a halogen atom (preferably a chlorine atom, a bromine atom or an iodine atom, and more preferably a chlorine atom). Y represents a halogen atom or a sulfonyloxy group (preferably a chlorine atom, a bromine atom or a trifluoromethanesulfonyloxy group, and more preferably a chorine atom). Prot represents a carboxyl group protecting group used in the field of organic synthetic chemistry (preferably a C₁-C₆ alkyl group, and more preferably a methyl group, an ethyl group, a propyl group or a 2-ethylhexyl group). Prot¹ represents a hydroxy group protecting group used in the field of organic synthetic chemistry (and preferably a 2,3-dimethyl-2,3-butylidene group or a benzylidene group, and more preferably a 2,3-dimethyl-2,3-butylidene group).

Method A is a method for producing a compound represented by general formula (Ia) in which R³ is a 1H-tetrazol-5-yl group among the compounds represented by general formula (I).

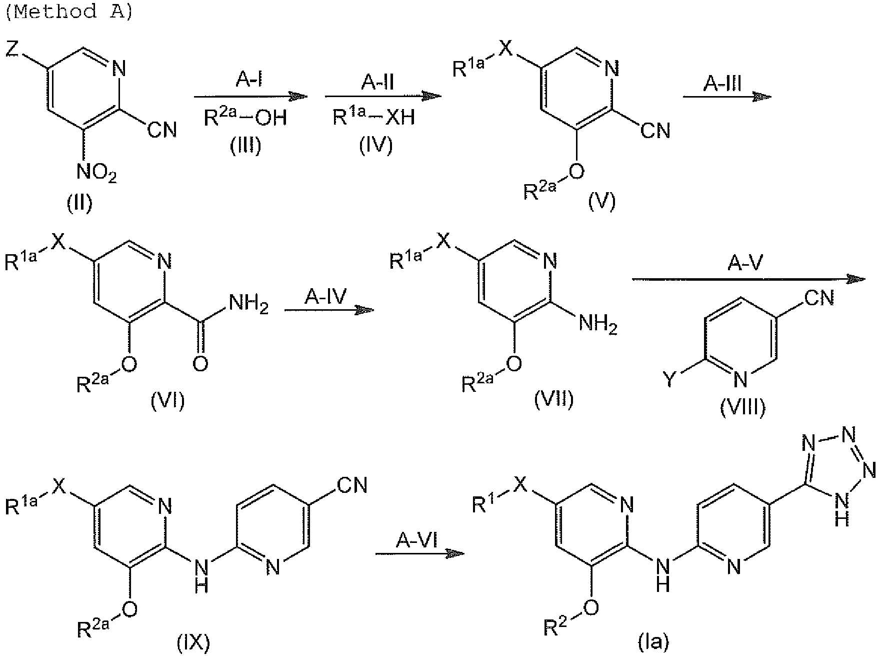

Step A-I and Step A-II:
These steps are steps for producing a compound represented by general formula (V) by going through a step A-I for reacting a compound represented by general formula (II) with a compound represented by general formula (III) in a solvent and in the presence of a base, followed by going through a step A-II for reacting with a compound represented by general formula (IV) in a solvent and in the presence of a base. Steps A-I and A-II can be carried out separately for each step or carried out in a single reaction vessel.

The compound represented by general formula (II), the compound represented by general formula (III) and the compound represented by general formula (IV) are either known compounds or are easily produced in accordance with known methods or methods similar thereto by using known compounds for the starting raw materials.

The solvent used in these steps is preferably an ether or an amide and more preferably tetrahydrofuran, 1,2-dimethoxyethane, 1,4-dioxane, N,N-dimethylformamide or N-methylpyrrolidone.

The base used in these steps is preferably an alkali metal hydride and more preferably sodium hydride or potassium hydride.

The reaction temperature in these steps is a temperature within the range of the cooling temperature to the heating reflux temperature, and the heating reflux temperature varies depending on the solvents. The reaction temperature is normally -50°C to 100°C and preferably -20°C to 20°C.

The reaction time in these steps is normally 30 minutes to 48 hours and preferably 1 hour to 8 hours.

### Step A-III:

This step is a step for producing a compound represented by general formula (VI) by reacting a compound represented by general formula (V) with an acid or a base in a solvent.

The solvent used in this step is preferably water.

The acid used in this step is preferably a protonic acid and more preferably sulfuric acid, hydrochloric acid or nitric acid.

The base used in this step is preferably an alkali metal hydroxide and more preferably lithium hydroxide, sodium hydroxide or potassium hydroxide.

The reaction temperature in this step is a temperature within the range of the cooling temperature to the heating reflux temperature, and the heating reflux temperature varies depending on the solvents. The reaction temperature is normally -50°C to 100°C and preferably 0°C to 40°C.

The reaction time in this step is normally 30 minutes to 48 hours and preferably 1 hour to 8 hours.

### Step A-IV:

This step is a step for producing a compound represented by general formula (VII) by reacting a compound represented by general formula (VI) with a mixed reagent of bromine and an alkali metal hydroxide in a solvent.

The solvent used in this step is preferably an ether and more preferably tetrahydrofuran, 1,2-dimethoxyethane or 1,4-dioxane.

The alkali metal hydroxide used in this step is preferably sodium hydroxide.

The reaction temperature in this step is a temperature within the range of the cooling temperature to the heating reflux temperature, and the heating reflux temperature varies depending on the solvent. The reaction temperature is normally -50°C to 100°C and preferably 0°C to 40°C.

The reaction time in this step is normally 30 minutes to 48 hours and preferably 1 hour to 8 hours.

### Step A-V:

This step is a step for producing a compound represented by general formula (IX) by reacting a compound represented by general formula (VII) with a compound represented by general formula (VIII) in a solvent and in the presence of a palladium catalyst and a base.

The compound represented by general formula (VIII) is either a known compound or is easily produced in accordance with known methods or methods similar thereto by using known compounds for the starting raw materials.

The solvent used in this step is preferably an ether, an aromatic hydrocarbon, an alcohol or an amide and more preferably tetrahydrofuran, 1,2-dimethoxyethane, 1,4-dioxane, benzene, toluene, methanol, ethanol, isopropanol, N,N-dimethylformamide, N,N-dimethylacetamide or N-methylpyrrolidone.

The palladium catalyst used in this step is preferably tetrakis(triphenylphosphine)palladium, [1,1'-bis(diphenylphosphino)ferrocene]palladium dichloride dichloromethane adduct (II) or tris(dibenzylideneacetone)dipalladium.

In this step, a phosphine derivative may be added as palladium ligand as necessary. The ligand used is preferably 4,5-bis(diphenylphosphino)-9,9-dimethylxanthene.

The base used in this step is preferably an organic base, an alkali metal carbonate, an alkali metal hydrogencarbonate, an alkali metal alkoxide or an alkali metal hydride and more preferably pyridine, triethylamine, N,N-diisopropylethylamine, 4-(dimethylamino)pyridine, N-methylmorpholine, 2,6-lutidine, collidine, sodium carbonate, potassium carbonate, cesium carbonate, sodium hydrogencarbonate, sodium ethoxide, potassium tert-butoxide, sodium hydride or potassium hydride.

The reaction temperature in this step is a temperature within the range of the cooling temperature to the heating reflux temperature, and the heating reflux temperature varies depending on the solvent. The reaction temperature is normally -50°C to 150°C and preferably 80°C to 120°C.

The reaction time in this step is normally 30 minutes to 48 hours and preferably 1 hour to 8 hours.

### Step A-VI:

This step is a step for producing a compound represented by general formula (Ia) by reacting a compound represented by general formula (IX) with an azide compound, followed by removing a protecting group of an amino group, a hydroxy group and/or a carboxyl group in R^{1a} and/or R^{2a} as desired.

The solvent used in this step is preferably an aromatic hydrocarbon or an amide and more preferably benzene, toluene, N,N-dimethylformamide, N,N-dimethylacetamide or N-methylpyrrolidone.

The azide compound used in this step is preferably tributyltin azide, trimethylsilyl azide or sodium azide.

The reaction temperature in this step is a temperature within the range of the cooling temperature to the heating reflux temperature, and the heating reflux temperature varies depending on the solvent. The reaction temperature is normally -50°C to 150°C and preferably 90°C to 130°C.

The reaction time in this step is normally 30 minutes to 48 hours and preferably 1 hour to 8 hours.

Method B is a method for producing a compound represented by general formula (Ib) in which R³ is a 5-oxo-4,5-dihydro-[1,2,4]oxadiazol-3-yl group among compounds represented by general formula (I).

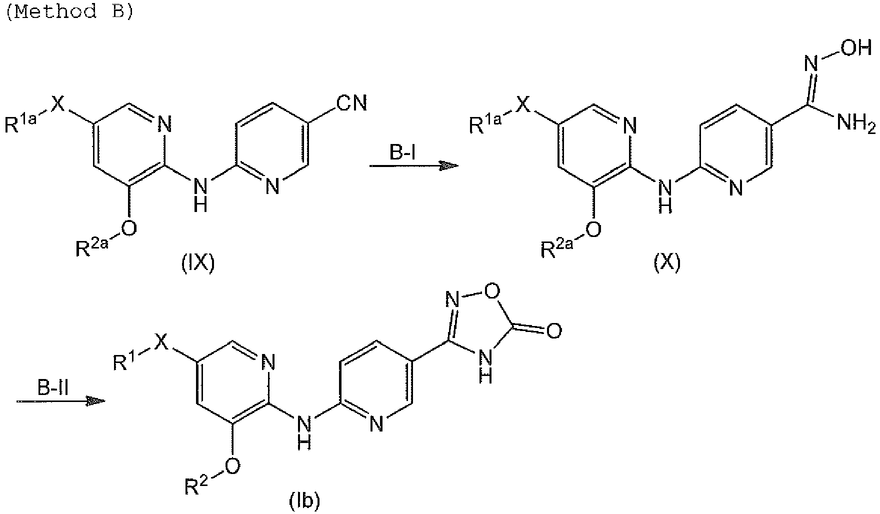

### Step B-I:

This step is a step for producing a compound represented by general formula (X) by reacting a compound represented by general formula (IX) with a hydroxyamine in a solvent.

The solvent used in this step is preferably an ether, an alcohol or an amide and more preferably tetrahydrofuran, 1,2-dimethoxyethane, 1,4-dioxane, methanol, ethanol, isopropanol, N,N-dimethylformamide, N,N-dimethylacetamide or N-methylpyrrolidone.

The reaction temperature in this step is a temperature within the range of the cooling temperature to the heating reflux temperature, and the heating reflux temperature varies depending on the solvent. The reaction temperature is normally -50°C to 150°C and preferably 60°C to 100°C.

The reaction time in this step is normally 30 minutes to 48 hours and preferably 1 hour to 8 hours.

### Step B-II:

This step is a step for producing a compound represented by general formula (Ib) by reacting a compound represented by general formula (X) with a carbonyl group equivalent, followed by removing a protecting group of an amino group, a hydroxy group and/or carboxyl group in R^{1a} and/or R^{2a} as desired.

The solvent used in this step is preferably an ether or an amide and more preferably tetrahydrofuran, 1,2-dimethoxyethane, 1,4-dioxane, N,N-dimethylformamide, N,N-dimethylacetamide or N-methylpyrrolidone.

The carbonyl group equivalent used in this step is preferably 1,1-carbonyldiimidazole, phosgene, triphosgene or ethyl chloroformate and more preferably 1,1-carbonyldiimidazole.

The reaction temperature in this step is a temperature within the range of the cooling temperature to the heating reflux temperature, and the heating reflux temperature varies depending on the solvent. The reaction temperature is normally -50°C to 150°C and preferably 80°C to 100°C.

The reaction time in this step is normally 30 minutes to 48 hours and preferably 1 hour to 8 hours.

Method C is a method for producing the compound represented by general formula (IX) used in the aforementioned step A-VI of Method A and in the aforementioned step B-I of Method B.

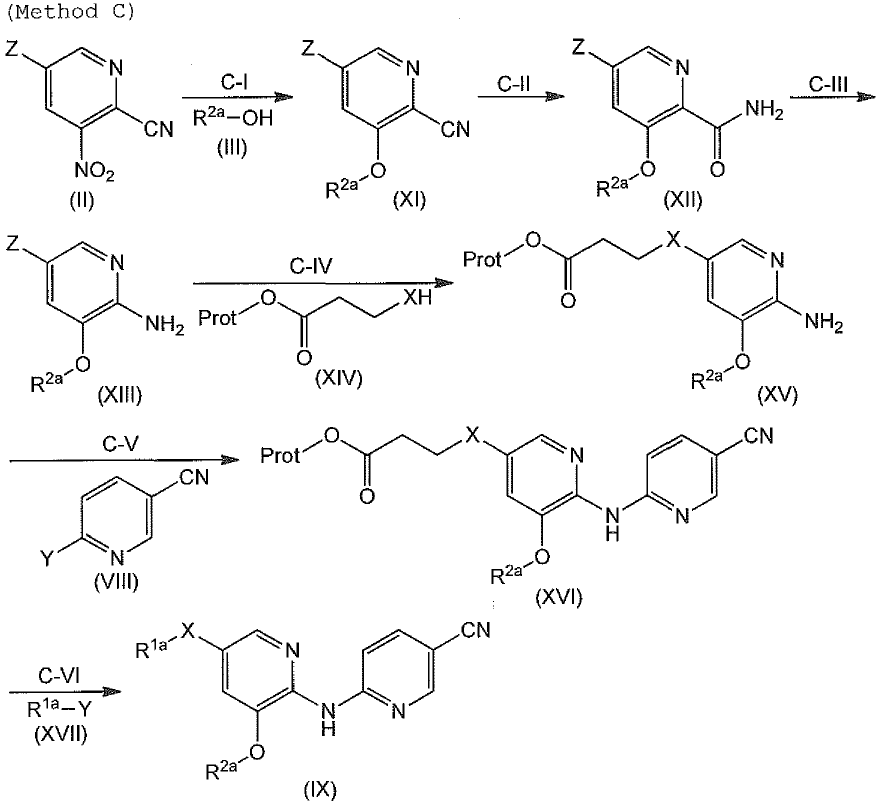

### Step C-I:

This step is carried out in the same manner as the above-mentioned step A-I of Method A by reacting a compound represented by general formula (II) with a compound represented by general formula (III) in a solvent and in the presence of a base, and is a step for producing a compound represented by general formula (XI).

### Step C-II:

This step is carried out in the same manner as the above-mentioned step A-III of Method A by reacting a compound represented by general formula (XI) with an acid or a base in a solvent, and is a step for producing a compound represented by general formula (XII).

### Step C-III:

This step is carried out in the same manner as the above-mentioned step A-IV of Method A by reacting a compound represented by general formula (XII) with a mixed reagent of bromine and an alkali metal hydroxide, in a solvent, and is a step for producing a compound represented by general formula (XIII).

### Step C-IV:

This step is a step for producing a compound represented by general formula (XV) by reacting a compound represented by general formula (XIII) with a compound represented by general formula (XIV) in a solvent and in the presence of a palladium catalyst and a base.

The compound represented by general formula (XIV) used in this step is either a known compound or is easily produced in accordance with known methods or methods similar thereto by using known compounds for the starting raw materials.

The solvent used in this step is preferably an ether, an aromatic hydrocarbon, an alcohol or an amide and more preferably tetrahydrofuran, 1,2-dimethoxyethane, 1,4-dioxane, benzene, toluene, methanol, ethanol, isopropanol, N,N-dimethylformamide, N,N-dimethylacetamide or N-methylpyrrolidone.

The palladium catalyst used in this step is preferably tetrakis(triphenylphosphine)palladium, [1,1'-bis(diphenylphosphino)ferrocene]palladium dichloride dichloromethane adduct (II) or tris(dibenzylideneacetone)dipalladium.

In this step, a phosphine derivative may be added as palladium ligand as necessary. The ligand used is preferably 4,5-bis(diphenylphosphino)-9,9-dimethylxanthene.

The base used in this step is preferably an organic base, an alkali metal carbonate, an alkali metal hydrogencarbonate, an alkali metal alkoxide or an alkali metal hydride and more preferably pyridine, triethylamine, N,N-diisopropylethylamine, 4-(dimethylamino)pyridine, N-methylmorpholine, 2,6-lutidine, collidine, sodium carbonate, potassium carbonate, cesium carbonate, sodium hydrogencarbonate, sodium ethoxide, potassium tert-butoxide, sodium hydride or potassium hydride.

The reaction temperature in this step is a temperature within the range of the cooling temperature to the heating reflux temperature, and the heating reflux temperature varies depending on the solvent. The reaction temperature is normally -50°C to 100°C and preferably 80°C to 120°C.

The reaction time in this step is normally 30 minutes to 48 hours and preferably 1 hour to 8 hours.

### Step C-V:

This step is carried out in the same manner as the above-mentioned step A-V of Method A by reacting a compound represented by general formula (XV) with a compound represented by general formula (VIII) in a solvent and in the presence of a palladium catalyst and a base, and is a step for producing a compound represented by general formula (XVI).

### Step C-VI:

This step is a step for producing a compound represented by general formula (IX) by reacting a compound represented by general formula (XVI) with a compound represented by general formula (XVII) in a solvent and in the presence of a base.

The compound represented by general formula (XVII) is either a known compound or is easily produced in accordance with known methods or methods similar thereto by using known compounds for the starting raw materials.

The solvent used in this step is preferably an ether, an alcohol or an amide and more preferably tetrahydrofuran, 1,2-dimethoxyethane, 1,4-dioxane, methanol, ethanol, isopropanol, N,N-dimethylformamide, N,N-dimethylacetamide or N-methylpyrrolidone.

The base used in this step is preferably an alkali metal alkoxide or an alkali metal hydride and more preferably sodium methoxide, sodium ethoxide, potassium tert-butoxide, sodium hydride or potassium hydride.

The reaction temperature in this step is a temperature within the range of the cooling temperature to the heating reflux temperature, and the heating reflux temperature varies depending on the solvent. The reaction temperature is normally -50°C to 150°C and preferably 0°C to 40°C.

The reaction time in this step is normally 30 minutes to 48 hours and preferably 1 hour to 8 hours.

Method D is a method for producing a compound represented by general formula (VIIc) in which X is a single bond among compounds represented by general formula (VII) used in the aforementioned step A-V of Method A.

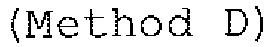

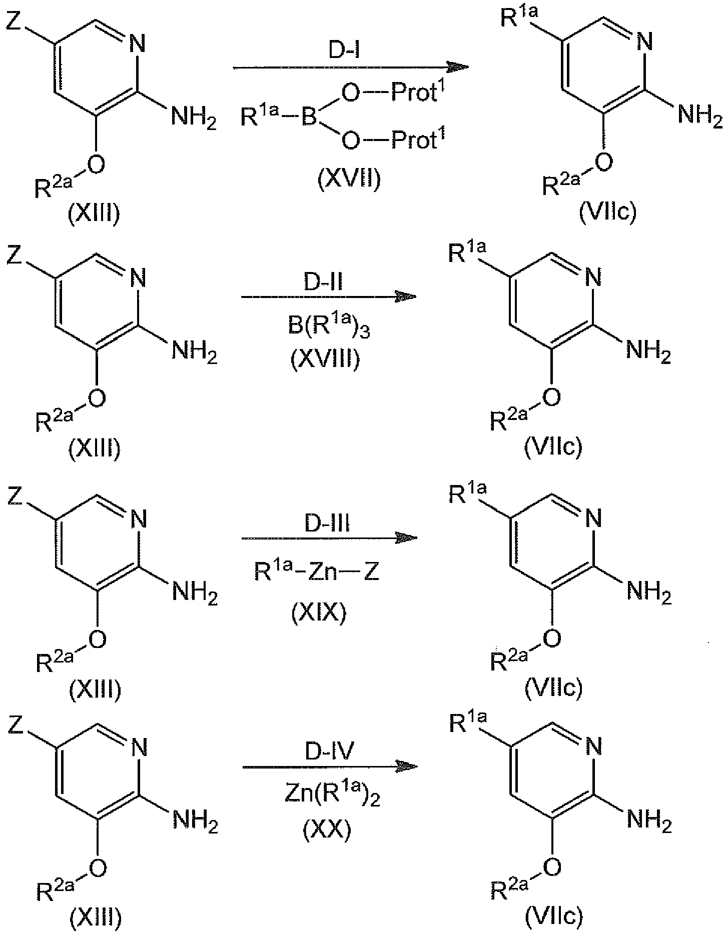

### Step D-1:

This step is a step for producing a compound represented by general formula (VIIc) by reacting a compound represented by general formula (XIII) with a compound represented by general formula (XVII) in a solvent and in the presence of a palladium catalyst and a base (Suzuki-Miyaura coupling).

The compound represented by general formula (XVII) is either a known compound or is easily produced in accordance with known methods or methods similar thereto by using known compounds for the starting raw materials.

The solvent used in this step is preferably a halogenated hydrocarbon, an ether, an aromatic hydrocarbon, an amide, water or a mixed solvent thereof, more preferably dichloromethane, tetrahydrofuran, toluene, N,N-dimethylformamide, water or a mixed solvent thereof and even more preferably a mixed solvent of toluene and water.

The palladium catalyst used in this step is preferably tetrakis(triphenylphosphine)palladium (0).

The base used in this step is preferably an alkali metal carbonate, an alkali metal hydrogencarbonate or an alkali metal hydroxide, more preferably sodium carbonate, potassium carbonate, sodium hydrogencarbonate or sodium hydroxide and even more preferably sodium carbonate.

The reaction temperature in this step is a temperature within the range of 0°C to the heating reflux temperature, and the heating reflux temperature varies depending on the solvent. The reaction temperature is normally 0°C to 150°C and preferably 10°C to 120°C.

The reaction time in this step is normally 30 minutes to 24 hours and preferably 1 hour to 8 hours.

The reaction of this step can also be carried out while leaving the hydroxy group of the compound represented by general formula (XVII) unprotected.

### Step D-II:

This step is carried out in the same manner as the above-mentioned step D-1 of Method D, and is a step for producing a compound represented by general formula (VIIc) by reacting a compound represented by general formula (XIII) with a compound represented by general formula (XVIII) in a solvent and in the presence of a palladium catalyst and a base (Suzuki-Miyaura coupling).

The compound represented by general formula (XVIII) is either a known compound or is easily produced in accordance with known methods or methods similar thereto by using known compounds for the starting raw materials.

### Step D-III:

This step is a step for producing a compound represented by general formula (VIIc) by reacting a compound represented by general formula (XIII) with a compound represented by general formula (XIX) in a solvent and in the presence of a palladium catalyst (Negishi coupling).

The compound represented by general formula (XIX) is either a known compound or is easily produced in accordance with known methods or similar methods thereto by using known compounds for the starting raw materials.

The solvent used in this step is preferably an ether, an aromatic hydrocarbon or a mixed solvent thereof, more preferably tetrahydrofuran, toluene or a mixed solvent thereof and even more preferably tetrahydrofuran.

The palladium catalyst used in this step is preferably tetrakis(triphenylphosphine)palladium (0) or palladium acetate (II).

In this step, a phosphine derivative may be added as palladium ligand as necessary. The ligand used is preferably 2-dicyclohexylphosphino-2',6'-dimethoxybiphenyl.

The reaction temperature in this step is a temperature within the range of 0°C to the heating reflux temperature, and the heating reflux temperature varies depending on the solvent. The reaction temperature is normally 0°C to 150°C and preferably 10°C to 120°C.

The reaction time in this step is normally 30 minutes to 24 hours and preferably 1 hour to 8 hours.

### Step D-IV:

This step is carried out in the same manner as the above-mentioned step D-III of Method D by reacting a compound represented by general formula (XIII) with a compound represented by general formula (XX) in a solvent and in the presence of a palladium catalyst (Negishi coupling), and is a step for producing a compound represented by general formula (VIIc).

The compound represented by general formula (XX) is either a known compound or is easily produced in accordance with known methods or methods similar thereto by using known compounds for the starting raw materials.

Method E is a method for producing a compound represented by general formula (VIId) in which X is a single bond and R^{1a} is R^{9a}CH₂CH₂ among compounds represented by general formula (VII) used in the above-mentioned step A-V of Method A.

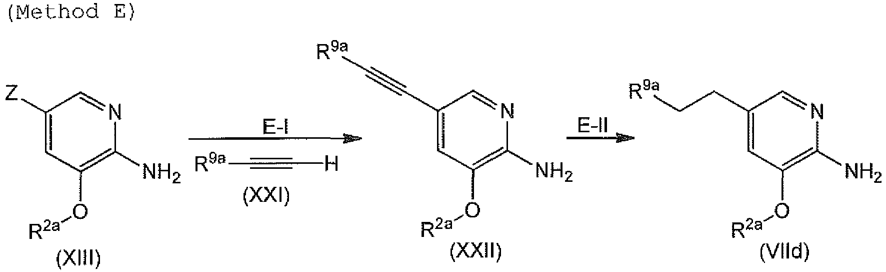

### Step E-I:

This step is a step for producing a compound represented by general formula (XXII) by reacting a compound represented by general formula (XIII) with a compound represented by general formula (XXI) in a solvent and in the presence of a palladium catalyst and/or a copper catalyst and a base (Sonogashira coupling).

The compound represented by general formula (XXI) is either a known compound or is easily produced in accordance with known methods or methods similar thereto by using known compounds for the starting raw materials.

The solvent used in this step is preferably an halogenated hydrocarbon, an ether, an aromatic hydrocarbon, an amide, water or a mixed solvent thereof, more preferably dichloromethane, tetrahydrofuran, toluene, N,N-dimethylformamide, N,N-dimethylacetamide, water or a mixed solvent thereof and even more preferably a mixed solvent of N,N-dimethylacetamide and water.

The palladium catalyst used in this step is preferably palladium-carbon.

The copper catalyst used in this step is preferably copper iodide.

In this step, a phosphine derivative may be added as palladium ligand as necessary.

The base used in this step is preferably an organic base and more preferably diisopropylethylamine or triethylamine.

The reaction temperature in this step is a temperature within the range of 0°C to the heating reflux temperature, and the heating reflux temperature varies depending on the solvent. The reaction temperature is normally 0°C to 150°C and preferably 10°C to 100°C.

The reaction time in this step is normally 30 minutes to 24 hours and preferably 1 hour to 8 hours.

### Step E-II:

This step is a step for producing a compound represented by general formula (VIId) by catalytically reducing a compound represented by general formula (XXII) under a stream of hydrogen, in a solvent and in the presence of a catalyst.

The solvent used in this step is preferably an alcohol or an ether, more preferably ethanol, methanol or tetrahydrofuran and even more preferably ethanol or methanol.

In this step, an acid may also be added as necessary. The acid is preferably acetic acid or trifluoroacetic acid.

The catalyst used in this step is palladium-carbon, platinum oxide, strontium carbonate, rhodium-alumina or rhodium-carbon and preferably palladium-carbon.

The amount of catalyst used in this step is normally 1% (w/w) to 50% (w/w) and more preferably 3% (w/w) to 20% (w/w) based on the weight of the compound represented by general formula (XXII).

The reaction temperature in this step is a temperature within the range of 0°C to the heating reflux temperature, and the heating reflux temperature varies depending on the solvent. The reaction temperature is normally 0°C to 100°C and preferably 20°C to 60°C.

The reaction time in this step is normally 3 hours to 24 hours and preferably 5 hours to 8 hours.

The hydrogen pressure in this step is normally from normal pressure to 10 MPa and preferably from normal pressure to 1.5 MPa.

Method F is a method for producing a compound represented by general formula (Vc) in which X is a single bond among compounds represented by general formula (V) used in the above-mentioned step A-III of Method A.

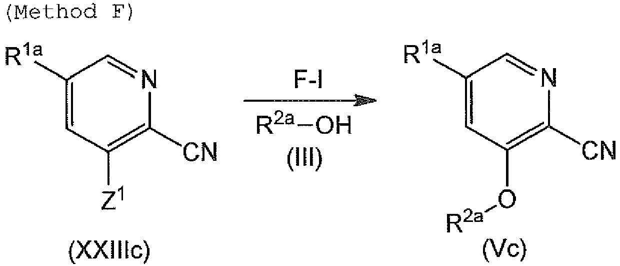

### Step F-I:

This step is carried out in the same manner as the above-mentioned step A-I of Method A by reacting a compound represented by general formula (XXIIIc) with a compound represented by general formula (III) in a solvent and in the presence of a base, and is a step for producing a compound represented by general formula (Vc).

The compound represented by general formula (XXIIIc) is either a known compound, or is easily produced in accordance with known methods or methods similar thereto by using known compounds for the starting raw materials.

In the above descriptions, a protecting group of an "optionally protected amino group", "optionally protected hydroxy group" and "optionally protected carboxyl group" in the definitions of R^{1a}, R^{2a} and R^{9a} refers to a protecting group able to be cleaved by a chemical method such as hydrogenolysis, hydrolysis, electrolysis or photolysis that is commonly used in organic synthetic chemistry (refer to, for example, T.W. Greene, et al., Protective Groups in Organic Synthesis, 3rd Edition, John Wiley & Sons, Inc. (1999)).

In the above descriptions, there are no particular limitations on the "protecting group" of an "optionally protected hydroxy group" in the definitions of R^{1a}, R^{2a} and R^{9a} provided it is a hydroxy group protecting group used in the field of organic synthetic chemistry, and examples include "alkylcarbonyl groups" such as a formyl group, the above-mentioned "C₂-C₇ alkylcarbonyl group", C₂-C₇ halogenated alkylcarbonyl groups such as 2,2,2-trichloroethylcarbonyl, alkoxyalkylcarbonyl groups such as methoxyacetyl, and unsaturated alkylcarbonyl groups such as acryloyl, propioloyl, methacryloyl, crotonoyl, isocrotonoyl and (E)-2-methyl-2-butenoyl; "arylcarbonyl groups" such as an arylcarbonyl groups such as benzoyl, α-naphthoyl and β-naphthoyl, halogenated arylcarbonyl groups such as 2-bromobenzoyl and 4-chlorobenzoyl, C₂-C₆ alkylated arylcarbonyl groups such as 2,4,6-trimethylbenzoyl and 4-toluoyl, C₁-C₆ alkoxylated arylcarbonyl groups such as 4-anisoyl, nitroarylcarbonyl groups such as 4-nitrobenzoyl and 2-nitrobenzoyl, C₂-C₇ alkoxycarbonylated arylcarbonyl groups such as 2-(methoxycarbonyl)benzoyl, and arylated arylcarbonyl groups such as 4-phenylbenzoyl; "alkoxycarbonyl groups" such as the above-mentioned "C₂-C₇ alkoxycarbonyl group", and C₂-C₇ alkoxycarbonyl groups substituted with a halogen or tri(C₁-C₆ alkyl)silyl such as 2,2,2-trichloroethoxycarbonyl and 2-trimethylsilylethoxycarbonyl group; "tetrahydropyranyl or tetrahydrothiopyranyl groups" such as tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl; "tetrahydrofuranyl or tetrahydrothiofuranyl groups" such as tetrahydrofuran-2-yl and tetrahydrothiofuran-2-yl; "silyl groups" such as tri(C₁-C₆ alkyl)silyl groups such as trimethylsilyl, triethylsilyl, isopropyldimethylsilyl, t-butyldimethylsilyl, methyldiisopropylsilyl, methyl-di-t-butylsilyl and triisopropylsilyl, and (C₁-C₆ alkyl)diarylsilyl and di-(C₁-C₆ alkyl)arylsilyl groups such as diphenylmethylsilyl, diphenylbutylsilyl, diphenylisopropylsilyl and phenyldiisopropylsilyl; "alkoxymethyl groups" such as a (C₁-C₆ alkoxy)methyl groups such as methoxymethyl, 1,1-dimethyl-1-methoxymethyl, ethoxymethyl, propoxymethyl, isopropoxymethyl, butoxymethyl and t-butoxymethyl, (C₁-C₆ alkoxy)-(C₁-C₆ alkoxy)methyl groups such as 2-methoxyethoxymethyl, and (C₁-C₆ halogenated alkoxy)methyl groups such as 2,2,2-trichloroethoxymethyl and bis(2-chloroethoxy)methyl; "substituted ethyl groups" such as a (C₁-C₆ alkoxy)ethyl groups such as 1-ethoxyethyl and 1-(isopropoxy)ethyl, and halogenated ethyl groups such as 2,2,2-trichloroethyl; "aralkyl groups" such as a C₁-C₆ alkyl groups substituted with 1 to 3 aryl group(s) such as benzyl, α-naphthylmethyl, β-naphthylmethyl, diphenylmethyl, triphenylmethyl, α-naphthyldiphenylmethyl and 9-anthrylmethyl, and a C₁-C₆ alkyl groups substituted with 1 to 3 aryl group(s) in which an aryl ring is substituted with a C₁-C₆ alkyl, C₁-C₆ alkoxy, nitro, halogen or cyano group such as 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl and 4-cyanobenzyl; "alkenyloxycarbonyl groups" such as a vinyloxycarbonyl and allyloxycarbonyl; and, "aralkyloxycarbonyl groups" in which an aryl ring is optionally substituted with 1 or 2 C₁-C₆ alkoxy or nitro group(s) such as a benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl, and preferably alkylcarbonyl groups, silyl groups or aralkyl groups.

In the above descriptions, there are no particular limitations on the "protecting group" of an "optionally protected carboxyl group" in the definitions of R^{1a}, R^{2a} and R^{9a} provided it is a carboxyl group protecting group used in the field of organic synthetic chemistry, and examples include the above-mentioned "C₁-C₆ alkyl group"; C₂-C₆ alkenyl groups such as vinyl or allyl; C₂-C₆ alkynyl groups such as ethynyl, 1-propynyl, 2-propynyl, 1-methyl-2-propynyl and 1-butynyl group; the above-mentioned "C₁-C₆ halogenated alkyl group"; the above-mentioned "C₁-C₆ hydroxyalkyl group"; (C₂-C₇ alkylcarbonyl)-(C₁-C₆ alkyl) groups such as acetylmethyl; the above-mentioned "aralkyl groups", and the above-mentioned "silyl groups", and preferably C₁-C₆ alkyl groups or aralkyl groups.

In the above descriptions, there are no particular limitations on the "protecting group" of an "optionally protected amino group" in the definitions of R^{1a}, R^{2a} and R^{9a} provided it is an amino group protecting group used in the field of organic synthetic chemistry, and examples include groups similar to the "alkylcarbonyl groups", "arylcarbonyl groups", "alkoxycarbonyl groups", "silyl groups", "aralkyl groups", "alkenyloxycarbonyl groups" and "aralkyloxycarbonyl groups" in the previously listed "hydroxy group protecting groups", and "substituted methylene groups that form a Schiff's base" such as N,N-dimethylaminomethylene, benzylidene, 4-methoxybenzylidene, 4-nitrobenzylidene, salicylidene, 5-chlorosalicylidene, diphenylmethylene and (5-chloro-2-hydroxyphenyl)phenylmethylene, preferably alkylcarbonyl groups, arylcarbonyl groups or alkoxycarbonyl groups, and more preferably alkoxycarbonyl groups.

The steps requiring protection/deprotection are performed according to known methods (for example, the methods described in Theodora W. Greene, Peter G. M. Wuts, "Protective Groups in Organic Synthesis," 1999, A Wiley-Interscience Publication, etc.).

The compound or pharmacologically acceptable salt thereof of the present invention can be administered in various forms. Examples of the route of administration include oral administration using tablets, capsules, granules, emulsions, pills, powders, syrups (solutions), and the like and parenteral administration using injections (intravenous, intramuscular, subcutaneous, or intraperitoneal administration), drip infusions, suppositories (rectal administration), and the like. These various formulations can be prepared as drug products according to usual methods using aids usually used in the field of drug formulation such as excipients, binders, disintegrants, lubricants, flavoring agents, dissolving aids, suspending agents, and coating agents in addition to an active ingredient. In the use as a tablet, examples of carriers that can be used include excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, potassium phosphate, and polyvinylpyrrolidone; disintegrants such as dry starch, sodium alginate, agar powder, laminaran powder, sodium hydrogencarbonate, calcium carbonate, polyoxyethylene sorbitan fatty acid esters, sodium lauryl sulfate, stearic monoglyceride, starch, and lactose; disintegration inhibitors such as sucrose, stearin, cocoa butter, and hydrogenated oil; absorption enhancers such as quaternary ammonium salts and sodium lauryl sulfate; humectants such as glycerine and starch; adsorbents such as starch, lactose, kaolin, bentonite, and colloidal silicic acid; lubricants such as purified talc, stearate, fluoboric acid powder, and polyethylene glycol, and so forth. Furthermore, tablets coated in usual ways such as, for example, sugar-coated tablets, gelatin-coated tablets, enteric-coated tablets, film-coated tablets, double-layer tablets, and multilayered tablets can be prepared as required.

In the use as a pill, examples of carriers that can be used include excipients such as glucose, lactose, cocoa butter, starch, hydrogenated vegetable oil, kaolin, and talc; binders such as powdered gum arabic, powdered tragacanth, gelatin, and ethanol; disintegrants such as laminaran, and agar, and so forth.

In the use as a suppository, a wide range of carriers known in this field can be used, and examples thereof include polyethylene glycol, cocoa butter, higher alcohols, higher alcohol esters, gelatin, semisynthetic glycerides, and so forth.

In the use as an injection, the formulations can be prepared as solutions, emulsions, or suspensions. Preferably, these solutions, emulsions, and suspensions are sterilized and are isotonic with blood. Solvents for producing these solutions, emulsions, and suspensions are not particularly limited so long as they can be used as diluents for medical use, and examples thereof include water, ethanol, propylene glycol, ethoxylated isostearyl alcohol, polyoxylated isostearyl alcohol, polyoxy ethylene sorbitan fatty acid esters, and so forth. In this case, a sufficient amount of sodium chloride, glucose, or glycerine may be added to the formulation to prepare an isotonic solution, and usual dissolving aids, buffers, soothing agents, and the like may also be added.

Furthermore, coloring agents, preservatives, perfumes, flavoring agents, sweeteners, and the like can be added to the above-mentioned formulation, if necessary. Furthermore, other drugs can also be added.

The amount of active ingredient compound contained in the above-mentioned formulations is not particularly limited, but is usually 0.5 to 70% by weight of the total composition, preferably 1 to 30% by weight.

The dose varies depending on symptoms, age, and the like of the patient (a warm-blooded animal, in particular, a human). In the case of oral administration, the recommended dosage per administration is from 0.001 mg/kg body weight as the lower limit (preferably 0.01 mg/kg body weight) to 500 mg/kg body weight as the upper limit (preferably 50 mg/kg body weight); in case of intravenous administration, the recommended dosage per administration is from 0.005 mg/kg body weight as the lower limit (preferably 0.05 mg/kg body weight) to 50 mg/kg body weight as the upper limit (preferably 5 mg/kg body weight), which are desirably administered from 1 to several times per day depending on the symptoms.

### EXAMPLES

Although the following provides a more detailed explanation of the present invention through examples and test examples thereof, the scope of the present invention is not limited thereby.

Elution in column chromatography indicated in the examples was carried out under observation by thin layer chromatography (TLC). During TLC observations, Silica Gel 60F₂₅₄ manufactured by Merck was used for the TLC plate, the solvent used for the elution solvent in column chromatography was used for the developing solvent, and a UV detector was used for the detection method. Silica Gel SK-85 (230 to 400 mesh) manufactured by Merck or Silica Gel FL100B manufactured by Fuji Silysia Chemical was used for the column silica gel. An automated chromatography system (Purif-α2) and a disposable column (Purif-pack) manufactured by Shoko Scientific Co., Ltd., as well as an automated preparative device manufactured by Yamazen Corp. were also suitably used in addition to ordinary column chromatography. Furthermore, the abbreviations used in the examples have the meanings indicated below:
mg: milligrams, g: grams, mL: milliliters, MHz: megahertz, Hz: hertz.

In the following examples, nuclear magnetic resonance (referred to as "¹H-NMR") spectra were obtained using tetramethylsilane for the standard substance, and chemical shift values are indicated with δ values (ppm). Deuterated chloroform (CDCl₃) or deuterated dimethyl-sulfoxide (DMSO-d₆) was used for the measuring solvent. In the splitting patterns, a singlet is indicated with an "s", a doublet with a "d", a triplet with a "t", a quartet with a "q", a quintet with a "quint", a sextet with a "sext", a heptet with a "hept", a multiplet with an "m", and a broad pattern with "br".

Mass spectrometry (referred to as "MS" hereinafter) was carried out with the Fast Atom Bombardment (FAB) method, Electron Ionization (EI) method or Electron Spray Ionization (ESI) method.

LCMS was carried out using the Agilent 1100 Series LC/MSD system. Analysis conditions were as indicated below:
Column: Intakt Cadenza CD-C18 (3 µm)
Solvent A: 0.1% aqueous ammonium acetate solution
Solvent B: Acetonitrile
Program: 0 to 1.5 min (A:B = 70:30 to 0:100, linear gradient), 0.8 mL/min; 1.5 to 3.8 min (A:B = 0:100), 0.8 mL/min; 3.8 to 4.5 min (A:B = 70:30), 1.2 mL/min
Detection: UV, 254 nm

### (Example 1)

### [3-(3,4-Difluorophenoxy)-5-(pyridin-2-ylsulfanyl)pyridin-2-yl]-[5-(1H-tetrazol-5-yl)pyridin-2-yl]amine

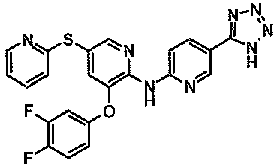

### (1a) 3-(3,4-Difluorophenoxy)-5-(pyridin-2-ylsulfanyl)pyridine-2-carbonitrile

To an N,N-dimethylformamide solution (12 mL) of 3,4-difluorophenol (1.6 g, 12.3 mmol), sodium hydride (content 63%)(0.49 g, 12.9 mmol) was added under a nitrogen stream at 0°C, followed by stirring for 10 minutes. Subsequently, to the reaction solution, 5-bromo-3-nitropyridine-2-carbonitrile (2.8 g, 12.3 mmol) was added, followed by stirring for 30 minutes.
To the reaction solution, 2-mercaptopyridine (1.37 g, 12.3 mmol) was added, and subsequently sodium hydride (content 63%) (0.47 g, 12.3 mmol) was added again, followed by stirring at room temperature overnight. The reaction solution was poured into a saturated aqueous ammonium chloride solution, and extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane-ethyl acetate) to afford the desired title compound (3.07 g, 74 %).
¹H-NMR (CDCl₃, 400 MHz) δ: 8.43-8.42 (1H m), 8.41 (1H, d, J = 2.0 Hz), 7.67-7.62 (1H, dt, J = 7.4, 1.9 Hz), 7.45 (1H, d, J = 1.5 Hz), 7.34-7.32 (1H, m), 7.24-7.18 (2H, m), 7.04-6.99 (1H, m), 6.91-6.87 (1H, m).

### (1b) 3-(3,4-Difluorophenoxy)-5-(pyridin-2-ylsulfanyl)pyridine-2-carboxamide

The compound (3.0 g, 8.8 mmol) obtained in Example (1a) was dissolved in sulfuric acid (6 mL) under a nitrogen stream, followed by stirring at 50°C for 1.5 hours. After the reaction solution was ice-cooled and neutralized with an aqueous sodium hydroxide solution to pH 6, extraction was carried out with ethyl acetate, and the extract was washed with water and brine sequentially. The resulting organic layer was dried over anhydrous sodium sulfate and subsequently concentrated under reduced pressure to afford a crude product (3.0 g) of the desired title compound, which was used in the next step without further purification.

### (1c) 2-Amino-3-(3,4-difluorophenoxy)-5-(pyridin-2-ylsulfanyl)pyridine

To a 2N-aqueous sodium hydroxide solution (16.6 mL), bromine (0.51 mL, 10 mmol) was added at 0°C, followed by stirring at room temperature for 15 minutes. To a 1,4-dioxane solution (25 mL) of the compound (3.0 g, 8.3 mmol) obtained in Example (1b), the adjusted reagent was added dropwise at room temperature, followed by stirring for 1 hour. The reaction solution was ice-cooled, and subsequently concentrated hydrochloric acid (4.5 mL) was added, followed by stirring for 5 minutes and neutralizing with a saturated aqueous sodium hydrogencarbonate solution under ice cooling. The product was extracted with ethyl acetate and the extract was washed with water and brine sequentially. The resulting organic layer was dried over anhydrous sodium sulfate and subsequently concentrated under reduced pressure to give a residue. The resulting residue was purified using silica gel column chromatography (hexane-ethyl acetate) to afford the desired title compound (2.2 g, 80 %).
¹H-NMR (CDCl₃, 400 MHz) δ: 8.38-8.37 (1H, m), 8.08 (1H, d, J = 2.0 Hz), 7.48 (1H, ddd, J = 9.4, 7.4, 2.0 Hz), 7.21 (1H, d, J = 1.9 Hz), 7.15 (1H, dd, J = 18.4, 8.6 Hz), 7.00 (1H, m), 6.93-6.91 (2H, m), 6.80-6.78 (1H, m), 4.97 (2H, brs).

### (1d) 6-[3-(3,4-Difluorophenoxy)-5-(pyridin-2-ylsulfanyl)pyridin-2-ylamino]nicotinonitrile

The compound (1.0 g, 3.0 mmol) obtained in Example (1c), 6-chloronicotinonitrile (0.42 g, 3.0 mmol), cesium carbonate (2.1 g, 6.0 mmol), tris(dibenzylideneacetone)dipalladium complex (0.14 g, 0.15 mmol), and 4,5-bis(diphenylphosphino)-9,9-dimethylxanthene (0.43 g, 0.75 mmol) were dissolved in 1,4-dioxane (10 mL) under a nitrogen stream at room temperature, followed by stirring at 100°C for 10 minutes. Subsequently, water (0.11 mL, 6.0 mmol) was added, followed by further stirring for 3 hours. The reaction solution was poured into a saturated aqueous ammonium chloride solution, and extraction was carried out with ethyl acetate, and the extract was washed with water and brine sequentially. The resulting organic layer was dried over anhydrous sodium sulfate and subsequently concentrated under reduced pressure to give a residue. The resulting residue was dissolved in a dichloromethane-methanol solution (9:1, v/v), silica gel (5 g) was added, and the solvent was distilled off. The resulting mixture was purified using silica gel column chromatography (hexane-ethyl acetate) to afford the desired title compound (1.2 g, 93 %).
¹H-NMR (DMSO-d₆, 400 MHz) δ: 9.68 (1H, s), 8.68 (1H, dd, J = 1.4, 1.2 Hz), 8.38 (1H, ddd, J = 4.7, 2.0, 0.8 Hz), 8.29 (1H, d, J = 2.0 Hz), 8.22-8.15 (2H, m), 7.70-7.65 (1H, m), 7.65-7.55 (1H, m), 7.55 (1H, d, J = 2.0 Hz), 7.49-7.47 (1H, m), 7.38 (1H, ddd, J = 11.7, 7.0, 3.1 Hz), 7.18-7.14 (2H, m), 7.05-7.00 (1H, m).

### (1e) [3-(3,4-Difluorophenoxy)-5-(pyridin-2-ylsulfanyl)pyridin-2-yl]-[5-(1H-tetrazol-5-yl)pyridin-2-yl]amine

A toluene solution (1 mL) of the compound (0.87 g, 2.0 mmol) obtained in Example (1d) and tributyltin azide (1.1 mL, 4.0 mmol) was heated to reflux under a nitrogen stream at 110°C for 9 hours. The reaction solution was concentrated, followed by purifying using silica gel column chromatography (hexane-ethyl acetate → ethyl acetate-methanol). Further, the resulting compound was suspended in an ethyl acetate-diethyl ether mixture. By filtering, the desired title compound (0.52 g, 55 %) was afforded.
¹H-NMR (DMSO-d₆, 500 MHz) δ: 9.38 (1H, s), 8.91 (1H, s), 8.41-8.35 (3H, m), 8.30 (1H, s), 7.69-7.66 (1H, m), 7.53-7.47 (2H, m), 7.43-7.40 (1H, m), 7.18-7.14 (2H, m), 7.07-7.05 (1H, m); LCMS (ESI, m/z): 477 (M+H)⁺, retention time: 2.0 min.

### (Example 2)

### [3-(4-Fluorophenoxy)-5-(pyridin-2-ylsulfanyl)pyridin-2-yl]-[5-(1H-tetrazol-5-yl)pyridin-2-yl]amine

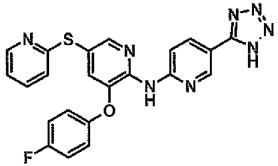

### (2a) 3-(4-Fluorophenoxy)-5-(pyridin-2-ylsulfanyl)pyridine-2-carbonitrile

Analogously to Example (1a), the desired title compound (11.2 g, 87 %) was obtained from 4-fluorophenol (4.5 g, 40 mmol).
¹H-NMR (CDCl₃, 400 MHz) δ: 8.41-8.39 (1H, m), 8.36 (1H, d, J = 2.0 Hz), 7.63 (1H, dt, J = 7.4, 1.6 Hz), 7.35 (1H, d, J = 2.0 Hz), 7.32-7.30 (1H, m), 7.18 (1H, ddd, J = 7.8, 5.1, 1.2 Hz), 7.13-7.10 (4H, m).

### (2b) 3-(4-Fluorophenoxy)-5-(pyridin-2-ylsulfanyl)pyridine-2-carboxamide

The compound (11.2 g, 34.6 mmol) obtained in Example (2a) was dissolved in ethanol (140 mL) and a 1N-aqueous potassium hydroxide solution (140 mL) was added, followed by stirring at 60°C for 1 hour. After the reaction, the reaction mixture was cooled to room temperature, acetic acid (10 mL) was added, and the solvent was distilled off under reduced pressure. After the residue was dissolved in ethyl acetate, washing was carried out with saturated brine, and the resulting organic layer was dried over anhydrous sodium sulfate and subsequently concentrated under reduced pressure. The resulting residue was purified using silica gel column chromatography (hexane-ethyl acetate → ethyl acetate-methanol) to afford the desired title compound (6.9 g, 58%).
¹H-NMR (CDCl₃, 400 MHz) δ: 8.41-8.39 (1H, m), 8.38 (1H, d, J = 1.6 Hz), 7.61-7.57 (1H, dt, J = 7.4, 2.0 Hz), 7.41 (1H, d, J = 2.0 Hz), 7.22 (1H, dt, J = 9.0, 0.8 Hz), 7.13 (1H, ddd, J = 7.8, 5.1, 1.2 Hz), 7.06 (2H, d, J = 1.6 Hz), 7.05 (2H, s), 5.64 (2H, brs).

### (2c) 2-Amino-3-(4-fluorophenoxy)-5-(pyridin-2-ylsulfanyl)pyridine

Analogously to Example (1c), the desired title compound (0.74 g, 79 %) was obtained from the compound (1.0 g, 3.2 mmol) obtained in Example (2b).
¹H-NMR (CDCl₃, 400 MHz) δ: 8.38-8.36 (1H, m), 8.04 (1H, d, J = 2.0 Hz), 7.46 (1H, dt, J = 7.4, 2.0 Hz), 7.11 (1H, d, J = 2.0 Hz), 7.08-6.96 (5H, m), 6.89-6.87 (1H, m), 5.02 (2H, brs).

### (2d) 6-[3-(4-Fluorophenoxy)-5-(pyridin-2-ylsulfanyl)pyridin-2-ylamino]nicotinonitrile

Analogously to Example (1d), the desired title compound (0.37 g, 65 %) was obtained from the compound (0.43 g, 1.37 mmol) obtained in Example (2c).
LCMS (ESI, m/z): 416 (M+H)⁺, retention time: 2.9 min.

### (2e) [3-(4-Fluorophenoxy)-5-(pyridin-2-ylsulfanyl)pyridin-2-yl]-[5-(1H-tetrazol-5-yl)pyridin-2-yl]amine

Analogously to Example (1e), the desired title compound (0.23 g, 58 %) was obtained from the compound (0.36 g, 0.87 mmol) obtained in Example (2d).
¹H-NMR (DMSO-d₆, 400 MHz) δ: 9.28 (1H, brs), 8.92-8.90 (1H, m), 8.44-8.42 (1H, m), 8.37-8.35 (2H, m), 8.26 (1H, d, J = 2.0 Hz) 7.67 (1H, ddd, J = 9.4, 7.4, 2.0 Hz), 7.34 (1H, d, J = 2.0 Hz), 7.28 (2H, d, J = 2.7 Hz), 7.26 (2H, s), 7.16 (1H, ddd, J = 7.4, 5.1, 1.2 Hz), 7.11 (1H, dt, J = 8.2, 0.8 Hz);
LCMS (ESI, m/z): 459 (M+H)⁺, retention time: 2.0 min.

### (Example 3)

### [3-(2-Chloro-4-fluorophenoxy)-5-(pyridin-2-ylsulfanyl)pyridin-2-yl]-[5-(1H-tetrazol-5-yl)pyridin-2-yl]amine

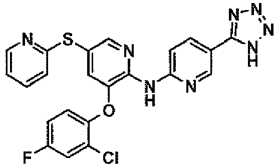

### (3a) 3-(2-Chloro-4-fluorophenoxy)-5-(pyridin-2-ylsulfanyl)pyridine-2-carbonitrile

Analogously to Example (1a), the desired title compound (3.1 g, 86 %) was obtained from 2-chloro-4-fluorophenol (1.05 g, 10 mmol).
¹H-NMR (CDCl₃, 400 MHz) δ: 8.91-8.39 (2H, m), 7.62 (1H, dt, J = 7.8, 2.0 Hz), 7.30-7.25 (2H, m), 7.23-7.16 (3H, m), 7.07 (1H, ddd, J = 10.2, 7.4, 2.7 Hz).

### (3b) 3-(2-Chloro-4-fluorophenoxy)-5-(pyridin-2-ylsulfanyl)pyridine-2-carboxamide

Analogously to Example (1b), a crude purified product (3.0 g) of the desired title compound was obtained from the compound (3.1 g, 8.7 mmol) obtained in Example (3a), which was used in the next step without further purification.

### (3c) 2-Amino-3-(2-chloro-4-fluorophenoxy)-5-(pyridin-2-ylsulfanyl)pyridine

Analogously to Example (1c), the desired title compound (2.3 g, 82 %) was obtained from the compound (3.0 g, 8.0 mmol) obtained in Example (3b).
¹H-NMR (CDCl₃, 400 MHz) δ: 8.37 (1H, ddd, J = 5.1, 2.0, 0.8 Hz), 8.04 (1H, d, J = 2.0 Hz), 7.45 (1H, ddd, J = 9.4, 7.4, 2.0 Hz), 7.22 (1H, dd, J = 7.8, 2.7 Hz), 7.10 (1H, dd, J = 9.0, 5.1 Hz), 7.03-6.96 (2H, m), 6.93 (1H, d, J = 1.6 Hz), 6.84 (1H, dt, J = 8.2, 1.2 Hz), 5.09 (2H, brs).

### (3d) 6-[3-(2-Chloro-4-fluorophenoxy)-5-(pyridin-2-ylsulfanyl)pyridin-2-ylamino]nicotinonitrile

Analogously to Example (1d), the desired title compound (1.15 g, 85 %) was obtained from the compound (1.04 g, 3.0 mmol) obtained in Example (3c).
¹H-NMR (DMSO-d₆, 500 MHz) δ: 9.67-9.65 (1H, m), 8.72 (1H, s), 8.37-8.36 (1H, m), 8.27-8.24 (2H, m), 8.21-8.19 (1H, m), 8.69-8.64 (2H, m), 7.44-7.41 (1H, m), 7.33-7.29 (1H, m), 7.24 (1H, t, J = 2.0 Hz), 7.18-7.15 (1H, m), 7.08 (1H, dd, J = 8.3, 1.0 Hz).

### (3e) [3-(2-Chloro-4-fluorophenoxy)-5-(pyridin-2-ylsulfanyl)pyridin-2-yl]-[5-(1H-tetrazol-5-yl)pyridin-2-yl]amine

Analogously to Example (1e), the desired title compound (0.75 g, 76 %) was obtained from the compound (0.90 g, 2.0 mmol) obtained in Example (3d).
¹H-NMR (DMSO-d₆, 400 MHz) δ: 9.32 (1H, s), 8.92 (1H, dd, J = 2.4, 0.8 Hz), 8.44 (1H, dd, J = 8.6, 0.8 Hz), 8.40-8.36 (2H, m), 8.26 (1H, d, J = 2.0 Hz) 7.69-7.64 (2H, m), 7.45 (1H, dd, J = 9.0, 5.1 Hz), 7.32 (1H, ddd, J = 11.0, 7.8, 3.1 Hz), 7.19 (1H, d, J = 2.0 Hz), 7.15 (1H, ddd, J = 7.4, 4.7, 1.8 Hz), 7.06 (1H, dt, J = 8.2, 0.8 Hz);
LCMS (ESI, m/z): 493, 495 (M+H)⁺, retention time: 2.0 min.

### (Example 4)

### [3-(2,4-Difluorophenoxy)-5-(pyridin-2-ylsulfanyl)pyridin-2-yl]-[5-(1H-tetrazol-5-yl)pyridin-2-yl]amine

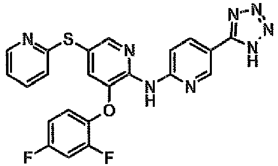

### (4a) 3-(2,4-Difluorophenoxy)-5-(pyridin-2-ylsulfanyl)pyridine-2-carbonitrile

To an N,N-dimethylformamide solution (10 mL) of 2,4-difluorophenol (0.98 g, 7.5 mmol), sodium hydride (content 55%) (360 mg, 8.25 mmol) was added under a nitrogen stream at 0°C, followed by stirring for 10 minutes. Subsequently, to the reaction solution, 5-bromo-3-nitropyridine-2-carbonitrile (1.71 g, 7.5 mmol) was added, followed by stirring for 30 minutes. To the reaction solution, 2-mercaptopyridine (0.83 g, 7.5 mmol) was added, and subsequently sodium hydride (content 55%) (360 mg, 8.25 mmol) was added again, followed by stirring at room temperature for 1.5 hours. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 3:1) to afford the desired title compound (2.06 g, 81 %) as a colorless oil.
¹H-NMR (CDCl₃, 400 MHz) δ: 8.42-8.40 (1H, m), 8.40 (1H, d, J = 1.6 Hz), 7.66 (1H, dt, J = 7.6, 2.0 Hz), 7.32-7.30 (1H, m), 7.30 (1H, d, J = 1.6 Hz), 7.20-7.01 (2H, m), 7.03-6.94 (2H, m).

### (4b) 3-(2,4-Difluorophenoxy)-5-(pyridin-2-ylsulfanyl)pyridine-2-carboxamide

The compound (2.06 g, 6.04 mmol) obtained in Example (4a) was dissolved in sulfuric acid (6 mL) under a nitrogen stream, followed by stirring at room temperature overnight. The reaction solution was poured into water, followed by neutralizing with an aqueous sodium hydroxide solution to pH 6. Subsequently, extraction was carried out with ethyl acetate and the extract was washed with water and brine sequentially. The resulting organic layer was dried over anhydrous sodium sulfate and subsequently concentrated under reduced pressure to give a residue. The resulting residue was purified using silica gel column chromatography (ethyl acetate) to afford the desired title compound (726 mg, 78 %) as a colorless solid.
¹H-NMR (CDCl₃, 500 MHz) δ: 8.40-8.38 (1H, m), 8.39 (1H, d, J = 1.6 Hz), 7.60 (1H, brs), 7.58 (1H, t, J = 7.6 Hz), 7.36 (1H, s), 7.19 (1H, d, J = 1.6 Hz), 7.18-7.12 (2H, m), 6.97-6.87 (2H, m), 5.71 (1H, brs).

### (4c) 2-Amino-3-(2,4-difluorophenoxy)-5-(pyridin-2-ylsulfanyl)pyridine

To a 2N-aqueous sodium hydroxide solution (10.6 mL), bromine (0.30 mL, 5.82 mmol) was added at 0°C, followed by stirring for 15 minutes. To a 1,4-dioxane solution (40 mL) of the compound (1.90 g, 5.29 mmol) obtained in Example (4b), the adjusted reagent was added dropwise at room temperature, followed by stirring for 1 hour. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 1:2) to afford the desired title compound (0.43 g, 25 %) as a brown solid.
¹H-NMR (CDCl₃, 400 MHz) δ: 8.38 (1H, d, J = 5.2 Hz), 8.04 (1H, d, J = 1.6 Hz), 7.47 (1H, dt, J = 7.2, 1.6 Hz), 7.15 (1H, dt, J = 13.8, 4.5 Hz), 7.00-6.86 (2H, m), 6.99 (1H, d, J = 1.6 Hz), 6.87-6.83 (1H, m), 6.83 (1H, d, J = 7.8 Hz), 5.12 (2H, brs).

### (4d) 6-[3-(2,4-Difluorophenoxy)-5-(pyridin-2-ylsulfanyl)pyridin-2-ylamino]nicotinonitrile

The compound (428 mg, 1.29 mmol) obtained in Example (4c), 6-chloronicotinonitrile (197 mg, 1.42 mmol), cesium carbonate (842 mg, 2.58 mmol), tris(dibenzylideneacetone)dipalladium complex (59 mg, 0.06 mmol), and 4,5-bis(diphenylphosphino)-9,9-dimethylxanthene (112 mg, 0.15 mmol) were dissolved in 1,4-dioxane (10 mL) under a nitrogen stream at room temperature, followed by stirring for 10 minutes. Subsequently, water (47 µL, 2.58 mmol) was added, followed by heating and stirring at 100°C for 3 hours. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 3:1) to afford the desired title compound (330 mg, 59 %) as a colorless solid. ¹H-NMR (CDCl₃, 500 MHz) δ: 8.79 (1H, d, J = 8.8 Hz), 8.56 (1H, s), 8.38 (1H, brs), 8.37 (1H, d, J = 5.9 Hz), 8.23 (1H, s), 7.94 (1H, d, J = 8.8 Hz), 7.50 (1H, t, J = 7.8 Hz), 7.24-7.19 (1H, m), 7.12-7.10 (1H, m), 7.04-6.92 (4H, m).

### (4e) [3-(2,4-Difluorophenoxy)-5-(pyridin-2-ylsulfanyl)pyridin-2-yl]-[5-(1H-tetrazol-5-yl)pyridin-2-yl]amine

A toluene solution (0.3 mL) of the compound (287 mg, 0.66 mmol) obtained in Example (4d) and tributyltin azide (440 mg, 1.32 mmol) was heated to reflux under a nitrogen stream at 110°C for 9 hours. The reaction solution was concentrated and subsequently purified using silica gel column chromatography (ethyl acetate) to afford the desired title compound (157 mg, 49 %) as a colorless solid.
¹H-NMR (DMSO-d₆, 400 MHz) δ: 9.46 (1H, s), 8.96 (1H, d, J = 2.4 Hz), 8.45-8.36 (3H, m), 8.27 (1H, d, J = 1.6 Hz), 7.68 (1H, dt, J = 8.8, 2.0 Hz), 7.55-7.42 (2H, m), 7.33 (1H, d, J = 1.6 Hz), 7.20-7.14 (2H, m), 7.08 (1H, d, J = 8.8 Hz);
MS (FAB, m/z): 499 (M+Na)⁺, 477 (M+H)⁺.

### (Example 5)

### {3-[4-(Methylsulfonyl)phenoxy]-5-(pyridin-2-ylsulfanyl)pyridin-2-yl}-[5-(1H-tetrazol-5-yl)pyridin-2-yl]amine

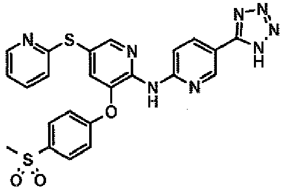

### (5a) 5-Bromo-3-[4-(methylsulfonyl)phenoxy]pyridine-2-carbonitrile

To an N,N-dimethylformamide solution (20 mL) of 4-(methylsulfonyl)phenol (2.0 g, 12 mmol), sodium hydride (content 60%) (511 mg, 13 mmol) was added under a nitrogen stream at 0°C, followed by stirring for 10 minutes. Subsequently, to the reaction solution, 5-bromo-3-nitropyridine-2-carbonitrile (2.8 g, 12 mmol) was added, followed by stirring for 2 hours. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. After the resulting residue was purified using silica gel column chromatography (hexane:ethyl acetate = 1:2), washing was carried out with diisopropyl ether to afford the desired title compound (3.4 g, 83 %) as a pale yellow solid.

¹H-NMR (CDCl₃, 400 MHz) δ: 8.59 (1H, d, J = 2.4 Hz), 8.07 (2H, d, J = 9.0 Hz), 7.52 (1H, d, J = 2.4 Hz), 7.27 (2H, d, J = 9.0 Hz), 3.10 (3H, s).

### (5b) 3-[4-(Methylsulfonyl)phenoxy]-5-(pyridin-2-ylsulfanyl)pyridine-2-carbonitrile

To an N,N-dimethylformamide solution (10 mL) of the compound (1.6 g, 4.5 mmol) obtained in Example (5a) and 2-mercaptopyridine(504 mg, 4.5 mmol), sodium hydride (content 60%) (199 mg, 5.0 mmol) was added under a nitrogen stream at 0°C, followed by stirring at room temperature for 2 hours. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 1:2) to afford the desired title compound (1.4 g, 84 %) as a pale yellow solid.
¹H-NMR (CDCl₃, 500 MHz) δ: 8.51 (1H, s), 8.42 (1H, d, J = 2.4 Hz), 8.01 (2H, d, J = 9.0 Hz), 7.68-7.64 (1H, m), 7.58 (1H, s), 7.36 (1H, dd, J = 9.0, 0.8 Hz), 7.28 (2H, d, J = 9.0 Hz), 7.23-7.19 (1H, m), 3.10 (3H, s).

### (5c) 3-[4-(Methylsulfonyl)phenoxy]-5-(pyridin-2-ylsulfanyl)pyridine-2-carboxamide

The compound (1.8 g, 5.0 mmol) obtained in Example (5b) was dissolved in sulfuric acid (3.0 mL) under a nitrogen stream, followed by stirring at 50°C for 2 hours. Under ice cooling, the reaction solution was poured into ice water, followed by neutralization with a 5N-aqueous sodium hydroxide solution to pH 8. Subsequently, extraction was carried out with methylene chloride and the extract was washed with water and brine sequentially. The resulting organic layer was dried over anhydrous sodium sulfate and subsequently concentrated under reduced pressure to give a residue. The residue was washed with methylene chloride to afford the desired title compound (1.4 g, 91 %) as a white solid.
¹H-NMR (DMSO-d₆, 500 MHz) δ: 8.60 (1H, s), 8.44 (1H, s), 8.06 (1H, s), 7.95-7.89 (3H, m), 7.78-7.73 (1H, m), 7.63 (1H, s), 7.37 (1H, d, J = 9.0 Hz), 7.28-7.23 (1H, m), 7.17 (2H, d, J = 9.0 Hz), 3.20 (3H, s).

### (5d) 2-Amino-3-[4-(methylsulfonyl)phenoxy]-5-(pyridin-2-ylsulfanyl)pyridine

To a 2N-aqueous sodium hydroxide solution (9.0 mL), bromine (0.35 mL, 6.9 mmol) was added at 0°C, followed by stirring for 30 minutes. To a 1,4-dioxane solution (10 mL) of the compound (1.4 g, 3.4 mmol) obtained in Example (5c), the adjusted reagent was added dropwise at room temperature, followed by stirring for 1 hour. Under ice cooling, to the reaction solution, concentrated hydrochloric acid was added, followed by stirring at room temperature for 20 minutes. After neutralizing the mixture with a 5N-aqueous sodium hydroxide solution to pH 8, extraction was carried out with methylene chloride and the extract was washed with water and brine sequentially. The resulting organic layer was dried over anhydrous sodium sulfate and subsequently concentrated under reduced pressure to give a residue. After the resulting residue was purified using silica gel column chromatography (ethyl acetate), washing was carried out with an ethyl acetate-diisopropyl ether mixture to afford the desired title compound (800 mg, 63 %) as a white solid.
¹H-NMR (CDCl₃, 500 MHz) δ: 8.39 (1H, d, J = 2.0 Hz), 8.18 (1H, s), 7.94 (2H, d, J = 9.0 Hz), 7.53-7.49 (1H, m), 7.39 (1H, s), 7.20 (2H, d, J = 9.0 Hz), 7.04-7.01 (1H, m), 6.98 (1H, d, J = 9.0 Hz), 4.93 (2H, brs), 3.07 (3H, s);
MS (FAB, m/z): 374 (M+H)⁺.

### (5e) 6-{3-[4-(Methylsulfonyl)phenoxy]-5-(pyridin-2-ylsulfanyl)pyridin-2-ylamino}nicotinonitrile

The compound (355 mg, 0.95 mmol) obtained in Example (5d), 6-bromonicotinonitrile (261 mg, 1.4 mmol), cesium carbonate (619 mg, 1.9 mmol), tris(dibenzylideneacetone)dipalladium complex (87 mg, 0.10 mmol), and 4,5-bis(diphenylphosphino)-9,9-dimethylxanthene (110 mg, 0.20 mmol) were dissolved in 1,4-dioxane (3 mL) under a nitrogen stream at room temperature, followed by heating and stirring at 100°C for 3 hours. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was washed with a methanol-methylene chloride mixture to afford the desired title compound (226 mg, 51 %) as a pale gray solid. ¹H-NMR (DMSO-d₆, 400 MHz) δ: 9.89 (1H, s), 8.65 (1H, s), 8.43-8.41 (1H, m), 8.39 (1H, d, J = 2.0 Hz), 8.16 (1H, s), 8.15 (1H, s), 7.93 (2H, d, J = 9.0 Hz), 7.78 (1H, d, J = 2.0 Hz), 7.72-7.67 (1H, m), 7.30 (2H, d, J = 9.0 Hz), 7.20-7.16 (2H, m), 3.20 (3H, s);
MS (FAB, m/z): 476 (M+H)⁺.

### (5f) {3-[4-(Methylsulfonyl)phenoxy]-5-(pyridin-2-ylsulfanyl)pyridin-2-yl}-[5-(1H-tetrazol-5-yl)pyridin-2-yl]amine

A toluene solution (5 mL) of the compound (226 mg, 0.48 mmol) obtained in Example (5e) and tributyltin azide (0.26 mL, 0.95 mmol) was heated to reflux under a nitrogen stream at 110°C for 15 hours. The reaction solution was concentrated and subsequently purified using silica gel column chromatography (ethyl acetate:methanol = 9:1) to afford the desired title compound (88 mg, 35 %) as a white solid.
¹H-NMR (DMSO-d₆, 400 MHz) δ: 9.55 (1H, s), 8.87 (1H, dd, J = 2.0, 1.2 Hz), 8.42-8.41 (1H, m), 8.38 (1H, d, J = 2.0 Hz), 8.36-8.34 (2H, m), 7.95 (2H, d, J = 9.0 Hz), 7.76 (1H, d, J = 2.0 Hz), 7.72-7.67 (1H, m), 7.34 (2H, d, J = 9.0 Hz), 7.19-7.16 (2H, m), 3.20 (3H, s);
MS (FAB, m/z): 519 (M+H)⁺.

### (Example 6)

### N,N-Dimethyl-4-{5-(pyridin-2-ylsulfanyl)-2-[5-(1H-tetrazol-5-yl)pyridin-2-ylamino]pyridin-3-yloxy}benzamide

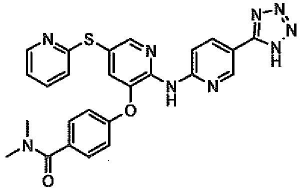

### (6a) 4-[2-Cyano-5-(pyridin-2-ylsulfanyl)pyridin-3-yloxy]-N,N-dimethylbenzamide

Analogously to Example (1a), the desired title compound (2.5 g, 62 %) was obtained as a pale yellow solid from N,N-dimethyl-4-hydroxybenzamide (1.8 g, 11 mmol), 5-bromo-3-nitropyridine-2-carbonitrile (2.5 g, 11 mmol), and 2-mercaptopyridine (1.2 g, 11 mmol).
¹H-NMR (CDCl₃, 500 MHz) δ: 8.43 (1H, s), 8.42 (1H, s), 7.66-7.61 (1H, m), 7.50 (2H, d, J = 9.0 Hz), 7.45 (1H, d, J = 0.8 Hz), 7.33 (1H, d, J = 9.0 Hz), 7.20-7.15 (3H, m), 3.13 (3H, brs), 3.02 (3H, brs);
LCMS (ESI, m/z): 377 (M+H)⁺.

### (6b) 3-[4-(Dimethylcarbamoyl)phenoxy]-5-(pyridin-2-ylsulfanyl)pyridine-2-carboxamide

Analogously to Example (5c), the desired title compound (2.6 g, 100 %) was obtained as a pale yellow solid from the compound (2.5 g, 6.7 mmol) obtained in Example (6a).
¹H-NMR (CDCl₃, 400 MHz) δ: 8.44 (1H, d, J = 2.0 Hz), 8.42-8.41 (1H, m), 7.62-7.57 (2H, m), 7.52 (1H, d, J = 2.0 Hz), 7.48-7.42 (2H, m), 7.26-7.24 (1H, m), 7.16-7.12 (1H, m), 7.09-7.05 (2H, m), 5.62 (1H, brs), 3.10 (3H, brs), 3.00 (3H, brs);
LCMS (ESI, m/z): 395 (M+H)⁺.

### (6c) 4-[2-Amino-5-(pyridin-2-ylsulfanyl)pyridin-3-yloxy]-N,N-dimethylbenzamide

Analogously to Example (5d), the desired title compound (1.4 g, 57 %) was obtained as a pale yellow solid from the compound (2.6 g, 6.7 mmol) obtained in Example (6b).
¹H-NMR (CDCl₃, 500 MHz) δ: 8.38 (1H, dd, J = 2.4, 0.8 Hz), 8.10 (1H, d, J = 0.8 Hz), 7.50-7.43 (4H, m), 7.09-7.05 (2H, m), 7.02-6.98 (1H, m), 6.91 (1H, d, J = 9.0 Hz), 5.01 (2H, brs), 3.12 (3H, brs), 3.02 (3H, brs).

### (6d) 4-[2-(5-Cyanopyridin-2-ylamino)-5-(pyridin-2-ylsulfanyl)pyridin-3-yloxy)-N,N-dimethylbenzamide

The compound (360 mg, 0.98 mmol) obtained in Example (6c), 6-chloronicotinonitrile (204 mg, 1.5 mmol), cesium carbonate (640 mg, 2.0 mmol), tris(dibenzylideneacetone)dipalladium complex (90 mg, 0.10 mmol), and 4,5-bis(diphenylphosphino)-9,9-dimethylxanthene (114 mg, 0.20 mmol) were dissolved in 1,4-dioxane (3 mL) under a nitrogen stream at room temperature, followed by stirring for 1 hour. Subsequently, water (35 µL, 2.0 mmol) was added, followed by heating and stirring at 100°C for 1.5 hours. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (ethyl acetate:methanol = 9:1) to afford the desired title compound (343 mg, 75 %) as a yellow solid. ¹H-NMR (CDCl₃, 400 MHz) δ: 8.79 (1H, dd, J = 9.0, 0.8 Hz), 8.54 (1H, dd, J = 2.4, 0.8 Hz), 8.40-8.36 (1H, m), 8.29 (1H, s), 8.27 (1H, d, J = 2.0 Hz), 7.93 (1H, dd, J = 9.0, 2.4 Hz), 7.51-7.47 (3H, m), 7.36 (1H, d, J = 2.0 Hz), 7.17-7.15 (2H, m), 7.05-7.02 (2H, m), 3.11 (3H, brs), 3.02 (3H, brs);
LCMS (ESI, m/z): 469 (M+H)⁺.

### (6e) N,N-Dimethyl-4-{5-(pyridin-2-ylsulfanyl)-2-[5-(1H-tetrazol-5-yl)pyridin-2-ylamino]pyridin-3-yloxy}benzamide

Analogously to Example (5f), the desired title compound (150 mg, 40 %) was obtained as a white solid from the compound (343 mg, 0.73 mmol) obtained in Example (6d).
¹H-NMR (DMSO-d₆, 400 MHz) δ: 9.32 (1H, s), 8.89 (1H, dd, J = 2.4, 1.2 Hz), 8.41-8.39 (2H, m), 8.36 (1H, dd, J = 9.0, 2.4 Hz), 8.32 (1H, d, J = 2.0 Hz), 7.71-7.66 (1H, m), 7.57 (1H, d, J = 2.0 Hz), 7.49-7.45 (2H, m), 7.22-7.14 (4H, m), 2.94 (6H, brs);
MS (FAB, m/z): 512 (M+H)⁺.

### (Example 7)

### [5-(Pyridin-2-ylsulfanyl)-3-(4-trifluoromethylphenoxy)pyridin-2-yl]-[5-(1H-tetrazol-5-yl)pyxidin-2-yl]amine

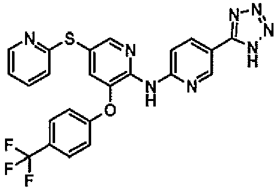

### (7a) 5-(Pyridin-2-ylsulfanyl)-3-(4-trifluoromethylphenoxy)pyridine-2-carbonitrile

Analogously to Example (4a), the desired title compound (1.37 g, 73 %) was obtained as a colorless oil from 5-bromo-3-nitropyridine-2-carbonitrile (1.14 g, 5.0 mmol).
¹H-NMR (CDCl₃, 400 MHz) δ: 8.45 (1H, d, J = 1.6 Hz), 8.42-8.40 (1H, m), 7.85 (2H, d, J = 8.0 Hz), 7.66 (1H, dt, J = 7.6, 2.0 Hz), 7.48 (1H, d, J = 1.6 Hz), 7.35 (1H, d, J = 8.0 Hz), 7.22 (2H, d, J = 8.0 Hz), 7.20-7.18 (1H, m).

### (7b) 5-(Pyridin-2-ylsulfanyl)-3-(4-trifluoromethylphenoxy)pyridine-2-carboxamide

Analogously to Example (4b), the desired title compound (1.11 g, 77 %) was obtained as a colorless solid from the compound (1.37 g, 3.67 mmol) obtained in Example (7a).
¹H-NMR (CDCl₃, 400 MHz) δ: 8.49 (1H, d, J = 2.0 Hz), 8.42 (1H, d, J = 4.8 Hz), 7.64-7.58 (5H, m), 7.29 (1H, d, J = 8.0 Hz), 7.17-7.14 (1H, m), 7.08 (2H, d, J = 7.2 Hz), 5.53 (1H, brs).

### (7c) 2-Amino-5-(pyridin-2-ylsulfanyl)-3-(4-trifluoromethylphenoxy)pyridine

Analogously to Example (4c), the desired title compound (286 mg, 28 %) was obtained as a pale yellow solid from the compound (1.11 g, 2.84 mmol) obtained in Example (7b).
¹H-NMR (CDCl₃, 400 MHz) δ: 8.39 (1H, d, J = 5.9 Hz), 8.14 (1H, d, J = 2.0 Hz), 7.63 (2H, d, J = 9.0 Hz), 7.50 (1H, dt, J = 11.1, 3.9 Hz), 7.32 (1H, d, J = 1.9 Hz), 7.14 (2H, d, J = 9.0 Hz), 7.02-7.00 (1H, m), 6.95 (1H, d, J = 7.8 Hz), 5.00 (2H, brs).

### (7d) 6-[5-(Pyridin-2-ylsulfanyl)-3-(4-trifluoromethylphenoxy)pyridin-2-ylamino]nicotinonitrile

Analogously to Example (4d), the desired title compound (147 mg, 40 %) was obtained as a colorless solid from the compound (285 mg, 0.78 mmol) obtained in Example (7c).
¹H-NMR (CDCl₃, 400 MHz) δ: 8.80 (1H, d, J = 9.0 Hz), 8.55 (1H, d, J = 2.4 Hz), 8.39 (1H, d, J = 4.6 Hz), 8.32 (1H, d, J = 1.9 Hz), 8.26 (1H, brs), 7.95 (1H, dd, J = 9.0, 2.3 Hz), 7.68 (2H, d, J = 9.0 Hz), 7.53 (1H, dt, J = 7.2, 2.0 Hz), 7.40 (1H, d, J = 2.0 Hz), 7.24 (2H, d, J = 9.0 Hz), 7.07 (1H, d, J = 7.0 Hz), 7.06 (1H, d, J = 7.0 Hz).

### (7e) [5-(Pyridin-2-ylsulfanyl)-3-(4-trifluoromethylphenoxy)pyridin-2-yl]-[5-(1H-tetrazol-5-yl)pyridin-2-yl]amine

Analogously to Example (4e), the desired title compound (107 mg, 66 %) was obtained as a colorless solid from the compound (147 mg, 0.32 mmol) obtained in Example (7d).
¹H-NMR (DMSO-d₆, 400 MHz) δ: 9.38 (1H, brs), 8.87 (1H, s), 8.42 (1H, d, J = 4.7 Hz), 8.38-8.32 (3H, m), 7.79 (2H, d, J = 9.0 Hz), 7.72 (1H, d, J = 3.9 Hz), 7.70 (1H, dt, J = 7.2, 2.0 Hz), 7.33 (2H, d, J = 8.6 Hz), 7.19-7.15 (2H, m);
MS (FAB, m/z): 509 (M+H)⁺.

### (Example 8)

### [3-(4-Fluoxophenoxy)-5-(pyrimidin-2-ylsulfanyl)pyridin-2-yl]-[5-(1H-tetrazol-5-yl)pyridin-2-yl]amine

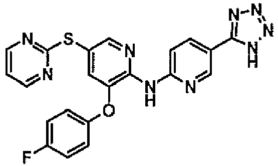

### (8a) 3-(4-Fluorophenoxy)-5-(pyrimidin-2-ylsulfanyl)pyridine-2-carbonitrile

To an N,N-dimethylformamide solution (10 mL) of 4-fluorophenol (0.56 g, 5.0 mmol), sodium hydride (content 55%) (240 mg, 5.5 mmol) was added under a nitrogen stream at 0°C, followed by stirring for 10 minutes. Subsequently, to the reaction solution, 5-bromo-3-nitropyridine-2-carbonitrile (1.14 g, 5.0 mmol) was added, followed by stirring for 30 minutes. To the reaction solution, 2-mercaptopyrimidine (0.56 g, 5.0 mmol) was added, and subsequently sodium hydride (content 55%) (240 mg, 5.5 mmol) was added again, followed by stirring at room temperature overnight. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 4:1) to afford the desired title compound (0.77 g, 48 %) as a pale yellow solid. ¹H-NMR (CDCl₃, 400 MHz) δ: 8.54 (1H, d, J = 1.6 Hz), 8.50 (2H, d, J = 5.2 Hz), 7.51 (1H, d, J = 1.6 Hz), 7.15 (2H, d, J = 8.4 Hz), 7.14 (2H, d, J = 8.4 Hz), 7.10 (1H, t, J = 5.2 Hz).

### (8b) 3-(4-Fluorophenoxy)-5-(pyrimidin-2-ylsulfanyl)pyridine-2-carboxamide

The compound (0.77 g, 2.37 mmol) obtained in Example (8a) was dissolved in sulfuric acid (4 mL) under a nitrogen stream, followed by stirring at room temperature overnight. The reaction solution was poured into water, followed by neutralization with an aqueous sodium hydroxide solution to pH 6. Subsequently, extraction was carried out with ethyl acetate and the extract was washed with water and brine sequentially. The resulting organic layer was dried over anhydrous sodium sulfate and subsequently concentrated under reduced pressure to give a residue. The resulting residue was purified using silica gel column chromatography (ethyl acetate) to afford the desired title compound (726 mg, 78 %) as a colorless solid.
¹H-NMR (CDCl₃, 400 MHz) δ: 8.51 (1H, d, J = 1.6 Hz), 8.48 (2H, d, J = 5.2 Hz), 7.64 (1H, brs), 7.56 (1H, d, J = 1.6 Hz), 7.08 (2H, d, J = 8.4 Hz), 7.07 (2H, d, J = 8.4 Hz), 7.05 (1H, t, J = 5.2 Hz), 5.78 (1H, brs).

### (8c) 2-Amino-[3-(4-fluorophenoxy)-5-(pyrimidin-2-ylsulfanyl)pyridine

To a 2N-aqueous sodium hydroxide solution (4.26 mL), bromine (0.12 mL, 2.35 mmol) was added at 0°C, followed by stirring for 15 minutes. To a 1,4-dioxane solution (14 mL) of the compound (0.73 g, 2.13 mmol) obtained in Example (8b), the adjusted reagent was added dropwise at room temperature, followed by stirring for 1 hour. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 1:5) to afford the desired title compound (0.44 g, 65 %) as a colorless solid. ¹H-NMR (CDCl₃, 400 MHz) δ: 8.46 (2H, d, J = 5.2 Hz), 8.02 (1H, d, J = 1.6 Hz), 7.13 (1H, d, J = 1.6 Hz), 7.06 (2H, d, J = 8.4 Hz), 7.05 (2H, d, J = 8.4 Hz), 6.97 (1H, t, J = 5.2 Hz), 5.05 (2H, brs).

### (8d) 6-[3-(4-Fluorophenoxy)-5-(pyrimidin-2-ylsulfanyl)pyridin-2-ylamino]nicotinonitrile

The compound (435 mg, 1.38 mmol) obtained in Example (8c), 6-chloronicotinonitrile (211 mg, 1.52 mmol), cesium carbonate (902 mg, 2.77 mmol), tris(dibenzylideneacetone)dipalladium complex (63 mg, 0.07 mmol), and 4,5-bis(diphenylphosphino)-9,9-dimethylxanthene (120 mg, 0.21 mmol) were dissolved in 1,4-dioxane (10 mL) under a nitrogen stream at room temperature, followed by stirring for 10 minutes. Subsequently, water (50 µL, 2.77 mmol) was added, followed by heating and stirring at 100°C for 3 hours. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (dichloromethane:ethyl acetate = 20:1) to afford the desired title compound (407 mg, 71 %) as a colorless solid.
¹H-NMR (CDCl₃, 400 MHz) δ: 8.81 (1H, dd, J = 8.8, 0.8 Hz), 8.55 (1H, dd, J = 2.4, 0.8 Hz), 8.47 (2H, d, J = 5.2 Hz), 8.37 (1H, brs), 8.23 (1H, d, J = 1.6 Hz), 7.93 (1H, dd, J = 8.8, 2.4 Hz), 7.24 (1H, d, J = 1.6 Hz), 7.12 (2H, d, J = 8.4 Hz), 7.10 (2H, dd, J = 8.4, 2.8 Hz), 7.01 (1H, t, J = 5.2 Hz).

### (8e) [3-(4-Fluorophenoxy)-5-(pyrimidin-2-ylsulfanyl)pyridin-2-yl]-[5-(1H-tetrazol-5-yl)pyridin-2-yl]amine

A toluene solution (1 mL) of the compound (186 mg, 0.45 mmol) obtained in Example (8d) and tributyltin azide (297 mg, 0.89 mmol) was heated to reflux at 110°C for 5 hours under a nitrogen stream. The reaction solution was concentrated and subsequently purified using silica gel column chromatography (ethyl acetate:methanol = 5:1) to afford the desired title compound (122 mg, 60 %) as a colorless solid.
¹H-NMR (DMSO-d₆, 400 MHz) δ: 9.27 (1H, s), 8.90 (1H, d, J = 2.0 Hz), 8.62 (2H, d, J = 4.8 Hz), 8.41 (1H, d, J = 9.2 Hz), 8.38 (1H, dd, J = 9.2, 2.0 Hz), 8.27 (1H, d, J = 2.0 Hz), 7.43 (1H, d, J = 2.0 Hz), 7.27 (1H, t, J = 4.8 Hz), 7.32-7.24 (4H, m); MS (FAB, m/z): 482 (M+Na)⁺.

### (Example 9)

### [3-(3,4-Difluorophenoxy)-5-(pyrimidin-2-ylsulfanyl)pyridin-2-yl]-[5-(1H-tetrazol-5-yl)pyridin-2-yl]amine

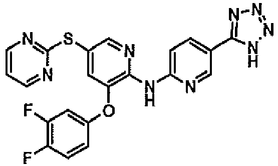

### (9a) 3-(3,4-Difluorophenoxy)-5-(pyrimidin-2-ylsulfanyl)pyridine-2-carbonitrile

Analogously to Example (8a), the desired title compound (1.31 g, 51 %) was obtained as a pale yellow oil from 5-bromo-3-nitropyridine-2-carbonitrile (1.71 g, 7.5 mmol).
¹H-NMR (CDCl₃, 400 MHz) δ: 8.58 (1H, d, J = 2.0 Hz), 8.52 (2H, d, J = 4.8 Hz), 7.62 (1H, d, J = 2.0 Hz), 7.26-7.24 (1H, m), 7.12 (1H, t, J = 4.8 Hz), 7.07-7.05 (1H, m), 6.97-6.95 (1H, m).

### (9b) 3-(3,4-Difluorophenoxy)-5-(pyrimidin-2-ylsulfanyl)pyridine-2-carboxamide

Analogously to Example (8b), the desired title compound (1.38 g, 86 %) was obtained as a colorless solid from the compound (1.31 g, 3.83 mmol) obtained in Example (9a).
¹H-NMR (CDCl₃, 400 MHz) δ: 8.55 (1H, d, J = 1.6 Hz), 8.51 (2H, d, J = 4.8 Hz), 7.68 (1H, d, J = 1.6 Hz), 7.64 (1H, brs), 7.17-7.15 (1H, m), 7.10 (1H, t, J = 4.8 Hz), 6.96-6.94 (1H, m), 6.84-6.82 (1H, m), 5.67 (1H, brs).

### (9c) 2-Amino-[3-(3,4-difluorophenoxy)-5-(pyrimidin-2-ylsulfanyl)pyridine

Analogously to Example (8c), the desired title compound (116 mg, 57 %) was obtained as a colorless solid from the compound (0.22 g, 0.61 mmol) obtained in Example (9b).
¹H-NMR (CDCl₃, 400 MHz) δ: 8.48 (2H, d, J = 5.2 Hz), 8.07 (1H, d, J = 1.6 Hz), 7.18 (1H, d, J = 1.6 Hz), 7.15 (1H, dd, J = 18.4, 9.2 Hz), 6.97-6.95 (1H, m), 6.85-6.83 (1H, m), 4.98 (2H, brs).

### (9d) 6-[3-(3,4-Difluorophenoxy)-5-(pyrimidin-2-ylsulfanyl)pyridin-2-ylamino]nicotinonitrile

Analogously to Example (8d), the desired title compound (118 mg, 79 %) was obtained as a colorless solid from the compound (115 mg, 0.35 mmol) obtained in Example (9c).
¹H-NMR (CDCl₃, 400 MHz) δ: 8.80 (1H, dd, J = 8.0, 0.8 Hz), 8.55 (1H, dd, J = 2.4, 0.8 Hz), 8.49 (2H, d, J = 5.2 Hz), 8.27 (1H, brs), 8.27 (1H, d, J = 2.0 Hz), 7.93 (1H, dd, J = 8.0, 2.4 Hz), 7.36 (1H, d, J = 1.6 Hz), 7.22 (1H, dd, J = 18.8, 8.8 Hz), 7.07-7.06 (1H, m), 7.02 (1H, t, J = 5.2 Hz), 6.93-6.92 (1H, m).

### (9e) [3-(3,4-Difluorophenoxy)-5-(pyrimidin-2-ylsulfanyl)pyridin-2-yl]-[5-(1H-tetrazol-5-yl)pyridin-2-yl]amine

Analogously to Example (8e), the desired title compound (113 mg, 87 %) was obtained as a colorless solid from the compound (118 mg, 0.27 mmol) obtained in Example (9d).
¹H-NMR (DMSO-d₆, 400 MHz) δ: 9.30 (1H, s), 8.88 (1H, d, J = 2.0 Hz), 8.63 (2H, d, J = 4.8 Hz), 8.36 (1H, d, J = 9.2 Hz), 8.35 (1H, d, J = 9.2 Hz), 8.29 (1H, d, J = 2.0 Hz), 7.60 (1H, d, J = 2.0 Hz), 7.52 (1H, dd, J = 18.8, 8.8 Hz), 7.41-7.40 (1H, m), 7.28 (1H, t, J = 5.2 Hz), 7.06-7.05 (1H, m);
MS (FAB, m/z): 478 (M+H)⁺.

### (Example 10)

### [3-(4-Fluorophenoxy)-5-(4H-[1,2,4]triazol-3-ylsulfanyl)pyridin-2-yl]-[5-(1H-tetrazol-5-yl)pyridin-2-yl]amine

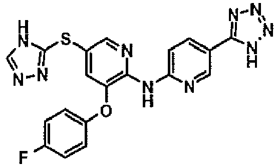

### (10a) 3-(4-Fluorophenoxy)-5-(4H-[1,2,4]triazol-3-ylsulfanyl)pyridine-2-carbonitrile

Analogously to Example (1a), the desired title compound (5.5 g, 77 %) was obtained as a yellow solid from 4-fluorophenol (2.5 g, 23 mmol), 5-bromo-3-nitropyridine-2-carbonitrile (5.5 g, 23 mmol), and 3-mercapto-[1,2,4]triazole (2.3 g, 23 mmol). ¹H-NMR (DMSO-d₆, 400 MHz) δ: 8.75 (1H, s), 8.35 (1H, d, J = 2.0 Hz), 7.36-7.28 (6H, m);
LCMS (ESI, m/z): 314 (M+H)⁺.

### (10b) 3-(4-Fluorophenoxy)-5-(4H-[1,2,4]triazol-3-ylsulfanyl)pyridine-2-carboxamide

Analogously to Example (5c), the desired title compound (5.1 g, 89 %) was obtained as a pale yellow solid from the compound (5.5 g, 18 mmol) obtained in Example (10a).
¹H-NMR (DMSO-d₆, 500 MHz) δ: 8.69 (1H, s), 8.33 (1H, s), 7.94 (1H, s), 7.58 (1H, s), 7.37 (1H, s), 7.25-7.20 (2H, m), 7.08-7.05 (2H, m);
LCMS (ESI, m/z): 332 (M+H)⁺.

### (10c) 2-Amino-3-(4-fluorophenoxy)-5-(4H-[1,2,4]triazol-3-ylsulfanyl)pyridine

Analogously to Example (5d), the desired title compound (2.2 g, 47 %) was obtained as a white solid from the compound (5.1 g, 16 mmol) obtained in Example (10b).
¹H-NMR (CDCl₃, 400 MHz) δ: 8.03 (1H, s), 8.02 (1H, d, J = 2.0 Hz), 7.11 (1H, d, J = 2.0 Hz), 7.07-6.97 (5H, m), 5.26 (2H, brs);
LCMS (ESI, m/z): 304 (M+H)⁺.

### (10d) 6-[3-(4-Fluorophenoxy)-5-(4H-[1,2,4]triazol-3-ylsulfanyl)pyridin-2-ylamino]nicotinonitrile

Analogously to Example (6d), the desired title compound (360 mg, 56 %) was obtained as a white solid from the compound (491 mg, 1.6 mmol) obtained in Example (10c).
¹H-NMR (DMSO-d₆, 500 MHz) δ: 9.53 (1H, s), 8.66 (1H, s), 8.62 (1H, s), 8.20 (1H, s), 8.14 (1H, d, J = 8.8 Hz), 8.09 (1H, d, J = 8.8 Hz), 7.33-7.17 (6H, m);
LCMS (APCI, m/z): 406 (M+H)⁺.

### (10e) [3-(4-Fluorophenoxy)-5-(4H-[1,2,4]triazol-3-ylsulfanyl)pyridin-2-yl]-[5-(1H-tetrazol-5-yl)pyridin-2-yl]amine

Analogously to Example (5f), the desired title compound (50 mg, 47 %) was obtained as a white solid from the compound (100 mg, 0.25 mmol) obtained in Example (10d).
¹H-NMR (DMSO-d₆, 400 MHz) δ: 9.20 (1H, s), 8.88 (1H, t, J = 1.6 Hz), 8.60 (1H, brs), 8.34 (1H, s), 8.33 (1H, s), 8.21 (1H, d, J = 2.0 Hz), 7.33-7.21 (6H, m);
MS (FAB, m/z): 449 (M+H)⁺.

### (Example 11)

### [3-(4-Fluorophenoxy)-5-(4-methyl-4H-[1,2,4]triazol-3-ylsulfanyl)pyridin-2-yl]-[5-(1H-tetrazol-5-yl)pyridin-2-yl]amine

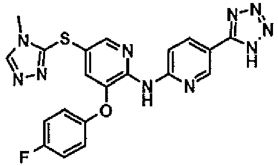

and

### (Example 12)

### [3-(4-Fluorophenoxy)-5-(1-methyl-1H-[1,2,4]triazol-3-ylsulfanyl)pyridin-2-yl]-[5-(1H-tetrazol-5-yl)pyridin-2-yl]amine

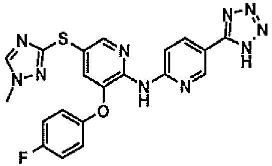

### (11a-12a)

To an N,N-dimethylformamide solution (5 mL) of the compound (500 mg, 1.6 mmol) obtained in Example (10c), potassium carbonate (456 mg, 3.3 mmol) and methyl iodide (0.1 mL, 1.6 mmol) were added sequentially at 0°C, followed by stirring at room temperature for 1 hour. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (ethyl acetate:methanol = 9:1) to afford a low polar amine compound (166 mg, 33%) as a pale yellow oil and a high polar amine compound (213 mg, 42%) as a pale yellow oil. Low polar amine compound: ¹H-NMR (CDCl₃, 400 MHz) δ: 8.02 (1H, s), 8.01 (1H, d, J = 2.0 Hz), 7.78 (1H, s), 7.09-7.00 (4H, m), 3.84 (3H, s);
MS (FAB, m/z): 318 (M+H)⁺.
High polar amine compound: ¹H-NMR (CDCl₃, 400 MHz) δ: 8.07 (1H, d, J = 2.0 Hz), 7.92 (1H, s), 7.19 (1H, d, J = 2.0 Hz), 7.07-6.99 (4H, m), 3.84 (3H, s):
MS (FAB, m/z): 318 (M+H)⁺.

### (12b-12b)

Analogously to Example (6d), a corresponding low polar nitrile compound (93 mg, 43 %) and a corresponding high polar nitrile compound (119 mg, 42 %) were obtained as an orange solid and a pale yellow solid respectively from the low polar amine compound (166 mg, 0.52 mmol) and the high polar amine compound (213 mg, 0.67 mmol) obtained in Example (11a-12a).
Low polar nitrile compound: ¹H-NMR (CDCl₃, 400 MHz) δ: 8.74 (1H, dd, J = 9.0, 0.8 Hz), 8.54 (1H, dd, J = 2.0, 0.8 Hz), 8.32 (1H, s), 8.18 (1H, d, J = 2.0 Hz), 7.92 (1H, dd, J = 9.0, 2.4 Hz), 7.80 (1H, s), 7.18 (1H, d, J = 2.0 Hz), 7.15-7.06 (4H, m), 3.87 (3H, s);
MS (FAB, m/z): 420 (M+H)⁺.
High polar nitrile compound: ¹H-NMR (CDCl₃, 400 MHz) δ: 8.75 (1H, dd, J = 9.0, 0.8 Hz), 8.53 (1H, dd, J = 2.0, 0.8 Hz), 8.27 (1H, s), 8.24 (1H, d, J = 2.0 Hz), 7.95 (1H, s), 7.91 (1H, dd, J = 9.0, 2.4 Hz), 7.26 (1H, d, J = 2.0 Hz), 7.11-7.07 (4H, m), 3.86 (3H, s);
MS (FAB, m/z): 420 (M+H)⁺.

### (11c-12c) [3-(4-Fluorophenoxy)-5-(4-methyl-4H-[1,2,4]triazol-3-ylsulfanyl)pyridin-2-yl]-[5-(1H-tetrazol-5-yl)pyridin-2-yl]amine and [3-(4-fluorophenoxy)-5-(1-methyl-1H-[1,2,4]triazol-3-ylsulfanyl)pyridin-2-yl]-[5-(1H-tetrazol-5-yl)pyridin-2-yl]amine

Analogously to Example (5f), the corresponding desired title compound A (72 mg, 71 %) as a white solid and the corresponding desired title compound B (79 mg, 61 %) were obtained respectively from the low polar nitrile compound (93 mg, 0.22 mmol) and the high polar nitrile compound (119 mg, 0.28 mmol) obtained in Example (11b-12b).
The desired title compound A: ¹H-NMR (DMSO-d₆, 400 MHz) δ: 9.25 (1H, s), 8.89 (1H, dd, J = 2.0, 1.2 Hz), 8.35 (1H, s), 8.34 (1H, s), 8.22 (1H, d, J = 2.0 Hz), 7.96 (1H, s), 7.34 (1H, d, J = 2.0 Hz), 7.31-7.20 (4H, m), 3.84 (3H, s);
MS (FAB, m/z): 463 (M+H)⁺.
The desired title compound B: ¹H-NMR (DMSO-d₆, 400 MHz) δ: 9.20 (1H, s), 8.88 (1H, dd, J = 2.0, 1.2 Hz), 8.49 (1H, s), 8.34 (1H, d, J = 2.4 Hz), 8.33 (1H, d, J = 1.2 Hz), 8.21 (1H, d, J = 2.0 Hz), 7.34 (1H, d, J = 2.4 Hz), 7.32-7.21 (4H, m), 3.81 (3H, s); MS (FAB, m/z): 463 (M+H)⁺.

### (Example 13)

### [3-(4-Fluorophenoxy)-5-(3-methylpyridin-2-ylsulfanyl)pyridin-2-yl]-[5-(1H-tetrazol-5-yl)pyridin-2-yl]amine

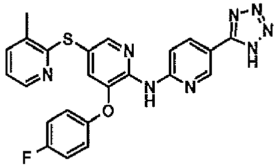

### (13a) 5-Bromo-3-(4-fluorophenoxy)pyridine-2-carbonitrile

To an N,N-dimethylformamide solution (50 mL) of 4-fluorophenol (2.4 g, 22 mmol), sodium hydride (content 60%)(954 mg, 24 mmol) was added under a nitrogen stream at 0°C, followed by stirring for 10 minutes. Subsequently, to the reaction solution, 5-bromo-3-nitropyridine-2-carbonitrile (5.2 g, 22 mmol) was added, followed by stirring for 30 minutes. The reaction solution was poured into water, extraction was carried out with diethyl ether, and the extract was washed with water and brine sequentially. The resulting organic layer was dried over anhydrous sodium sulfate and subsequently concentrated under reduced pressure to give a residue. The resulting residue was washed with diisopropyl ether to afford the desired title compound (5.7 g, 89 %) as a pale gray solid.
¹H-NMR (CDCl₃, 400 MHz) δ: 8.45 (1H, d, J = 2.0 Hz), 7.30 (1H, d, J = 2.0 Hz), 7.21-7.10 (4H, m).

### (13b) 5-Bromo-3-(4-fluorophenoxy)pyridine-2-carboxamide

The compound (2.0 g, 6.8 mmol) obtained in Example (13a) was dissolved in sulfuric acid (4.4 mL) under a nitrogen stream, followed by stirring at 50°C for 30 minutes. Under ice cooling, the reaction solution was poured into ice water, followed by neutralization with a 5N-aqueous sodium hydroxide solution to pH 8. Subsequently, extraction was carried out with methylene chloride and the extract was washed with water and brine sequentially. The resulting organic layer was dried over anhydrous sodium sulfate and subsequently concentrated under reduced pressure to give a residue. The residue was washed with water and diisopropyl ether sequentially to afford the desired title compound (2.1 g, 100 %) as a white solid.
¹H-NMR (CDCl₃, 400 MHz) δ: 8.40 (1H, d, J = 2.0 Hz), 7.53 (1H, brs), 7.37 (1H, d, J = 2.0 Hz), 7.14-7.04 (4H, m), 5.67 (1H, brs).

### (13c) 2-Amino-5-bromo-3-(4-fluorophenoxy)pyridine

To a 2N-aqueous sodium hydroxide solution (17 mL), bromine (0.70 mL, 14 mmol) was added at 0°C, followed by stirring for 20 minutes. To a 1,4-dioxane solution (20 mL) of the compound (2.1 g, 6.8 mmol) obtained in Example (13b), the adjusted reagent was added dropwise at room temperature, followed by stirring for 40 minutes. Under ice cooling, to the reaction solution, concentrated hydrochloric acid was added, followed by stirring at room temperature for 20 minutes. After neutralizing with a 5N-aqueous sodium hydroxide solution to pH 8, extraction was carried out with methylene chloride and the extract was washed with water and brine sequentially. The resulting organic layer was dried over anhydrous sodium sulfate and subsequently concentrated under reduced pressure to give a residue. The resulting residue was washed with an ethyl acetate-diisopropyl ether mixture to afford the desired title compound (1.0 g, 54 %) as a pale gray solid.
¹H-NMR (CDCl₃, 500 MHz) δ: 7.87 (1H, s), 7.11-7.08 (2H, m), 7.03-6.98 (3H, m), 4.77 (2H, brs).

### (13d) Methyl 3-[6-amino-5-(4-fluorophenoxy)pyridin-3-ylsulfanyl]propionate

The compound (1.0 g, 3.6 mmol) obtained in Example (13c), methyl 3-mercaptopropionate (437 mg, 3.6 mmol), diisopropylethylamine (1.6 mL, 9.1 mmol), tris(dibenzylideneacetone)dipalladium complex (333 mg, 0.36 mmol), and 4,5-bis(diphenylphosphino)-9,9-dimethylxanthene (421 mg, 0.73 mmol) were dissolved in 1,4-dioxane (11 mL) under a nitrogen stream at room temperature, followed by heating and stirring at 90°C for 1.5 hours. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 1:1) to afford the desired title compound (754 mg, 64 %) as a pale yellow solid. ¹H-NMR (CDCl₃, 400 MHz) δ: 7.93 (1H, s), 7.10-7.07 (2H, m), 7.02-6.99 (3H, m), 4.87 (2H, brs), 3.65 (3H, s), 2.92 (2H, t, J = 7.3 Hz), 2.53 (2H, t, J = 7.3 Hz).

### (13e) Methyl 3-[6-(5-cyanopyridin-2-ylamino)-5-(4-fluorophenoxy)pyridin-3-ylsulfanyl]propionate

The compound (754 mg, 2.3 mmol) obtained in Example (13d), 6-chloronicotinonitrile (486 mg, 3.5 mmol), cesium carbonate (1.5 g, 4.7 mmol), tris(dibenzylideneacetone)dipalladium complex (214 mg, 0.23 mmol), and 4,5-bis(diphenylphosphino)-9,9-dimethylxanthene (271 mg, 0.47 mmol) were dissolved in 1,4-dioxane (7 mL) under a nitrogen stream at room temperature, followed by stirring for 1 hour. Subsequently, water (84 µL, 4.6 mmol) was added, followed by heating and stirring at 100°C for 1 hour. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 2:1) to afford the desired title compound (520 mg, 53 %) as a yellow solid.
¹H-NMR (CDCl₃, 400 MHz) δ: 8.72 (1H, dd, J = 8.8, 0.8 Hz), 8.53 (1H, dd, J = 2.4, 0.8 Hz), 8.25 (1H, s), 8.10 (1H, d, J = 2.0 Hz), 7.91 (1H, dd, J = 8.8, 2.4 Hz), 7.16-7.06 (5H, m), 3.66 (3H, s), 3.01 (2H, t, J = 7.2 Hz), 2.56 (2H, t, J = 7.2 Hz).

### (13f) 6-[3-(4-Fluorophenoxy)-5-(3-methylpyridin-2-ylsulfanyl)pyridin-2-ylamino]nicotinonitrile

The compound (137 mg, 0.32 mmol) obtained in Example (13e) and 2-bromo-3-picoline (54 µL, 0.48 mmol) were dissolved in N,N-dimethylformamide (1 mL) under a nitrogen stream, and potassium tert-butoxide (72 mg, 0.65 mmol) was added under ice cooling, followed by stirring at 90°C for 4 hours. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 2:1) to afford the desired title compound (34 mg, 25 %) as a pale yellow solid.
¹H-NMR (CDCl₃, 400 MHz) δ: 8.79 (1H, dd, J = 8.8, 0.8 Hz), 8.54 (1H, dd, J = 2.4, 0.8 Hz), 8.31 (1H, s), 8.17 (1H, d, J = 2.0 Hz), 8.14-8.12 (1H, m), 7.91 (1H, dd, J = 8.8, 2.4 Hz), 7.37-7.35 (1H, m), 7.22 (1H, d, J = 2.0 Hz), 7.14-7.06 (4H, m), 6.96 (1H, dd, J = 7.4, 4.7 Hz), 2.33 (3H, s).

### (13g) [3-(4-Fluorophenoxy)-5-(3-methylpyridin-2-ylsulfanyl)pyridin-2-yl]-[5-(1H-tetrazol-5-yl)pyridin-2-yl]amine

Analogously to Example (5f), the desired title compound (6 mg, 16 %) was obtained as a pale yellow solid from the compound (34 mg, 79 µmol) obtained in Example (13f).
¹H-NMR (DMSO-d₆, 500 MHz) δ: 9.23 (1H, s), 8.90 (1H, s), 8.40 (1H, d, J = 8.9 Hz), 8.36 (1H, d, J = 8.9 Hz), 8.20-8.19 (2H, m), 7.58 (1H, d, J = 7.3 Hz), 7.31-7.24 (5H, m), 7.12-7.09 (1H, m), 2.31-2.29 (3H, m);
MS (FAB, m/z): 473 (M+H)⁺.

### (Example 14)

### [3-(4-Fluorophenoxy)-5-(3-trifluoromethylpyridin-2-ylsulfanyl)pyridin-2-yl]-[5-(1H-tetrazol-5-yl)pyridin-2-yl]amine

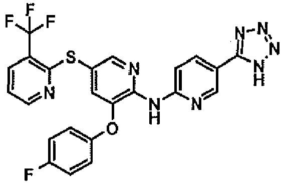

### (14a) 6-[3-(4-Fluorophenoxy)-5-(3-trifluoromethylpyridin-2-ylsulfanyl)pyridin-2-ylamino]nicotinonitrile

Analogously to Example (13f), the desired title compound (81 mg, 49 %) was obtained as a white solid from the compound (144 mg, 0.34 mmol) obtained in Example (13e) and 2-bromo-3-(trifluoromethyl)pyridine (100 mg, 0.44 mmol).
¹H-NMR (CDCl₃, 400 MHz) δ: 8.81 (1H, dd, J = 9.0, 0.8 Hz), 8.55 (1H, dd, J = 2.4, 0.8 Hz), 8.40 (1H, d, J = 4.0 Hz), 8.36 (1H, s), 8.19 (1H, d, J = 2.0 Hz), 7.93 (1H, dd, J = 9.0, 2.4 Hz), 7.86 (1H, d, J = 7.8 Hz), 7.20 (1H, d, J = 2.0 Hz), 7.15-7.07 (5H, m);
LCMS (ESI, m/z): 484 (M+H)⁺.

### (14b) [3-(4-Fluorophenoxy)-5-(3-trifluoromethylpyridin-2-ylsulfanyl)pyridin-2-yl]-[5-(1H-tetrazol-5-yl)pyridin-2-yl]amine

Analogously to Example (5f), the desired title compound (43 mg, 48 %) was obtained as a white solid from the compound (81 mg, 0.17 mmol) obtained in Example (14a).
¹H-NMR (CDCl₃, 400 MHz) δ: 8.91 (1H, s), 8.50 (1H, d, J = 8.8 Hz), 8.40 (1H, s), 8.32 (1H, dd, J = 8.8, 2.0 Hz), 8.11 (1H, s), 7.89 (1H, d, J = 7.8 Hz), 7.18-7.03 (6H, m);
MS (FAB, m/z): 527 (M+H)⁺.

### (Example 15)

Ethyl 6-{5-(3,4-difluorophenoxy)-6-[5-(1H-tetrazol-5-yl)pyridin-2-ylamino]pyridin-3-ylsulfanyl}nicotinate

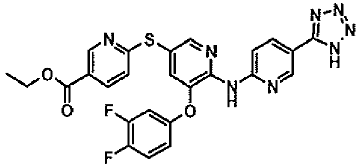

### (15a) 3-(3,4-Difluorophenoxy)-5-bromopyridine-2-carboxamide

To an N,N-dimethylformamide solution (40 mL) of 3,4-difluorophenol (5.2 g, 40 mmol), sodium hydride (content 63%) (1.6 g, 42 mmol) was added under a nitrogen stream at 0°C, followed by raising the temperature to room temperature and stirring for 10 minutes. After cooling to 0°C again, to the reaction solution, 5-bromo-3-nitropyridine-2-carbonitrile (9.1 g, 40 mmol) was added, followed by stirring for 10 minutes. Subsequently, the temperature was raised to room temperature, followed by stirring for 2 hours. To the reaction solution, water was added, followed by concentration. The resulting residue was dissolved in ethyl acetate, followed by washing with water and brine sequentially. The resulting organic layer was dried over anhydrous sodium sulfate and subsequently concentrated under reduced pressure to afford a solid. The resulting solid was dissolved in sulfuric acid (25 mL), followed by stirring at 50°C for 3 hours. The reaction solution was ice-cooled and neutralized with a 6N-aqueous sodium hydroxide solution (150 mL) and a saturated aqueous sodium hydrogencarbonate solution. To the resulting mixture, water (200 mL) was added and the deposited solid was filtered off, followed by washing with water. The resulting solid was dried to afford the desired title compound (11.6 g, 88 %).
¹H-NMR (DMSO-d₆, 400 MHz) δ: 8.56 (1H, d, J = 2.0 Hz), 7.95 (1H, brs), 7.86 (1H, d, J = 2.0 Hz), 7.63 (1H, brs), 7.50-7.43 (1H, m), 7.26 (1H, ddd, J = 11.7, 6.7, 3.1 Hz), 6.92-6.88 (1H, m).

### (15b) 2-Ethylhexyl 3-[6-amino-5-(3,4-difluorophenoxy)pyridin-3-ylsulfanyl]propionate

Analogously to Example (13c), 2-amino-3-(3,4-difluorophenoxy)-5-bromopyridine (7.1 g) was obtained from the compound (11.6 g, 35 mmol) obtained in Example (15a). Further, analogously to Example (13d), from the thus obtained compound (6.5 g) and 2-ethylhexyl 3-mercaptopropionate (4.9 mL, 21.5 mmol), the desired title compound (8.9 g, overall yield 64 %) was obtained.
¹H-NMR (CDCl₃, 500 MHz) δ: 7.99 (1H, s), 7.20-7.13 (2H, m), 6.90-6.86 (1H, m), 6.77-6.75 (1H, m), 4.82 (2H, brs), 4.02-3.96 (2H, m), 2.95 (2H, t, J = 7.3 Hz), 2.55 (2H, t, J = 7.3 Hz), 1.58-1.53 (1H, m), 1.37-1.27 (8H, m), 0.90-0.86 (6H, m).

### (15c) 2-Ethylhexyl 3-[6-(5-cyanopyridin-2-ylamino)-5-(3,4-difluorophenoxy)pyridin-3-ylsulfanyl]propionate

Analogously to Example (1d), the desired title compound (7.4 g, 93 %) was obtained from the compound (6.5 g, 15 mmol) obtained in Example (15b).
¹H-NMR (CDCl₃, 500 MHz) δ: 8.71 (1H, d, J = 9.3 Hz), 8.53 (1H, s), 8.16-8.14 (2H, m), 7.92-7.90 (1H, m), 7.24-7.21 (1H, m), 7.16 (1H, s), 6.98-6.94 (1H, m), 6.86-6.84 (1H, m), 4.02-3.95 (2H, m), 3.04 (2H, t, J = 7.3 Hz), 2.58 (2H, t, J = 7.3 Hz), 1.56-1.52 (1H, m), 1.36-1.25 (8H, m), 0.89-0.86 (6H, m).

### (15d) Ethyl 6-[5-(3,4-difluorophenoxy)-6-(5-cyanopyridin-2-ylamino)pyridin-3-ylsulfanyl]nicotinate

The compound (0.27 g, 0.5 mmol) obtained in Example (15c) and methyl 6-chloronicotinate (0.10 g, 0.6 mmol) were dissolved in ethanol (5 mL) under a nitrogen stream, and a 20% sodium ethoxide ethanol solution (0.43 mL) was added at 0°C, followed by stirring at room temperature for 15 hours. After the reaction, a 5% acetic acid aqueous solution was added, the deposited solid was filtered off, and washing was carried out with water to afford the desired title compound (0.12 g, 46 %). LCMS (ESI, m/z): 506 (M+H)⁺, retention time: 3.1 min.

### (15e) Ethyl 6-{5-(3,4-difluorophenoxy)-6-[5-(1H-tetrazol-5-yl)pyridin-2-ylamino]pyridin-3-ylsulfanyl}nicotinate

Analogously to Example (1e), the desired title compound (98 mg, 82 %) was obtained from the compound (0.11 g, 0.22 mmol) obtained in Example (15d).
LCMS (ESI, m/z): 549 (M+H)⁺, retention time: 2.2 min.

### (Example 16)

### 6-{5-(3,4-Difluorophenoxy)-6-[5-(1H-tetrazol-5-yl)pyridin-2-ylamino]pyridin-3-ylsulfanyl}pyridin-3-ylmethanol

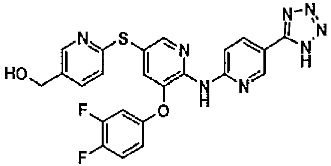

To a tetrahydrofuran solution (2 mL) of the compound (92 mg, 0.167 mmol) obtained in Example (15e), a 1M-diisobutylaluminium hydride-toluene solution (1.0 mL, 1.0 mmol) was added dropwise under a nitrogen stream at 0°C, followed by stirring for 30 minutes. The reaction solution was poured into a saturated aqueous ammonium chloride solution, extraction was carried out with dichloromethane, and the extract was washed with water and brine sequentially. The resulting organic layer was dried over anhydrous sodium sulfate and subsequently concentrated under reduced pressure to give a residue. The resulting residue was purified using silica gel column chromatography to afford the desired title compound (15 mg, 18 %).
¹H-NMR (DMSO-d₆, 400 MHz) δ: 9.19 (1H, brs), 8.88 (1H, s), 8.38 (1H, d, J = 8.8 Hz), 8.34-8.33 (2H, m), 8.28-8.27 (1H, m), 7.62-7.61 (1H, m), 7.53-7.47 (2H, m), 7.45-7.41 (1H, m), 7.10 (1H, d, J = 10.2 Hz), 7.07-7.06 (1H, m);
LCMS (ESI, m/z): 507 (M+H)⁺.

### (Example 17)

### [3-(4-Fluorophenoxy)-5-[(3-methoxypropylsulfanyl)pyridin-2-yl]-[5-(1H-tetrazol-5-yl)pyridin-2-yl]amine

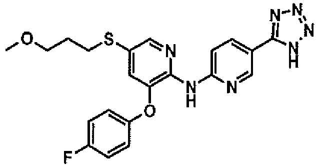

### (17a) 6-[3-(4-Fluorophenoxy)-5-(3-methoxypropylsulfanyl)pyridin-2-ylamino]nicotinonitrile

The compound (900 mg, 2.1 mmol) obtained in Example (13e) and 1-bromo-3-methoxypropane (422 mg, 2.8 mmol) were dissolved in tetrahydrofuran (10 mL) under a nitrogen stream, and potassium tert-butoxide (523 mg, 4.7 mmol) was added under ice cooling, followed by stirring at room temperature for 2 hours. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 2:1) to afford the desired title compound (600 mg, 70 %) as a white solid.
¹H-NMR (CDCl₃, 400 MHz) δ: 8.71 (1H, dd, J = 9.0, 0.8 Hz), 8.52 (1H, dd, J = 2.4, 0.8 Hz), 8.21 (1H, s), 8.07 (1H, d, J = 2.0 Hz), 7.90 (1H, dd, J = 9.0, 2.4 Hz), 7.15-7.05 (5H, m), 3.43 (2H, t, J = 6.1 Hz), 3.30 (3H, s), 2.86 (2H, t, J = 7.4 Hz), 1.85-1.78 (2H, m);
LCMS (ESI, m/z): 411 (M+H)⁺.

### (17b) [3-(4-Fluorophenoxy)-5-[(3-methoxypropylsulfanyl)pyridin-2-yl]-[5-(1H-tetrazol-5-yl)pyridin-2-yl]amine

Analogously to Example (5f), the desired title compound (578 mg, 85 %) was obtained as a yellow solid from the compound (600 mg, 1.5 mmol) obtained in Example (17a).
¹H-NMR (CDCl₃, 400 MHz) δ: 8.98 (1H, s), 8.44 (1H, dd, J = 9.0, 2.4 Hz), 8.38 (1H, d, J = 9.0 Hz), 7.93 (1H, d, J = 1.6 Hz), 7.05-6.96 (5H, m), 3.40 (2H, t, J = 6.1 Hz), 3.26 (3H, s), 2.81 (2H, t, J = 7.2 Hz), 1.81-1.75 (2H, m);
MS (FAB, m/z): 454 (M+H)⁺.

### (Example 18)

### [3-(4-Fluorophenoxy)-5-(2-methoxyethylsulfanyl)pyridin-2-yl]-[5-(1H-tetrazol-5-yl)pyridin-2-yl]amine

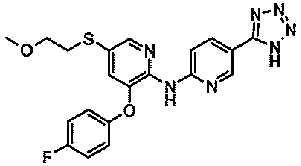

### (18a) 6-[3-(4-Fluorophenoxy)-5-(2-methoxyethylsulfanyl)pyridin-2-ylamino]nicotinonitrile

Analogously to Example (17a), the desired title compound (72 mg, 67 %) was obtained as a pale yellow solid from the compound (116 mg, 0.27 mmol) obtained in Example (13e) and 2-bromoethylmethyl ether (39 µL, 0.41 mmol).
¹H-NMR (CDCl₃, 400 MHz) δ: 8.71 (1H, dd, J = 9.0, 0.8 Hz), 8.52 (1H, dd, J = 2.4, 0.8 Hz), 8.24 (1H, s), 8.11 (1H, d, J = 2.0 Hz), 7.91 (1H, dd, J = 9.0, 2.4 Hz), 7.15-7.05 (5H, m), 3.51 (2H, t, J = 6.4 Hz), 3.31 (3H, s), 2.95 (2H, t, J = 6.4 Hz).

### (18b) [3-(4-Fluorophenoxy)-5-(2-methoxyethylsulfanyl)pyridin-2-yl]-[5-(1H-tetrazol-5-yl)pyridin-2-yl]amine

Analogously to Example (5f), the desired title compound (30 mg, 38 %) was obtained as a yellow solid from the compound (72 mg, 0.18 mmol) obtained in Example (18a).
¹H-NMR (CDCl₃, 400 MHz) δ: 8.96 (1H, s), 8.40 (1H, brs), 7.97 (1H, s), 7.06-6.98 (5H, m), 3.47 (2H, t, J = 6.3 Hz), 3.26 (3H, s), 2.90 (2H, t, J = 6.3 Hz);
MS (FAB, m/z): 440 (M+H)⁺.

### (Example 19)

### 3-(4-Fluorophenoxy)-5-[(4-methoxybutylsulfanyl)pyridin-2-yl]-[5-(1H-tetrazol-5-yl)pyridin-2-yl]amine

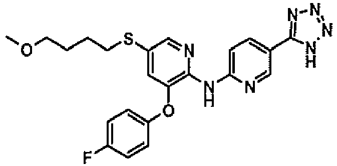

### (19a) 6-[3-(4-Fluorophenoxy)-5-(4-methoxybutylsulfanyl)pyridin-2-ylamino]nicotinonitrile

Analogously to Example (17a), the desired title compound (283 mg, 90 %) was obtained as a pale yellow solid from the compound (316 mg, 0.74 mmol) obtained in Example (13e) and 4-bromobutylmethyl ether (170 mg, 0.97 mmol).
¹H-NMR (CDCl₃, 400 MHz) δ: 8.71 (1H, dd, J = 8.8, 0.8 Hz), 8.52 (1H, dd, J = 2.4, 0.8 Hz), 8.21 (1H, s), 8.07 (1H, d, J = 2.0 Hz), 7.90 (1H, dd, J = 8.8, 2.4 Hz), 7.15-7.04 (5H, m), 3.36-3.34 (2H, m), 3.30 (3H, s), 2.82-2.78 (2H, m), 1.66-1.61 (4H, m) ;
MS (FAB, m/z): 425 (M+H)⁺.

### (19b) 3-(4-Fluorophenoxy)-5-[(4-methoxybutylsulfanyl)pyridin-2-yl]-[5-(1H-tetrazol-5-yl)pyridin-2-yl]amine

Analogously to Example (5f), the desired title compound (234 mg, 75 %) was obtained as a yellow solid from the compound (283 mg, 0.67 mmol) obtained in Example (19a).
¹H-NMR (CDCl₃, 400 MHz) δ: 9.01 (1H, s), 8.51 (1H, d, J = 9.0 Hz), 8.45 (1H, dd, J = 9.0, 2.4 Hz), 7.99 (1H, d, J = 2.0 Hz), 7.08-6.98 (5H, m), 3.34 (2H, t, J = 5.9 Hz), 3.29 (3H, s), 2.76 (2H, t, J = 6.8 Hz), 1.64-1.59 (4H, m);
MS (FAB, m/z): 468 (M+H)⁺.

### (Example 20)

### 3-{5-(4-Fluorophenoxy)-6-[5-(1H-tetrazol-5-yl)pyridin-2-ylamino]pyridin-3-ylsulfanyl}propan-1-ol

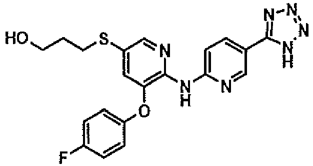

### (20a) 6-{5-[3-(tert-Butyldimethylsiloxy)propylsulfanyl]-3-(4-fluorophenoxy)pyridin-2-ylamino}nicotinonitrile

Analogously to Example (17a), the desired title compound (660 mg, 78 %) was obtained as a colorless oil from the compound (700 mg, 1.6 mmol) obtained in Example (13e) and (3-bromopropoxy)-tert-butyldimethylsilane (0.5 mL, 2.1 mmol). ¹H-NMR (CDCl₃, 400 MHz) δ: 8.71 (1H, dd, J = 9.0, 0.8 Hz), 8.52 (1H, dd, J = 2.4, 0.8 Hz), 8.21 (1H, s), 8.07 (1H, d, J = 2.0 Hz), 7.90 (1H, dd, J = 9.0, 2.4 Hz), 7.15-7.03 (5H, m), 3.67 (2H, t, J = 6.1 Hz), 2.87 (2H, t, J = 7.2 Hz), 1.79-1.72 (2H, m), 0.87 (9H, s), 0.03 (6H, s);
MS (FAB, m/z): 511 (M+H)⁺.

### (20b) 6-[3-(4-Fluorophenoxy)-5-(3-hydroxypropylsulfanyl)pyridin-2-ylamino]nicotinonitrile

The compound (660 mg, 1.3 mmol) obtained in Example (20a) was dissolved in tetrahydrofuran (5 mL) under a nitrogen stream, and a 1M tetrahydrofuran solution of tetrabutylammonium fluoride (3.0 mL, 3.0 mmol) was added under ice cooling, followed by stirring at room temperature for 2 hours. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was washed with an ethyl acetate-diisopropyl ether mixture to afford the desired title compound (447 mg, 87 %) as a white solid. ¹H-NMR (CDCl₃, 400 MHz) δ: 8.71 (1H, dd, J = 8.6, 0.8 Hz), 8.52 (1H, dd, J = 2.4, 0.8 Hz), 8.22 (1H, s), 8.08 (1H, d, J = 2.0 Hz), 7.90 (1H, dd, J = 8.6, 2.4 Hz), 7.15-7.05 (5H, m), 3.74 (2H, dt, J = 5.9, 5.5 Hz), 2.90 (2H, t, J = 7.2 Hz), 1.85-1.78 (2H, m), 1.39 (1H, t, J = 5.5 Hz);
MS (FAB, m/z): 397 (M+H)⁺.

### (20c) 3-{5-(4-Fluorophenoxy)-6-[5-(1H-tetrazol-5-yl)pyridin-2-ylamino]pyridin-3-ylsulfanyl}propan-1-ol

Analogously to Example (5f), the desired title compound (185 mg, 38 %) was obtained as a yellow solid from the compound (447 mg, 1.1 mmol) obtained in Example (20b).
¹H-NMR (DMSO-d₆, 500 MHz) δ: 9.07 (1H, s), 8.85 (1H, s), 8.31 (1H, d, J = 8.8 Hz), 8.25 (1H, d, J = 8.8 Hz), 8.11 (1H, s), 7.30-7.19 (5H, m), 4.53 (1H, brs), 3.45 (2H, t, J = 5.9 Hz), 2.92 (2H, t, J = 7.3 Hz), 1.68-1.62 (2H, m);
MS (FAB, m/z): 440 (M+H)⁺.

### (Example 21)

### [5-(Cyclopropylmethylsulfanyl)-3-(4-fluorophenoxy)pyridin-2-yl]-[5-(1H-tetrazol-5-yl)pyridin-2-yl]amine

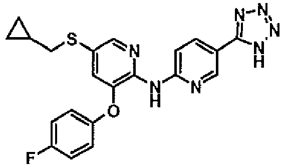

### (21a) 6-[5-(Cyclopropylmethylsulfanyl)-3-(4-fluorophenoxy)pyridin-2-ylamino]nicotinonitrile

Analogously to Example (17a), the desired title compound (200 mg, 68 %) was obtained as a white solid from the compound (320 mg, 0.75 mmol) obtained in Example (13e) and (bromomethyl)cyclopropane (95 µL, 0.98 mmol).
¹H-NMR (CDCl₃, 400 MHz) δ: 8.71 (1H, dd, J = 8.6, 0.8 Hz), 8.52 (1H, dd, J = 2.4, 0.8 Hz), 8.22 (1H, s), 8.12 (1H, d, J = 2.0 Hz), 7.90 (1H, dd, J = 8.6, 2.4 Hz), 7.15-7.04 (5H, m), 2.71 (2H, d, J = 7.0 Hz), 1.00-0.90 (1H, m), 0.56-0.51 (1H, m), 0.18-0.14 (2H, m).

### (21b) [5-(Cyclopropylmethylsulfanyl)-3-(4-fluorophenoxy)pyridin-2-yl]-[5-(1H-tetrazol-5-yl)pyridin-2-yl]amine

Analogously to Example (5f), the desired title compound (151 mg, 68 %) was obtained as a yellow solid from the compound (200 mg, 0.51 mmol) obtained in Example (21a).
¹H-NMR (CDCl₃, 400 MHz) δ: 8.97 (1H, s), 8.43-8.42 (2H, m), 7.97 (1H, s), 7.06-6.99 (5H, m), 2.66 (2H, d, J = 7.0 Hz), 0.94-0.86 (1H, m), 0.51-0.46 (2H, m), 0.15-0.11 (2H, m);
MS (FAB, m/z): 436 (M+H)⁺.

### (Example 22)

### 1-{5-(4-Fluorophenoxy-6-[5-(1H-tetrazol-5-yl)pyridin-2-ylamino]pyridin-3-ylsulfanyl}-3-methoxypropan-2-ol

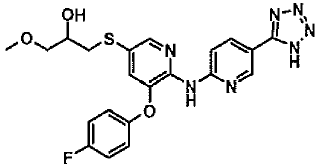

### (22a) 6-[3-(4-Fluorophenoxy)-5-(2-hydroxy-3-methoxypropylsulfanyl)pyridin-2-ylamino]nicotinonitrile

Analogously to Example (17a), the desired title compound (176 mg, 70 %) was obtained as a white solid from the compound (252 mg, 0.59 mmol) obtained in Example (13e) and glycidylmethyl ether (80 µL, 0.89 mmol).
¹H-NMR (CDCl₃, 400 MHz) δ: 8.71 (1H, dd, J = 8.7, 0.8 Hz), 8.53 (1H, dd, J = 2.4, 0.8 Hz), 8.23 (1H, s), 8.11 (1H, d, J = 2.0 Hz), 7.91 (1H, dd, J = 8.7, 2.4 Hz), 7.15-7.05 (5H, m), 3.83-3.78 (1H, m), 3.45 (1H, dd, J = 9.6, 3.9 Hz), 3.39 (1H, dd, J = 9.6, 5.9 Hz), 3.35 (3H, s), 2.95 (1H, dd, J = 13.7, 5.5 Hz), 2.87 (1H, dd, J = 13.7, 7.4 Hz), 2.52 (1H, d, J = 4.3 Hz);
MS (FAB, m/z): 427 (M+H)⁺.

### (22b) 1-{5-(4-Fluorophenoxy)-6-[5-(1H-tetrazol-5-yl)pyridin-2-ylamino]pyridin-3-ylsulfanyl}-3-methoxypropan-2-ol

Analogously to Example (5f), the desired title compound (150 mg, 78 %) was obtained as a yellow solid from the compound (176 mg, 0.41 mmol) obtained in Example (22a).
¹H-NMR (DMSO-d₆, 500 MHz) δ: 9.07 (1H, s), 8.85 (1H, s), 8.31 (1H, dd, J = 8.8, 2.0 Hz), 8.24 (1H, d, J = 8.8 Hz), 8.14 (1H, d, J = 2.0 Hz), 7.31-7.18 (5H, m), 5.13 (1H, brs), 3.69 (1H, brs), 3.31 (2H, d, J = 5.4 Hz), 3.20 (3H, s), 3.00 (1H, dd, J = 13.7, 4.6 Hz), 2.85 (1H, dd, J = 13.7, 6.8 Hz);
MS (FAB, m/z): 470 (M+H)⁺.

### (Example 23)

### [5-(2-Fluoro-3-methoxypropylsulfanyl)-3-(4-fluorophenoxy)pyridin-2-yl]-[5-(1H-tetrazol-5-yl)pyridin-2-yl]amine

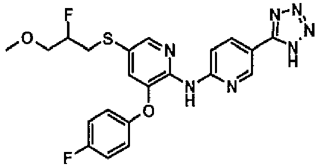

### (23a) 6-[5-(2-Fluoro-3-methoxypropylsulfanyl)-3-(4-fluorophenoxy)pyridin-2-ylamino]nicotinonitrile

The compound (170 mg, 0.40 mmol) obtained in Example (22a) was dissolved in methylene chloride (2 mL), and bis(2-methoxyethyl)aminosulfur-trifluoride (73 µL, 0.40 mmol) was added under ice cooling, followed by stirring at room temperature for 20 minutes. The reaction solution was poured into water, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 2:1) to afford the desired title compound (97 mg, 57 %) as a white solid.
¹H-NMR (CDCl₃, 400 MHz) δ: 8.72 (1H, dd, J = 9.0, 0.8 Hz), 8.53 (1H, dd, J = 2.4, 0.8 Hz), 8.24 (1H, s), 8.13 (1H, d, J = 2.0 Hz), 7.91 (1H, dd, J = 9.0, 2.4 Hz), 7.16-7.05 (5H, m), 4.73-4.56 (1H, m), 3.62 (1H, d, J = 3.9 Hz), 3.56 (1H, dd, J = 3.9, 2.2 Hz), 3.35 (3H, s), 3.08 (1H, dd, J = 6.3, 2.2 Hz), 3.03 (1H, d, J = 6.3 Hz);
MS (FAB, m/z): 429 (M+H)⁺.

### (23b) [5-(2-Fluoro-3-methoxypropylsulfanyl)-3-(4-fluorophenoxy)pyridin-2-yl]-[5-(1H-tetrazol-5-yl)pyridin-2-yl]amine

Analogously to Example (5f), the desired title compound (50 mg, 46 %) was obtained as a yellow solid from the compound (97 mg, 0.23 mmol) obtained in Example (23a).
¹H-NMR (CDCl₃, 400 MHz) δ: 8.98 (1H, s), 8.48-8.42 (2H, m), 8.03 (1H, s), 7.09-7.00 (5H, m), 4.70-4.56 (1H, m), 3.57 (2H, d, J = 20.0 Hz), 3.33 (3H, s), 3.02 (2H, dd, J = 20.0, 5.9 Hz);
MS (FAB, m/z): 472 (M+H)⁺.

### (Example 24)

### 2-{5-(4-Fluorophenoxy)-6-[5-(1H-tetrazol-5-yl)pyridin-2-ylamino]pyridin-3-ylsulfanylmethyl}cyclopropylmethanol

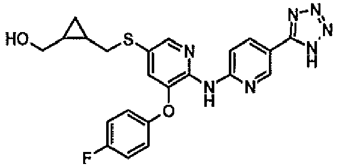

### (24a) 6-{5-[2-(tert-Butyldimethylsiloxymethyl)cyclopropylmethylsulfanyl]-3-(4-fluorophenoxy)pyridin-2-ylamino}nicotinonitrile

Analogously to Example (17a), the desired title compound (325 mg, 50 %) was obtained as a colorless oil from the compound (503 mg, 1.2 mmol) obtained in Example (13e) and [2-(bromomethyl)cyclopropyl]methoxy-tert-butyldimethylsilane (430 mg, 1.5 mmol).
¹H-NMR (CDCl₃, 400 MHz) δ: 8.72 (1H, dd, J = 9.0, 0.8 Hz), 8.52 (1H, dd, J = 2.4, 0.8 Hz), 8.21 (1H, s), 8.12 (1H, d, J = 2.0 Hz), 7.90 (1H, dd, J = 9.0, 2.4 Hz), 7.15-7.04 (5H, m), 3.46 (2H, d, J = 5.9 Hz), 2.78 (1H, dd, J = 13.0, 6.8 Hz), 2.70 (1H, dd, J = 13.0, 6.8 Hz), 0.90-0.84 (2H, m), 0.87 (9H, s), 0.53-0.49 (1H, m), 0.40-0.35 (1H, m), 0.02 (6H, s);
MS (FAB, m/z): 537 (M+H)⁺.

### (24b) 6-{3-(4-Fluorophenoxy)-5-[2-(hydroxymethyl)cyclopropylmethylsulfanyl]pyridin-2-ylamino}nicotinonitrile

Analogously to Example (20b), the desired title compound (242 mg, 94 %) was obtained as a white solid from the compound (325 mg, 0.61 mmol) obtained in Example (24a).
¹H-NMR (CDCl₃, 400 MHz) δ: 8.72 (1H, dd, J = 9.0, 0.8 Hz), 8.53 (1H, dd, J = 2.4, 0.8 Hz), 8.24 (1H, s), 8.11 (1H, d, J = 2.0 Hz), 7.91 (1H, dd, J = 9.0, 2.4 Hz), 7.16-7.05 (5H, m), 3.51-3.45 (1H, m), 3.38-3.32 (1H, m), 2.79 (1H, dd, J = 13.1, 6.8 Hz), 2.70 (1H, dd, J = 13.1, 6.8 Hz), 1.27 (1H, brs), 1.01-0.86 (2H, m), 0.55-0.45 (2H, m);
MS (FAB, m/z): 423 (M+H)⁺.

### (24c) 2-{5-(4-Fluorophenoxy)-6-[5-(1H-tetrazol-5-yl)pyridin-2-ylamino]pyridin-3-ylsulfanylmethyl}cyclopropylmethanol

Analogously to Example (5f), the desired title compound (145 mg, 55 %) was obtained as a pale yellow solid from the compound (242 mg, 0.57 mmol) obtained in Example (24b).
¹H-NMR (DMSO-d₆, 400 MHz) δ: 9.07 (1H, s), 8.85 (1H, dd, J = 2.4, 0.8 Hz), 8.32 (1H, dd, J = 9.0, 2.4 Hz), 8.26 (1H, dd, J = 9.0, 0.8 Hz), 8.15 (1H, d, J = 2.0 Hz), 7.30-7.18 (5H, m), 4.43 (1H, brs), 3.23-3.15 (2H, m), 2.91 (1H, dd, J = 13.1, 6.8 Hz), 2.79 (1H, dd, J = 13.1, 6.8 Hz), 0.85-0.77 (2H, m), 0.42-0.38 (1H, m), 0.35-0.31 (1H, m);
MS (FAB, m/z): 466 (M+H)⁺.

### (Example 25)

### 2,2-Difluoro-2-{5-(4-fluorophenoxy)-6-[5-(1H-tetrazol-5-yl)pyridin-2-ylamino]pyridin-3-ylsulfanyl}ethanol

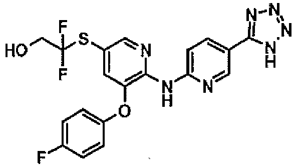

### (25a) Ethyl [6-(5-cyanopyridin-2-ylamino)-5-(4-fluorophenoxy)pyridin-3-ylsulfanyl]difluoroacetate

Analogously to Example (13f), the desired title compound (100 mg, 36 %) was obtained as a pale yellow solid from the compound (250 mg, 0.59 mmol) obtained in Example (13e) and ethyl bromodifluoroacetate (0.10 mL, 0.77 mmol).
¹H-NMR (CDCl₃, 400 MHz) δ: 8.77 (1H, d, J = 9.4 Hz), 8.55 (1H, d, J = 2.3 Hz), 8.36 (1H, s), 8.21 (1H, d, J = 2.3 Hz), 7.94 (1H, dd, J = 9.4, 2.3 Hz), 7.17-7.04 (5H, m), 2.80 (2H, q, J = 7.4 Hz), 1.24 (3H, t, J = 7.4 Hz);
LCMS (ESI, m/z): 460 (M⁺).

### (25b) 6-{5-[(1,1-Difluoro-2-hydroxyethyl)sulfanyl]-3-(4-fluorophenoxy)pyridin-2-ylamino}nicotinonitrile

The compound (100 mg, 0.21 mmol) obtained in Example (25a) was dissolved in an ethanol-tetrahydrofuran mixture (8 mL, 3:1 v/v) under a nitrogen stream, and sodium borohydride (8 mg, 0.21 mmol) was added under ice cooling, followed by stirring at room temperature for 1 hour. Water and acetone were poured into the reaction solution, extraction was carried out with a diethyl ether-ethyl acetate mixture, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 2:1) to afford the desired title compound (24 mg, 27 %) as a white solid.
¹H-NMR (CDCl₃, 400 MHz) δ: 8.78 (1H, d, J = 9.0 Hz), 8.56 (1H, d, J = 2.5 Hz), 8.36 (1H, s), 8.23 (1H, d, J = 2.5 Hz), 7.95 (1H, dd, J = 9.0, 2.5 Hz), 7.20-7.07 (5H, m), 3.91 (2H, td, J = 11.5, 7.1 Hz), 2.06 (1H, t, J = 7.1 Hz);
MS (FAB, m/z): 419 (M+H)⁺.

### (25c) 2,2-Difluoro-2-{5-(4-fluorophenoxy)-6-[5-(1H-tetrazol-5-yl)pyridin-2-ylamino]pyridin-3-ylsulfanyl}ethanol

Analogously to Example (5f), the desired title compound (8 mg, 30 %) was obtained as a white solid from the compound (24 mg, 57 µmol) obtained in Example (25b).
¹H-NMR (DMSO-d₆, 500 MHz) δ: 9.28 (1H, s), 8.91 (1H, s), 8.41 (1H, d, J = 9.0 Hz), 8.37 (1H, d, J = 9.0 Hz), 8.23 (1H, s), 7.33-7.25 (5H, m), 5.99 (1H, t, J = 6.1 Hz), 3.80-3.74 (2H, m);
MS (FAB, m/z): 462 (M+H)⁺.

### (Example 26)

### [3-(4-Fluorophenoxy)-5-(2-methoxy-1-methylethylsulfanyl)pyridin-2-yl]-[5-(1H-tetrazol-5-yl)pyridin-2-yl]amine

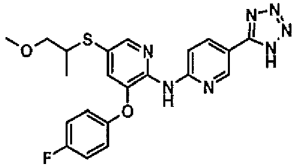

### (26a) 2-Ethylhexyl 3-[6-amino-5-(4-fluorophenoxy)pyridin-3-ylsulfanyl]propionate

Analogously to Example (13d), the desired title compound (4.07 g, 91 %) was obtained as a colorless oil from 2-amino-5-bromo-3-(4-fluorophenoxy)pyridine (3.00 g, 10.6 mmol).
¹H-NMR (CDCl₃, 400 MHz) δ: 7.95 (1H, d, J = 2.0 Hz), 7.11-6.99 (4H, m), 7.04 (1H, d, J = 2.0 Hz), 4.85 (2H, brs), 4.14-3.94 (2H, m), 2.92 (2H, t, J = 7.2 Hz), 2.53 (2H, t, J = 7.4 Hz), 1.62-1.25 (9H, m), 0.90-0.86 (6H, m).

### (26b) 2-Ethylhexyl 3-[6-(5-cyanopyridyl-2-ylamino)-5-(4-fluorophenoxy)pyridin-3-ylsulfanyl]propionate

Analogously to Example (1d), the desired title compound (0.47 g, 43 %) was obtained as a colorless oil from the compound (0.87 g, 2.07 mmol) obtained in Example (26a).
¹H-NMR (CDCl₃, 500 MHz) δ: 8.72 (1H, d, J = 8.8 Hz), 8.53 (1H, s), 8.25 (1H, s), 8.09 (1H, s), 7.91 (1H, d, J = 8.8 Hz), 7.15-7.12 (2H, m), 7.09-7.06 (3H, m), 4.01-3.95 (2H, m), 3.01 (2H, t, J = 7.3 Hz), 2.56 (2H, t, J = 7.4 Hz), 1.56-1.53 (1H, m), 1.36-1.25 (8H, m), 0.89-0.86 (6H, m).

### (26c) 6-[3-(4-Fluorophenoxy)-5-(2-methoxy-1-methylethylsulfanyl)pyridin-2-ylamino]nicotinonitrile

Analogously to Example (17a), the desired title compound (119 mg, 36 %) was obtained as a pale yellow solid from the compound (0.42 g, 0.80 mmol) obtained in Example (26b).
¹H-NMR (CDCl₃, 400 MHz) δ: 8.75 (1H, d, J = 9.0 Hz), 8.54 (1H, d, J = 1.5 Hz), 8.27 (1H, brs), 8.14 (1H, d, J = 2.0 Hz), 7.92 (1H, dd, J = 9.0, 2.3 Hz), 7.16-7.06 (5H, m), 3.34 (2H, dd, J = 6.5, 1.8 Hz), 3.30 (3H, s), 3.14 (1H, q, J = 6.7 Hz), 1.23 (3H, d, J = 6.6 Hz).

### (26d) [3-(4-Fluorophenoxy)-5-(2-methoxy-1-methylethylsulfanyl)pyridin-2-yl]-[5-(1H-tetrazol-5-yl)pyridin-2-yl]amine

Analogously to Example (1e), the desired title compound (98 mg, 75 %) was obtained as a pale yellow solid from the compound (118 mg, 0.29 mmol) obtained in Example (26c).
¹H-NMR (DMSO-d₆, 400 MHz) δ: 9.09 (1H, brs), 8.88 (1H, d, J = 1.6 Hz), 8.34 (2H, d, J = 1.5 Hz), 8.15 (1H, d, J = 2.0 Hz), 7.33-7.22 (5H, m), 3.35-3.28 (3H, m), 3.19 (3H, s), 1.15 (3H, d, J = 6.6 Hz);
MS (FAB, m/z): 454 (M+H)⁺.

### (Example 27)

### 3-{5-(4-Fluorophenoxy)-6-[5-(1H-tetrazol-5-yl)pyridin-2-ylamino]pyridin-3-ylsulfanyl]butan-1-ol

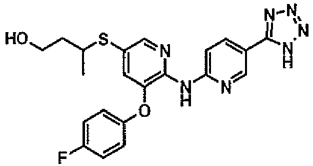

### (27a) 6-{5-[3-(tert-Butyldimethylsiloxy)-1-methylpropylsulfanyl]-3-(4-fluorophenoxy)pyridin-2-ylamino}nicotinonitrile

Analogously to Example (17a), the desired title compound (1.24 g, 96 %) was obtained as a colorless oil from the compound (1.33 g, 2.46 mmol) obtained in Example (26b).
¹H-NMR (CDCl₃, 400 MHz) δ: 8.75 (1H, d, J = 9.8 Hz), 8.54 (1H, d, J = 1.6 Hz), 8.25 (1H, brs), 8.11 (1H, d, J = 2.0 Hz), 7.92 (1H, dd, J = 9.0, 2.4 Hz), 7.15-7.11 (2H, m), 7.08-7.05 (2H, m), 7.07 (1H, d, J = 2.0 Hz), 3.75-3.72 (1H, m), 3.70-3.67 (1H, m), 3.21-3.15 (1H, m), 1.73-1.65 (2H, m), 1.22 (3H, d, J = 6.7 Hz), 0.86 (9H, s), 0.02 (3H, s), 0.02 (3H, s).

### (27b) 6-[3-(4-Fluorophenoxy)-5-(3-hydroxy-1-methylpropylsulfanyl)pyridin-2-ylamino]nicotinonitrile

A tetrahydrofuran solution (5 mL) of the compound (246 mg, 0.47 mmol) obtained in Example (27a) was added to a mixture of a 1M tetrahydrofuran solution of tetrabutylammonium fluoride (1.41 mL, 1.41 mmol) and acetic acid (113 mg, 1.88 mmol) under a nitrogen stream at room temperature, followed by stirring overnight. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was suspended in a mixed solvent of hexane and ethyl acetate and the solid was filtered off to afford the desired title compound (108 mg, 56 %) as a colorless solid.
¹H-NMR (DMSO-d₆, 500 MHz) δ: 9.45 (1H, s), 8.64 (1H, s), 8.12-8.06 (3H, m), 7.28-7.16 (5H, m), 4.50 (1H, t, J = 4.9 Hz), 3.50-3.45 (2H, m), 3.31-3.27 (1H, m), 1.66-1.55 (2H, m), 1.17 (3H, d, J = 6.8 Hz).

### (27c) 3-{5-(4-Fluorophenoxy)-6-[5-(1H-tetrazol-5-yl)pyridin-2-ylamino]pyridin-3-ylsulfanyl]butan-1-ol

Analogously to Example (1e), the desired title compound (78 mg, 66 %) was obtained as a pale yellow solid from the compound (107 mg, 0.26 mmol) obtained in Example (27b).
¹H-NMR (DMSO-d₆, 400 MHz) δ: 9.11 (1H, s), 8.88 (1H, s), 8.34 (2H, s), 8.14 (1H, s), 7.34-7.28 (2H, m), 7.24-7.21 (3H, m), 4.52 (1H, brs), 3.50-3.48 (2H, m), 3.25 (1H, d, J = 7.0 Hz), 1.67-1.52 (2H, m), 1.18 (3H, d, J = 6.6 Hz);
MS (FAB, m/z): 454 (M+H)⁺.

### (Example 28)

### [3-(3,4-Difluorophenoxy)-5-(3-methoxypropylsulfanyl)pyridin-2-yl]-[5-(1H-tetrazol-5-yl)pyridin-2-yl]amine

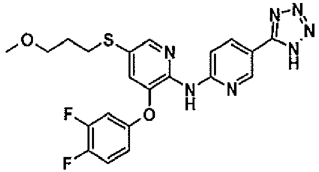

### (28a) 6-[3-(3,4-Difluorophenoxy)-5-(3-methoxypropylsulfanyl)pyridin-2-ylamino]nicotinonitrile

The compound (0.43 g, 0.8 mmol) obtained in Example (15c) and 1-bromo-3-methoxypropane (0.18 mL) were dissolved in tetrahydrofuran (8 mL) under a nitrogen stream, and potassium tert-butoxide (0.23 g, 2.0 mmol) was added, followed by stirring at room temperature for 1 hour. After the reaction, a 5% acetic acid aqueous solution was added, extraction was carried out with ethyl acetate, and the extract was washed with water and brine sequentially. The resulting organic layer was dried over anhydrous sodium sulfate and subsequently concentrated under reduced pressure to give a residue. The residue was purified using silica gel column chromatography (hexane-ethyl acetate) to afford the desired title compound (0.32 g, 94%).
¹H-NMR (CDCl₃, 500 MHz) δ: 8.69 (1H, dd, J = 9.0, 0.8 Hz), 8.51 (1H, dd, J = 2.4, 0.8 Hz), 8.12-8.11 (2H, m), 7.90 (1H, dd, J = 8.2, 1.6 Hz), 7.26-7.19 (1H, m), 7.12 (1H, d, J = 2.0 Hz), 6.94 (1H, ddd, J = 10.6, 6.3, 2.7 Hz), 6.85-6.81 (1H, m), 3.44 (2H, t, J = 6.3 Hz), 3.30 (3H, s), 2.89 (2H, t, J = 7.0 Hz), 1.86-1.80 (2H, m).

### (28b) [3-(3,4-Difluorophenoxy)-5-(3-methoxypropylsulfanyl)pyridin-2-yl]-[5-(1H-tetrazol-5-yl)pyridin-2-yl]amine

Analogously to Example (1e), the desired title compound (0.17 g, 50 %) was obtained from the compound (0.31 g, 0.72 mmol) obtained in Example (28a).
¹H-NMR (DMSO-d₆, 500 MHz) δ: 9.17 (1H, s), 8.84 (1H, s), 8.30
(1H, d, J = 9.3 Hz), 8.20 (1H, d, J = 8.8 Hz), 8.14 (1H, s), 7.52-7.46 (1H, m), 7.40 (1H, s), 7.36-7.32 (1H, m), 6.99-6.97 (1H, m), 3.37 (2H, t, J = 5.9 Hz), 3.19 (3H, s), 2.93 (2H, t, J = 7.8 Hz), 1.76-1.71 (2H, m);
LCMS (ESI, m/z): 472 (M+H)⁺. retention time: 2.0 min.

### (Example 29)

### 3-{5-(3,4-Difluorophenoxy)-6-[5-(1H-tetrazol-5-yl)pyridin-2-ylamino]pyridin-3-ylsulfanyl}propan-1-ol

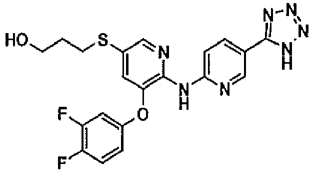

### (29a) 6-[3-(3,4-Difluorophenoxy)-5-(3-hydroxypropylsulfanyl)pyridin-2-ylamino]nicotinonitrile

The compound (0.43 g, 0.8 mmol) obtained in Example (15c) and (3-bromopropoxy)-tert-butyldimethylsilane (0.28 mL, 1.2 mmol) were dissolved in tetrahydrofuran (8 mL) under a nitrogen stream, and potassium tert-butoxide (0.23 g, 2.0 mmol) was added, followed by stirring at room temperature for 1 hour. After the reaction, a 5% acetic acid aqueous solution was added, extraction was carried out with ethyl acetate, and the extract was washed with water and brine sequentially. The resulting organic layer was dried over anhydrous sodium sulfate and subsequently concentrated under reduced pressure to give a residue. The residue was purified using silica gel column chromatography (hexane-ethyl acetate) to afford a nitrile compound. Further, the resulting nitrile compound was dissolved in tetrahydrofuran (5 mL), and a 1M tetrahydrofuran solution of tetrabutylammonium fluoride (2.4 mL, 2.4 mmol) was added, followed by stirring at room temperature for 2 hours. After the reaction, a 5% acetic acid aqueous solution was added, extraction was carried out with ethyl acetate, and the resulting organic layer was dried over anhydrous sodium sulfate and subsequently concentrated under reduced pressure to give a residue. The residue was purified using silica gel column chromatography (hexane-ethyl acetate) to afford the desired title compound (0.14 g, 42 %).
¹H-NMR (DMSO-d₆, 400 MHz) δ: 9.50 (1H, s), 8.60 (1H, dd, J = 2.4, 0.8 Hz), 8.14 (1H, d, J = 2.0 Hz), 8.08 (1H, dd, J = 8.6, 2.4 Hz), 7.93 (1H, dd, J = 20.0, 9.0 Hz), 7.40 (1H, d, J = 2.0 Hz), 7.29 (1H, ddd, J = 11.7, 6.7, 3.1 Hz), 6.95-6.91 (1H, m), 4.53 (1H, t, J = 5.1 Hz), 3.45 (2H, q, J = 5.9 Hz), 2.96 (2H, t, J = 7.4 Hz), 1.67-1.66 (2H, m).

### (29b) 3-{5-(3,4-Difluorophenoxy)-6-[5-(1H-tetrazol-5-yl)pyridin-2-ylamino]pyridin-3-ylsulfanyl}propan-1-ol

Analogously to Example (1e), the desired title compound (62 mg, 40 %) was obtained from the compound (0.14 g, 0.34 mmol) obtained in Example (29a).
¹H-NMR (DMSO-d₆, 500 MHz) δ: 9.16 (1H, s), 8.84 (1H, s), 8.31-8.29 (1H, m), 8.20 (1H, d, J = 8.8 Hz), 8.14 (1H, s), 7.52-7.46 (1H, m), 7.41-7.40 (1H, m), 7.36-7.32 (1H, m), 6.98-6.96 (1H, m), 4.53 (1H, brs), 3.46 (2H, s), 2.94 (2H, t, J = 7.8 Hz), 1.69-1.64 (2H, m).

### (Example 30)

### Ethyl 2-{5-(3,4-difluorophenoxy)-6-[5-(1H-tetrazol-5-yl)pyridin-2-ylamino]pyridin-3-ylsulfanyl}propionate

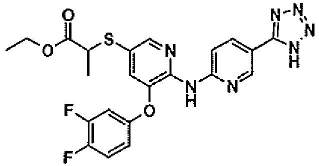

### (30a) Ethyl 2-[5-(3,4-difluorophenoxy)-6-(5-cyanopyridin-2-ylamino)pyridin-3-ylsulfanyl}propionate

The compound (0.54 g, 1.0 mmol) obtained in Example (15c) and ethyl 2-bromopropionate (0.20 mL, 1.5 mmol) were dissolved in ethanol (10 mL) under a nitrogen stream, and a 20% sodium ethoxide ethanol solution (0.86 mL) was added at 0°C, followed by stirring at room temperature for 1 hour. After the reaction, a 5% acetic acid aqueous solution was added, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane-ethyl acetate) to afford the desired title compound (0.40 g, 87 %).
¹H-NMR (CDCl₃, 500 MHz) δ: 8.73 (1H, d, J = 8.8 Hz), 8.54-8.53 (1H, m), 8.20 (1H, brs), 8.16 (1H, d, J = 2.0 Hz), 7.92 (1H, dd, J = 8.8, 2.0 Hz), 7.25-7.21 (1H, m), 7.17 (1H, d, J = 2.0 Hz), 7.00-6.96 (1H, m), 6.88-6.85 (1H, m), 4.13-4.05 (2H, m), 3.57 (1H, q, J = 7.3 Hz), 1.41 (3H, d, J = 6.8 Hz), 1.19 (3H, t, J = 7.3 Hz).

### (30b) Ethyl 2-{5-(3,4-difluorophenoxy)-6-[5-(1H-tetrazol-5-yl)pyridin-2-ylamino]pyridin-3-ylsulfanyl}propionate

Analogously to Example (1e), the desired title compound (0.31 g, 72 %) was obtained from the compound (0.40 g, 0.87 mmol) obtained in Example (30a).
¹H-NMR (DMSO-d₆, 400 MHz) δ: 9.31 (1H, s), 8.89-8.88 (1H, m), 8.36-8.30 (2H, m), 8.17 (1H, d, J = 1.6 Hz), 7.53 (1H, q, J = 9.4 Hz), 7.40-7.35 (2H, m), 7.03-7.01 (1H, m), 4.00 (2H, q, J = 7.0 Hz), 3.89 (1H, q, J = 7.0 Hz), 1.32 (3H, d, J = 7.0 Hz), 1.07 (3H, t, J = 7.0 Hz);
LCMS (ESI, m/z): 500 (M+H)⁺, retention time: 2.1 min.

### (Example 31)

### Ethyl 2-{5-(3,4-difluorophenoxy)-6-[5-(1H-tetrazol-5-yl)pyridin-2-ylamino]pyridin-3-ylsulfanyl}-2-methylpropionate

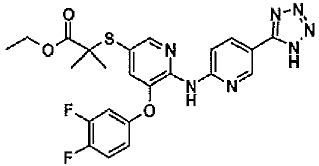

### (31a) Ethyl 2-{5-(3,4-difluorophenoxy)-6-[5-cyanopyridin-2-ylamino]pyridin-3-ylsulfanyl}-2-methylpropionate

Analogously to Example (30a), the desired title compound (0.15 g, 65 %) was obtained from the compound (0.27 g, 0.5 mmol) obtained in Example (15c) and ethyl 2-bromo-2-methylpropionate (0.18 mL, 1.3 mmol).
LCMS (ESI, m/z): 471 (M+H)⁺. retention time: 3.1 min.

### (31b) Ethyl 2-{5-(3,4-difluorophenoxy)-6-[5-(1H-tetrazol-5-yl)pyridin-2-ylamino]pyridin-3-ylsulfanyl}-2-methylpropionate

Analogously to Example (1e), the desired title compound (70 mg, 44 %) was obtained from the compound (0.15 g, 0.32 mmol) obtained in Example (31a).
¹H-NMR (DMSO-d₆, 400 MHz) δ: 9.34 (1H, s), 8.91-8.90 (1H, m), 8.39-8.34 (2H, m), 8.13 (1H, d, J = 2.0 Hz), 7.58-7.50 (1H, m), 7.39 (1H, ddd, J = 11.7, 7.0, 3.1 Hz), 7.22 (1H, d, J = 2.0 Hz), 7.06-7.01 (1H, m), 3.97 (2H, q, J = 7.0 Hz), 1.40 (6H, s), 1.08 (3H, t, J = 7.0 Hz);
LCMS (ESI, m/z): 514 (M+H)⁺, retention time: 2.2 min.

### (Example 32)

### 2-{5-(3,4-Difluorophenoxy)-6-[5-(1H-tetrazol-5-yl)pyridin-2-ylamino]pyridin-3-ylsulfanyl}propan-1-ol

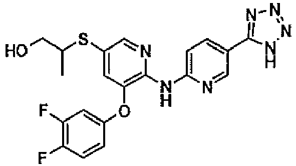

Analogously to Example 16, the desired title compound (0.23 g, 85 %) was obtained from the compound (0.30 g, 0.60 mmol) obtained in Example (30b).
¹H-NMR (DMSO-d₆, 500 MHz) δ: 9.21 (1H, s), 8.86 (1H, s), 8.34-8.31 (1H, m), 8.28-8.26 (1H, m), 8.19-8.18 (1H, m), 7.53-7.47 (1H, m), 7.45-7.44 (1H,m), 7.38-7.34 (1H, m), 7.00-6.98 (1H, m), 4.92 (1H, brs), 3.46-3.43 (1H, m), 3.34-3.31 (1H, m), 3.19-3.15 (1H, m), 1.17 (3H, d, J = 6.8 Hz);
LCMS (ESI, m/z): 458 (M+H) ⁺.

### (Example 33)

### [3-(3,4-Difluorophenoxy)-5-(1-methylethylsulfanyl)pyridin-2-yl]-[5-(1H-tetrazol-5-yl)pyridin-2-yl]amine

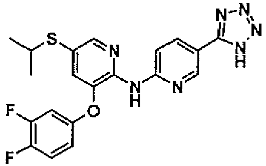

Analogously to Example (30a), 6-[3-(3,4-difluorophenoxy)-5-(1-methylethylsulfanyl)pyridin-2-ylamino]nicotinonitrile (0.30 g) was obtained as a mixture with impurities from the compound (0.43 g, 0.8 mmol) obtained in Example (15c) and 2-iodopropane (0.16 mL, 1.6 mmol). Further, analogously to Example (1e), this mixture was reacted with tributyltin azide (0.41 mL, 1.5 mmol) to afford the desired title compound (0.22 g, 67 %).
¹H-NMR (DMSO-d₆, 400 MHz) δ: 9.19 (1H, s), 8.85 (1H, dd, J = 2.4, 1.2 Hz), 8.34-8.31 (1H, m), 8.29-8.27 (1H, m), 8.16 (1H, d, J = 2.4 Hz), 7.54-7.46 (1H, m), 7.39-7.35 (2H, m), 7.01-6.97 (1H, m), 3.37-3.31 (1H, m), 1.19 (6H, d, J = 6.7 Hz);
LCMS (ESI, m/z): 442 (M+H)⁺, retention time: 2.1 min.

### (Example 34)

### {3-(3,4-Difluorophenoxy)-5-[3-(dimethylamino)propylsulfanyl]pyridin-2-yl}-[5-(1H-tetrazol-5-yl)pyridin-2-yl]amine

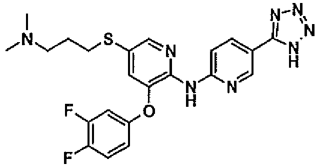

### (34a) 6-{3-(3,4-Difluorophenoxy)-5-[3-(dimethylamino)propylsulfanyl]pyridin-2-ylamino}nicotinonitrile

The compound (0.43 g, 0.8 mmol) obtained in Example (15c) and (3-chloropropyl)dimethylamine hydrochloride (0.19 g, 1.2 mmol) were dissolved in tetrahydrofuran (8 mL) under a nitrogen stream, and potassium tert-butoxide (0.23 g, 2.0 mmol) was added, followed by stirring at room temperature for 1 hour. After the reaction, acetic acid (1 mL) and Celite (2 g) were added, and the solvent was distilled off. The residue was purified using silica gel column chromatography (hexane-ethyl acetate) to afford the desired title compound (0.16 g, 46%). LCMS (ESI, m/z): 442 (M+H)⁺.

### (34b) {3-(3,4-Difluorophenoxy)-5-[3-(dimethylamino)propylsulfanyl]pyridin-2-yl}-[5-(1H-tetrazol-5-yl)pyridin-2-yl]amine

Analogously to Example (1e), the desired title compound (66 mg, 38 %) was obtained from the compound (0.16 g, 0.36 mmol) obtained in Example (34a).
¹H-NMR (DMSO-d₆, 400 MHz) δ: 8.79 (1H, t, J = 1.6 Hz), 8.63 (1H, s), 8.25-8.24 (2H, m), 8.16 (1H, d, J = 2.0 Hz), 7.54-7.47 (1H, m), 7.40-7.35 (2H, m), 7.03-6.99 (1H, m), 2.88 (2H, t, J = 7.0 Hz), 2.73 (2H, t, J = 6.7 Hz), 2.44 (6H, s), 1.77-1.70 (2H, m); LCMS (ESI, m/z): 485 (M+H)⁺.

### (Example 35)

### [3-(3,4-Difluorophenoxy)-5-(2-ethoxyethylsulfanyl)pyridin-2-yl]-[5-(1H-tetrazol-5-yl)pyridin-2-yl]amine

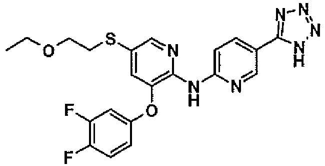

### (35a) 6-[3-(3,4-Difluorophenoxy)-5-(2-ethoxyethylsulfanyl)pyridin-2-ylamino]nicotinonitrile

Analogously to Example (17a), the desired title compound (0.27 mg, 79%) was obtained from the compound (0.43 g, 0.8 mmol) obtained in Example (15c) and 2-ethoxyethylbromide (0.13 mL, 1.2 mmol).
¹H-NMR (CDCl₃, 400 MHz) δ: 8.70 (1H, dd, J = 9.0, 0.8 Hz), 8.52 (1H, dd, J = 2.4, 0.8 Hz), 8.15 (1H, d, J = 2.0 Hz), 8.13 (1H, brs), 7.91 (1H, dd, J = 9.0, 2.9 Hz), 7.26-7.21 (1H, m), 7.19 (1H, d, J = 2.0 Hz), 6.94 (1H, ddd, J = 10.6, 6.3, 2.7 Hz), 6.85-6.82 (1H, m), 3.57 (2H, t, J = 6.7 Hz), 3.46 (2H, q, J = 7.0 Hz), 2.98 (2H, t, J = 6.7 Hz), 1.15 (3H, t, J = 7.0 Hz).

### (35b) [3-(3,4-Difluorophenoxy)-5-(2-ethoxyethylsulfanyl)pyridin-2-yl]-[5-(1H-tetrazol-5-yl)pyridin-2-yl]amine

Analogously to Example (1e), the desired title compound (0.13 g, 44 %) was obtained from the compound (0.26 g, 0.61 mmol) obtained in Example (35a).
¹H-NMR (DMSO-d₆, 400 MHz) δ: 9.18 (1H, brs), 8.84 (1H, dd, J = 2.4, 0.8 Hz), 8.31 (1H, dd, J = 9.0, 2.4 Hz), 8.21 (1H, dd, J = 9.0, 0.8 Hz), 8.16 (1H, d, J = 2.0 Hz), 7.53-7.46 (1H, m), 7.45 (1H, d, J = 2.0 Hz), 7.36 (1H, ddd, J = 11.7, 6.7, 3.1 Hz), 7.01-6.97 (1H, m), 3.51 (2H, t, J = 6.3 Hz), 3.38 (2H, q, J = 7.0 Hz), 3.06 (2H, t, J = 6.3 Hz), 1.04 (3H, t, J = 7.0 Hz); LCMS (ESI, m/z): 472 (M+H)⁺, retention time: 2.0 min.

### (Example 36)

### [3-(2,4-Difluorophenoxy)-5-(3-methoxypropoxy)pyridin-2-yl]-[5-(1H-tetrazol-5-yl)pyridin-2-yl]amine

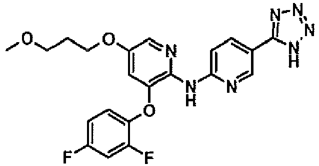

### (36a) 3-(2,4-Difluorophenoxy)-5-(3-methoxypropoxy)pyridine-2-carbonitrile

Analogously to Example (4a), the desired title compound (0.27 g, 17 %) was obtained as a pale yellow solid from 5-bromo-3-nitropyridine-2-carbonitrile (1.14 g, 5.0 mmol).
¹H-NMR (CDCl₃, 400 MHz) δ: 8.11 (1H, d, J = 2.3 Hz), 7.23 (1H, dt, J = 9.2, 4.4 Hz), 7.03-6.96 (2H, m), 6.53-6.52 (1H, m), 4.06 (2H, t, J = 6.2 Hz), 3.50 (2H, t, J = 5.9 Hz), 3.32 (3H, s), 2.03 (2H, t, J = 6.1 Hz).

### (36b) 3-(2,4-Difluorophenoxy)-5-(3-methoxypropoxy)pyridine-2-carboxamide

Analogously to Example (4b), the desired title compound (0.41 g, 68 %) was obtained as a colorless solid from the compound (0.58 g, 1.81 mmol) obtained in Example (36a).
¹H-NMR (CDCl₃, 500 MHz) δ: 8.05 (1H, d, J = 2.4 Hz), 7.54 (1H, brs), 7.14-7.09 (1H, m), 7.00-6.96 (1H, m), 6.91-6.86 (1H, m), 6.63 (1H, d, J = 2.4 Hz), 5.51 (1H, brs), 4.06 (2H, t, J = 6.4 Hz), 3.51 (2H, t, J = 6.1 Hz), 3.33 (3H, s), 2.03 (2H, quint, J = 6.4 Hz).

### (36c) 2-Amino-3-(2,4-difluorophenoxy)-5-(3-methoxypropoxy)pyridine

Analogously to Example (4c), the desired title compound (229 mg, 60 %) was obtained as a colorless oil from the compound (0.41 g, 1.22 mmol) obtained in Example (36b).
¹H-NMR (CDCl₃, 400 MHz) δ: 7.55 (1H, d, J = 2.3 Hz), 7.09 (1H, dt, J = 13.8, 4.5 Hz), 7.02-6.96 (1H, m), 6.92-6.87 (1H, m), 6.53 (1H, d, J = 2.3 Hz), 4.50 (2H, brs), 3.96 (2H, t, J = 6.2 Hz), 3.50 (2H, t, J = 6.0 Hz), 3.33 (3H, s), 2.01-1.94 (2H, quint, J = 6.3 Hz).

### (36d) 6-[5-(Pyridin-2-ylsulfanyl)-3-(4-trifluoromethylphenoxy)pyridin-2-ylamino]nicotinonitrile

Analogously to Example (4d), the desired title compound (177 mg, 58 %) was obtained as a pale yellow solid from the compound (228 mg, 0.73 mmol) obtained in Example (36c).
¹H-NMR (CDCl₃, 500 MHz) δ: 8.58 (1H, d, J = 8.8 Hz), 8.50 (1H, d, J = 2.4 Hz), 8.06 (1H, brs), 7.85 (1H, dd, J = 9.0, 2.2 Hz), 7.74 (1H, d, J= 2.4 Hz), 7.17 (1H, dt, J = 13.5, 4.4 Hz), 7.04-7.02 (1H, m), 7.00-6.93 (1H, m), 6.56 (1H, d, J = 2.5 Hz), 4.02 (2H, t, J = 6.1 Hz), 3.51 (2H, t, J = 6.1 Hz), 3.33 (3H, s), 2.00 (2H, quint, J = 5.9 Hz).

### (36e) [3-(2,4-Difluorophenoxy)-5-(3-methoxypropoxy)pyridin-2-yl]-[5-(1H-tetrazol-5-yl)pyridin-2-yl]amine

Analogously to Example (4e), the desired title compound (157 mg, 80 %) was obtained as a colorless solid from the compound (177 mg, 0.43 mmol) obtained in Example (36d).
¹H-NMR (DMSO-d₆, 400 MHz) δ: 9.01 (1H, brs), 8.79 (1H, d, J = 2.4 Hz), 8.23 (1H, dd, J = 9.0, 2.3 Hz), 7.90 (1H, d, J = 2.4 Hz), 7.88 (1H, d, J = 9.0 Hz), 7.54-7.48 (1H, m), 7.39-7.33 (1H, m), 7.18-7.13 (1H, m), 6.88 (1H, d, J = 2.4 Hz), 4.03 (2H, t, J = 6.5 Hz), 3.44 (2H, t, J = 6.4 Hz), 3.23 (3H, s), 1.90 (2H, quint, J = 6.3 Hz);
MS (FAB, m/z): 456 (M+H)⁺.

### (Example 37)

### 3-{5-(2,4-Difluorophenoxy)-6-[5-(1H-tetrazol-5-yl)pyridin-2-ylamino]pyridin-3-ylsulfanyl]butan-1-ol

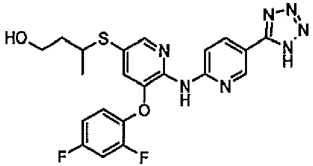

### (37a) 3-(2,4-Difluorophenoxy)-5-bromopyridine-2-carboxamide

Analogously to Example (15a), 3-(2,4-difluorophenoxy)-5-bromopyridine-2-carbonitrile (14.5 g, 93 %) was obtained as a pale yellow solid from 2,4-difluorophenol (6.5 g, 50 mmol) and 5-bromo-3-nitropyridine-2-carbonitrile (11.4 g, 50 mmol).
¹H-NMR (CDCl₃, 400 MHz) δ: 8.49 (1H, d, J = 1.6 Hz), 7.30-7.24 (1H, m), 7.25 (1H, d, J = 1.5 Hz), 7.10-7.04 (1H, m), 7.03-6.99 (1H, m).

Further, the resulting compound (14.5 g, 46.7 mmol) was hydrolyzed in sulfuric acid to afford the desired title compound (13.3 g, 87 %) as a colorless solid.
¹H-NMR (CDCl₃, 400 MHz) δ: 8.41 (1H, d, J = 1.6 Hz), 8.41 (1H, brs), 7.31 (1H, d, J = 1.6 Hz), 7.19-7.16 (1H, m), 7.04-6.92 (2H, m), 5.59 (1H, brs).

### (37b) 2-Ethylhexyl 3-[6-amino-5-(2,4-difluorophenoxy)pyridin-3-ylsulfanyl]propionate

Analogously to Example (13c), 2-amino-5-bromo-3-(2,4-difluorophenoxy)pyridine (2.9 g, 88 %) was obtained from the compound (3.62 g, 11 mmol) obtained in Example (37a).
¹H-NMR (CDCl₃, 500 MHz) δ: 7.86 (1H, d, J = 1.9 Hz), 7.14-7.09 (1H, m), 7.02-6.98 (1H, m), 6.94-6.90 (1H, m), 6.86 (1H, d, J = 1.9 Hz), 4.83 (2H, brs).
Further, analogously to Example (13d), the desired title compound (2.16 g, 100 %) was obtained from the resulting compound (1.4 g, 4.65 mmol) and 2-ethylhexyl 3-mercaptopropionate (1.12 g, 5.11 mmol).
¹H-NMR (CDCl₃, 400 MHz) δ: 7.94 (1H, d, J = 1.9 Hz), 7.12 (1H, dt, J = 13.8, 4.5 Hz), 7.03-6.97 (1H, m), 6.94-6.90 (1H, m), 6.92 (1H, d, J = 1.6 Hz), 4.92 (2H, brs), 4.02-3.94 (2H, m), 2.91 (2H, t, J = 7.2 Hz), 2.52 (2H, t, J = 7.2 Hz), 1.56-1.53 (1H, m), 1.37-1.25 (8H, m), 0.90-0.86 (6H, m).

### (37c) 2-Ethylhexyl 3-[6-(5-cyanopyridin-2-ylamino)-5-(2,4-difluorophenoxy)pyridin-3-ylsulfanyl]propionate

Analogously to Example (1d), the desired title compound (1.84 g, 73 %) was obtained from the compound (2.04 g, 4.65 mmol) obtained in Example (37b).
¹H-NMR (CDCl₃, 400 MHz) δ: 8.73 (1H, d, J = 9.0 Hz), 8.55 (1H, d, J = 1.5 Hz), 8.28 (1H, brs), 8.11 (1H, d, J = 1.9 Hz), 7.93 (1H, dd, J= 8.6, 2.3 Hz), 7.21 (1H, dt, J = 13.6, 4.5 Hz), 7.07-6.96 (2H, m), 6.98 (1H, d, J = 1.9 Hz), 4.00-3.95 (2H, m), 3.01 (2H, t, J = 7.2 Hz), 2.56 (2H, t, J = 7.2 Hz), 1.56-1.53 (1H, m), 1.37-1.25 (8H, m), 0.90-0.86 (6H, m).

### (37d) 6-{5-[3-(tert-Butyldimethylsiloxy)-1-methylpropylsulfanyl]-3-(2,4-difluorophenoxy)pyridin-2-ylamino}nicotinonitrile

Analogously to Example (17a), the desired title compound (0.34 g, 63 %) was obtained as a pale yellow solid from the compound (0.54 g, 1.0 mmol) obtained in Example (37c).
¹H-NMR (CDCl₃, 400 MHz) δ: 8.75 (1H, d, J= 7.8 Hz), 8.55 (1H, s), 8.28 (1H, brs), 8.11 (1H, d, J = 2.0 Hz), 7.92 (1H, dd, J = 8.8, 2.1 Hz), 7.22 (1H, dt, J = 17.5, 7.7 Hz), 7.06-7.04 (1H, m), 7.03-6.94 (2H, m), 3.77-3.72 (1H, m), 3.69-3.64 (1H, m), 3.21-3.15 (1H, m), 1.76-1.61 (2H, m), 1.21 (3H, d, J = 6.7 Hz), 0.87 (9H, s), 0.03 (3H, s), 0.02 (3H, s).

### (37e) 6-[3-(2,4-Difluorophenoxy)-5-(3-hydroxy-1-methylpropylsulfanyl)pyridin-2-ylamino]nicotinonitrile

Analogously to Example (27b), the desired title compound (236 mg, 87 %) was obtained as a colorless solid from the compound (0.34 g, 0.63 mmol) obtained in Example (37d).
¹H-NMR (DMSO-d₆, 500 MHz) δ: 9.61 (1H, s), 8.67 (1H, s), 8.15-8.08 (3H, m), 7.54-7.50 (1H, m), 7.40-7.35 (1H, m), 7.40 (1H, s), 7.16-7.14 (1H, m), 4.51 (1H, t, J = 5.1 Hz), 3.52-3.44 (2H, m), 3.30-3.26 (1H, m), 1.66-1.52 (2H, m), 1.17 (3H, d, J = 6.8 Hz).

### (37f) 3-{5-(2,4-Difluorophenoxy)-6-[5-(1H-tetrazol-5-yl)pyridin-2-ylamino]pyridin-3-ylsulfanyl]butan-1-ol

Analogously to Example (1e), the desired title compound (206 mg, 79 %) was obtained as a pale yellow solid from the compound (236 mg, 0.55 mmol) obtained in Example (37e).
¹H-NMR (DMSO-d₆, 400 MHz) δ: 9.30 (1H, brs), 8.90 (1H, s), 8.37-8.32 (2H, m), 8.13 (1H, d, J = 2.0 Hz), 7.58-7.52 (1H, m), 7.45-7.39 (1H, m), 7.21-7.16 (2H, m), 4.51 (1H, brs), 3.53-3.43 (2H, m), 3.28-3.22 (1H, m), 1.66-1.51 (2H, m), 1.16 (3H, d, J = 6.6 Hz);
MS (FAB, m/z): 472 (M+H)⁺.

### (Example 38)

### [3-(2,4-Difluorophenoxy)-5-trifluoromethylpyridin-2-yl]-[5-(1H-tetrazol-5-yl)pyridin-2-yl]amine

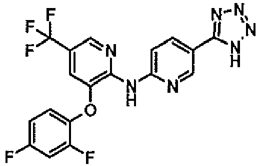

### (38a) 3-(2,4-Difluorophenoxy)-5-trifluoromethylpyridine-2-carbonitrile

To an N,N-dimethylformamide solution (6 mL) of 2,4-difluorophenol (0.57 mL, 6.0 mmol), sodium hydride (content 63%) (0.23 g, 6.0 mmol) was added under a nitrogen stream at 0°C, followed by stirring for 10 minutes. Subsequently, to the reaction solution, 3-chloro-5-trifluoromethylpyridine-2-carbonitrile (2.8 g, 12.3 mmol) was added, followed by stirring for 1 hour. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane-ethyl acetate) to afford the desired title compound (1.3 g, 71 %). ¹H-NMR (CDCl₃, 400 MHz) δ: 8.68-8.67 (1H, m), 7.32-7.27 (2H, m), 7.11-7.02 (2H, m).

### (38b) [3-(2,4-Difluorophenoxy)-5-trifluoromethylpyridine-2-carboxamide

The compound (1.3 g, 4.3 mmol) obtained in Example (38a) was dissolved in sulfuric acid (2.5 mL) under a nitrogen stream, followed by stirring at 50°C for 2 hours. The reaction solution was ice-cooled and neutralized with an aqueous sodium hydrogencarbonate to pH 6. Subsequently, extraction was carried out with ethyl acetate, and the extract was washed with water and brine sequentially. The resulting organic layer was dried over anhydrous sodium sulfate and subsequently concentrated under reduced pressure to afford a crude product (1.26 g) of the desired title compound as a white solid, which was used in the next step without further purification.

### (38c) 2-Amino-3-(2,4-difluorophenoxy)-5-trifluoromethylpyridine

Analogously to Example (1c), the desired title compound (0.55 g, 46 %) was obtained from the compound (1.2 g, 3.8 mmol) obtained in Example (38b).
¹H-NMR (CDCl₃, 400 MHz) δ: 8.09-8.08 (1H, m), 7.18-7.12 (1H, m), 7.05-6.99 (1H, m), 7.97-6.92 (1H, m), 6.87 (1H, s), 5.12 (2H, brs).

### (38d) 6-[3-(2,4-Difluorophenoxy)-5-trifluoromethylpyridin-2-ylamino]nicotinonitrile

Analogously to Example (1d), the desired title compound (0.47 g, 71 %) was obtained from the compound (0.49 g, 1.7 mmol) obtained in Example (38c).
¹H-NMR (DMSO-d₆, 400 MHz) δ: 8.80 (1H, d, J = 8.8 Hz), 8.58 (1H, s), 8.45 (1H, s), 8.32 (1H, s), 7.97 (1H, dd, J = 8.8, 1.5 Hz), 7.26-7.22 (1H, m), 7.09-6.99 (3H, m).

### (38e) [3-(2,4-Difluorophenoxy)-5-trifluoromethylpyridin-2-yl]-[5-(1H-tetrazol-5-yl)pyridin-2-yl]amine

Analogously to Example (1e), the desired title compound (0.14 g, 64 %) was obtained from the compound (0.20 g, 0.5 mmol) obtained in Example (38d).
¹H-NMR (DMSO-d₆, 500 MHz) δ: 9.65 (1H, s), 8.95 (1H, s), 8.46-8.39 (3H, m), 7.57-7.54 (1H, m), 7.50-7.45 (1H, m), 7.42 (1H, s), 7.22-7.18 (1H, m);
LCMS (ESI, m/z): 436 (M+H)⁺, retention time: 2.0 min.

### (Example 39)

### 3-{6-[3-(4-Fluorophenoxy)-5-(pyridin-2-ylsulfanyl)-pyridin-2-ylamino]pyridin-3-yl}-4H-[1,2,4]oxadiazol-5-one

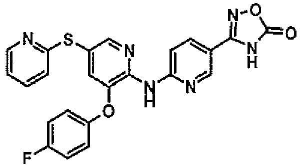

### (39a) 6-[3-(4-Fluorophenoxy)-5-(pyridin-2-ylsulfanyl)pyridin-2-ylamino]-N-hydroxynicotineamidine

The compound (0.10 g, 0.25 mmol) obtained in Example (2d) was dissolved in ethanol (2.5 mL), and a 50% hydroxyamine aqueous solution (0.25 mL) was added, followed by heating to reflux for 2 hours. After the reaction, the solvent was distilled off, and water was added to the residue to allow it to be suspended. The deposited solid was filtered off to afford a crude product (105 mg) of the desired title compound.

### (39b) 3-{6-[3-(4-Fluorophenoxy)-5-(pyridin-2-ylsulfanyl)pyridin-2-ylamino]pyridin-3-yl}-4H-[1,2,4]oxadiazol-5-one

To a 1,4-dioxane (10 mL) solution of the compound (0.10 g, 0.23 mmol) obtained in Example (39a), 1,1-carbonyldiimidazole (45 mg, 0.28 mmol) and 1,8-diazabicyclo[5,4,0]undec-7-ene (39 µL, 0.26 mmol) were added, followed by heating to reflux at 110°C for 2 hours. After the reaction, a 10% citric acid aqueous solution was added, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (dichloromethane-methanol) to afford the desired title compound (48 mg, 43 %).
¹H-NMR (DMSO-d₆, 500 MHz) δ: 13.0 (1H, brs), 9.37 (1H, s), 8.67 (1H, s), 8.39-8.38 (1H, m), 8.35 (1H, d, J = 8.8 Hz), 8.26 (1H, s), 8.14 (1H, d, J = 8.8 Hz), 7.67 (1H, t, J = 7.8 Hz), 7.35 (1H, s), 7.30-7.24 (4H, m), 7.16 (1H, t, J = 6.8 Hz), 7.11 (1H, d, J = 7.8 Hz);
LCMS (ESI, m/z): 475 (M+H)⁺, retention time: 2.1 min.

### (Example 40)

### 3-{6-[3-(3,4-Difluorophenoxy)-5-(pyridin-2-ylsulfanyl)pyridin-2-ylamino]pyridin-3-yl}-4H-[1,2,4]oxadiazol-5-one

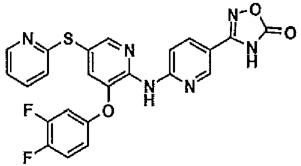

### (40a) 6-[3-(3,4-Difluorophenoxy)-5-(pyridin-2-ylsulfanyl)pyridin-2-ylamino]-N-hydroxynicotineamidine

Analogously to Example (39a), a crude product (0.13 g) of the desired title compound was obtained from the compound (0.13 g, 0.3 mmol) obtained in Example (1d).

### (40b) 3-{6-[3-(3,4-Difluorophenoxy)-5-(pyridin-2-ylsulfanyl)pyridin-2-ylamino]pyridin-3-yl}-4H-[1,2,4]oxadiazol-5-one

Analogously to Example (39b), the desired title compound (84 mg, 61%) was obtained from the compound (0.10 g, 0.23 mmol) obtained in Example (40a).
¹H-NMR (DMSO-d₆, 500 MHz) δ: 13.0 (1H, brs), 9.47 (1H, s), 8.66 (1H, s), 8.38 (1H, d, J = 4.9 Hz), 8.32 (1H, d, J = 8.8 Hz), 8.30-8.29 (1H, m), 8.16-8.13 (1H, m), 7.70-7.66 (1H, m), 7.54-7.42 (2H, m), 7.42-7.38 (1H, m), 7.19-7.14 (2H, m), 7.06-7.04 (1H, m);
LCMS (ESI, m/z): 493 (M+H)⁺, retention time: 2.1 min.

### (Example 41)

### 3-{6-[3-(2,4-Difluorophenoxy)-5-trifluoromethylpyridin-2-ylamino]pyridin-3-yl}-4H-[1,2,4]oxadiazol-5-one

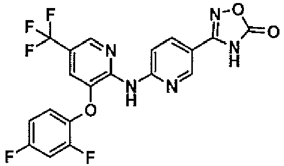

### (41a) 6-[3-(2,4-Difluorophenoxy)-5-trifluoromethylpyridin-2-ylamino]-N-hydroxynicotineamidine

Analogously to Example (39a), a crude product (0.20 g) of the desired title compound was obtained from the compound (0.20 g, 0.5 mmol) obtained in Example (38d).

### (41b) 3-{6-[3-(2,4-Difluorophenoxy)-5-trifluoromethylpyridin-2-ylamino]pyridin-3-yl}-4H-[1,2,4]oxadiazol-5-one

Analogously to Example (39b), the desired title compound (0.15 g, 71%) was obtained from the compound (0.20 g, 0.47 mmol) obtained in Example (41a).
¹H-NMR (DMSO-d₆, 500 MHz) δ: 13.0 (1H, brs), 9.74 (1H, s), 8.71 (1H, s), 8.46 (1H, s), 8.38 (1H, d, J = 8.8 Hz), 8.18 (1H, d, J = 8.8 Hz), 8.58-8.54 (1H, m), 7.49-7.44 (2H, m), 7.21-7.17 (1H, m) ;
LCMS (ESI, m/z): 452 (M+H)⁺, retention time: 2.1 min.

### (Example 42)

### 3-{6-[3-(4-Fluorophenoxy)-5-(3-hydroxy-1-methylpropylsulfanyl)pyridin-2-ylamino]pyridin-3-yl]-4H-[1,2,4]oxadiazol-5-one

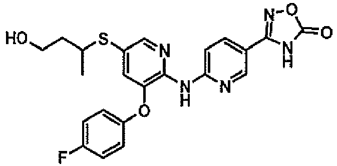

### (42a) 3-{6-[5-(3-tert-Butyldimethylsiloxy-1-methylpropylsulfanyl)-3-(4-fluorophenoxy)pyridin-2-ylamino]pyridin-3-yl}-4H[1,2,4]oxadiazol-5-one

Analogously to Example (39a), 6-{5-[3-(tert-butyldimethylsiloxy)-1-methylpropylsulfanyl]-3-(4-fluorophenoxy)pyridin-2-ylamino}-N-hydroxynicotineamidine was obtained from the compound (0.38 g, 0.72 mmol) obtained in Example (27a). The resulting compound was used in the next reaction without purification, and analogously to Example (39b), the desired title compound (117 mg, 30 %) was obtained as a pale yellow oil.
¹H-NMR (CDCl₃, 400 MHz) δ: 8.80 (1H, d, J = 9.0 Hz), 8.68 (1H, d, J = 2.4 Hz), 8.25 (1H, brs), 8.13 (1H, d, J = 1.9 Hz), 8.08 (1H, dd, J = 9.0, 2.4 Hz), 7.15-7.06 (5H, m), 3.74-3.66 (2H, m), 3.21-3.15 (1H, m), 1.75-1.64 (2H, m), 1.23 (3H, d, J = 6.6 Hz), 0.87 (9H, s), 0.03 (3H, s), 0.02 (3H, s).

### (42b) 3-{6-[3-(4-Fluorophenoxy)-5-(3-hydroxy-1-methylpropylsulfanyl)pyridin-2-ylamino]pyridin-3-yl}-4H-[1,2,4]oxadiazol-5-one

Analogously to Example (27b), the desired title compound (41 mg, 44 %) was obtained as a colorless solid from the compound (117 mg, 0.20 mmol) obtained in Example (42a).
¹H-NMR (DMSO-d₆, 400 MHz) δ: 13.0 (1H, brs), 9.23 (1H, brs), 8.63 (1H, d, J = 1.9 Hz), 8.24 (1H, d, J = 9.0 Hz), 8.13 (1H, d, J = 1.9 Hz), 8.12 (1H, dd, J = 10.2, 2.4 Hz), 7.35-7.19 (5H, m), 4.52 (1H, t, J = 5.1 Hz), 3.54-3.45 (2H, m), 3.30-3.24 (1H, m), 1.74-1.50 (2H, m), 1.18 (3H, d, J = 6.6 Hz);
MS (FAB, m/z): 470 (M+H)⁺.

### (Example 43)

### [3-(2,4-Difluorophenoxy)-5-(3-methoxypropylsulfanyl)pyridin-2-yl]-[5-(1H-tetrazol-5-yl)pyridin-2-yl]amine

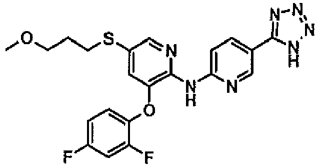

### (43a) 6-[3-(2,4-Difluorophenoxy)-5-(3-methoxypropylsulfanyl)pyridin-2-ylamino]nicotinonitrile

Analogously to Example (17a), the desired title compound (276 mg, 65 %) was obtained as a pale yellow solid from the compound (0.54 g, 1.00 mmol) obtained in Example (37c).
¹H-NMR (CDCl₃, 400 MHz) δ: 8.72 (1H, d, J = 8.6 Hz), 8.55 (1H, d, J = 2.4 Hz), 8.25 (1H, brs), 8.08 (1H, d, J = 2.0 Hz), 7.92 (1H, dd, J = 8.6, 2.4 Hz), 7.20 (1H, dt, J = 13.7, 4.4 Hz), 7.07-7.03 (1H, m), 7.02-6.96 (1H, m), 6.95 (1H, d, J = 2.0 Hz), 3.43 (2H,
t, J = 5.9 Hz), 3.30 (3H, s), 2.86 (2H, t, J = 7.2 Hz), 1.82 (2H, quint, J = 7.4 Hz).

### (43b) [3-(2,4-Difluorophenoxy)-5-(3-methoxypropylsulfanyl)pyridin-2-yl]-[5-(1H-tetrazol-5-yl)pyridin-2-yl]amine

Analogously to Example (1e), the desired title compound (226 mg, 75 %) was obtained as a pale yellow solid from the compound (275 mg, 0.64 mmol) obtained in Example (43a).
¹H-NMR (DMSO-d₆, 400 MHz) δ: 9.22 (1H, brs), 8.88 (1H, d, J = 2.3 Hz), 8.34 (1H, dd, J = 9.0, 2.3 Hz), 8.27 (1H, d, J = 9.0 Hz), 8.12 (1H, d, J = 2.0 Hz), 7.57-7.51 (1H, m), 7.41 (1H, dt, J = 14.2, 4.5 Hz), 7.20 (1H, d, J = 2.0 Hz), 7.18-7.15 (1H, m), 3.36 (2H, t, J = 6.2 Hz), 3.18 (3H, s), 2.90 (2H, t, J = 7.2 Hz), 1.71 (2H, quint, J = 7.1 Hz);
MS (FAB, m/z): 472 (M+H)⁺.

### (Example 44)

### [3-(2,4-Difluorophenoxy)-5-(3-methoxy-1-methylpropylsulfanyl)pyridin-2-yl]-[5-(1H-tetrazol-5-yl)pyridin-2-yl]amine

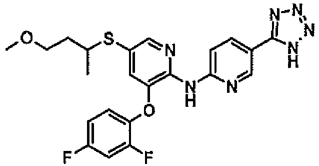

### (44a) 6-[5-(3-Methoxy-1-methylpropylsulfanyl)-3-(2,4-difluorophenoxy)pyridin-2-ylamino]nicotinonitrile

Analogously to Example (17a), the desired title compound (418 mg, 32 %) was obtained as a pale yellow oil from the compound (1.62 g, 3.0 mmol) obtained in Example (37c).
¹H-NMR (CDCl₃, 400 MHz) δ: 8.74 (1H, d, J = 9.0 Hz), 8.55 (1H, d, J = 2.4 Hz), 8.28 (1H, brs), 8.11 (1H, d, J = 1.6 Hz), 7.93 (1H, dd, J = 9.2, 2.5 Hz), 7.20 (1H, dt, J = 13.7, 4.4 Hz), 7.06-6.95 (2H, m), 6.98 (1H, d, J = 1.6 Hz), 3.53-3.41 (2H, m), 3.29 (3H, s), 3.16-3.11 (1H, m), 1.81-1.67 (2H, m), 1.22 (3H, d, J = 6.7 Hz).

### (44b) [3-(2,4-Difluorophenoxy)-5-(3-methoxy-1-methylpropylsulfanyl)pyridin-2-yl]-[5-(1H-tetrazol-5-yl)pyridin-2-yl]amine

Analogously to Example (1e), the desired title compound (128 mg, 69 %) was obtained as a pale yellow solid from the compound (163 mg, 0.38 mmol) obtained in Example (44a).
¹H-NMR (DMSO-d₆, 400 MHz) δ: 9.12 (1H, brs), 8.88 (1H, t, J = 1.8 Hz), 8.33 (2H, d, J = 1.6 Hz), 8.12 (1H, d, J = 2.0 Hz), 7.59-7.53 (1H, m), 7.44 (1H, dt, J = 14.1, 4.6 Hz), 7.22-7.17 (1H, m), 7.15 (1H, d, J = 1.6 Hz), 3.45-3.34 (2H, m), 3.21-3.13 (1H, m), 3.17 (3H, s), 1.70-1.59 (2H, m), 1.17 (3H, d, J = 6.7 Hz); MS (FAB, m/z): 486 (M+H)⁺.

### (Example 45)

### 3-{6-[3-(2,4-Difluorophenoxy)-5-(3-methoxypropylsulfanyl)pyridin-2-ylamino]pyridin-3-yl}-4H-[1,2,4]oxadiazol-5-one

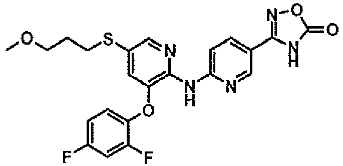

### (45a) 6-[3-(2,4-Difluorophenoxy)-5-(3-methoxypropylsulfanyl)pyridin-2-ylamino]-N-hydroxynicotineamidine

Analogously to Example (39a), a crude product (540 mg, 100 %) of the desired title compound was obtained as a colorless oil from the compound (497 mg, 1.16 mmol) obtained in Example (43a).

### (45b) 3-{6-[3-(2,4-Difluorophenoxy)-5-(3-methoxypropylsulfanyl)pyridin-2-ylamino]pyridin-3-yl}-4H-[1,2,4]oxadiazol-5-one

Analogously to Example (39b), the desired title compound (293 mg, 51 %) was obtained as a colorless solid from the compound (540 mg, 1.16 mmol) obtained in Example (45a).
¹H-NMR (DMSO-d₆, 400 MHz) δ: 13.0 (1H, brs), 9.34 (1H, brs), 8.64 (1H, d, J = 2.4 Hz), 8.18 (1H, d, J = 8.6 Hz), 8.10 (1H, dd, J = 8.1, 3.3 Hz), 8.11 (1H, d, J = 1.9 Hz), 7.56-7.50 (1H, m), 7.39 (1H, dt, J = 14.2, 4.7 Hz), 7.20 (1H, d, J = 1.9 Hz), 7.21-7.15 (1H, m), 3.35 (2H, t, J = 6.1 Hz), 3.18 (3H, s), 2.90 (2H, t, J = 7.2 Hz), 1.75-1.68 (2H, m);
MS (FAB, m/z): 488 (M+H)⁺.

### (Example 46)

### 3-{6-[3-(2,4-Difluorophenoxy)-5-(3-methoxy-1-methylpropylsulfanyl)pyridin-2-ylamino]pyridin-3-yl}-4H-[1,2,4]oxadiazol-5-one

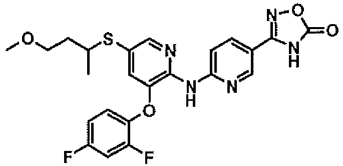

### (46a) 6-[3-(2,4-Difluorophenoxy)-5-(3-methoxy-1-methylpropylsulfanyl)pyridin-2-ylamino]-N-hydroxynicotineamidine

Analogously to Example (39a), a crude product (279 mg, 99 %) of the desired title compound was obtained as a colorless oil from the compound (254 mg, 0.59 mmol) obtained in Example (44a).

### (46b) 3-{6-[3-(2,4-Difluorophenoxy)-5-(3-methoxy-1-methylpropylsulfanyl)pyridin-2-ylamino]pyridin-3-yl}-4H-[1,2,4]oxadiazol-5-one

Analogously to Example (39b), the desired title compound (185 mg, 60 %) was obtained as a colorless solid from the compound (279 mg, 0.61 mmol) obtained in Example (46a).
¹H-NMR (DMSO-d₆, 400 MHz) δ: 13.0 (1H, brs), 9.40 (1H, brs), 8.66 (1H, d, J = 2.4 Hz), 8.25 (1H, d, J = 8.9 Hz), 8.13 (1H, dd, J = 8.6, 2.8 Hz), 8.12 (1H, d, J = 2.0 Hz), 7.58-7.52 (1H, m), 7.41 (1H, dt, J = 14.1, 4.6 Hz), 7.21-7.18 (1H, m), 7.18 (1H, d, J = 2.0 Hz), 3.43-3.35 (2H, m), 3.23-3.18 (1H, m), 3.16 (3H, s), 1.69-1.62 (2H, m), 1.17 (3H, d, J = 7.1 Hz);
MS (FAB, m/z): 502 (M+H)⁺.

### (Example 47)

### [5-(Cyclopropylmethylsulfanyl)-3-(2,4-difluorophenoxy)pyridin-2-yl]-[5-(1H-tetrazol-5-yl)pyridin-2-yl]amine

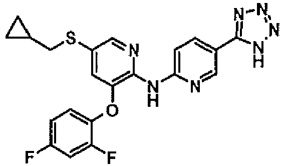

### (47a) 6-[5-(Cyclopropylmethylsulfanyl)-3-(2,4-difluorophenoxy)pyridin-2-ylamino]nicotinonitrile

Analogously to Example (17a), the desired title compound (213 mg, 61 %) was obtained as a pale yellow oil from the compound (463 mg, 0.86 mmol) obtained in Example (37c).
¹H-NMR (CDCl₃, 400 MHz) δ: 8.71 (1H, dd, J = 9.0, 0.8 Hz), 8.54 (1H, dd, J = 2.4, 0.8 Hz), 8.25 (1H, s), 8.12 (1H, d, J = 2.0 Hz), 7.91 (1H, dd, J = 9.0, 2.4 Hz), 7.19 (1H, dt, J = 9.0, 5.5 Hz), 7.06-6.94 (3H, m), 2.70 (2H, d, J = 7.0 Hz), 0.99-0.86 (1H, m), 0.55-0.50 (2H, m), 0.17-0.13 (2H, m);
MS (FAB, m/z): 411 (M+H)⁺.

### (47b) [5-(Cyclopropylmethylsulfanyl)-3-(2,4-difluorophenoxy)pyridin-2-yl]-[5-(1H-tetrazol-5-yl)pyridin-2-yl]amine

Analogously to Example (1e), the desired title compound (141 mg, 60 %) was obtained as a pale yellow solid from the compound (213 mg, 0.52 mmol) obtained in Example (47a).
¹H-NMR (DMSO-d₆, 400 MHz) δ: 9.23 (1H, s), 8.88 (1H, dd, J = 2.4, 0.8 Hz), 8.33 (2H, dd, J = 9.0, 2.4 Hz), 8.27 (1H, dd, J = 9.0, 0.8 Hz), 8.14 (1H, d, J = 2.0 Hz), 7.56-7.50 (1H, m), 7.40 (1H, dt, J = 9.0, 5.5 Hz), 7.22 (1H, d, J = 1.6 Hz), 7.20-7.14 (1H, m), 2.83 (2H, d, J = 7.0 Hz), 0.96-0.88 (1H, m), 0.49-0.44 (2H, m), 0.17-0.13 (2H, m);
MS (FAB, m/z): 454 (M+H)⁺.

### (Example 48)

### [5-Ethyl-3-(2,4-difluorophenoxy)pyridin-2-yl]-[5-(1H-tetrazol-5-yl)pyridin-2-yl]amine

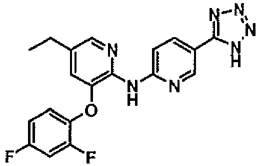

### (48a) 6-[5-Bromo-3-(2,4-difluorophenoxy)pyridin-2-ylamino]nicotinonitrile

Analogously to Example (1d), the desired title compound (0.62 g, 31 %) was obtained from 2-amino-5-bromo-3-(2,4-difluorophenoxy)pyridine (1.5 g, 5.0 mmol) synthesized in Example (37b).
¹H-NMR (DMSO-d₆, 400 MHz) δ: 7.69 (1H, s), 8.67 (1H, d, J = 2.4 Hz), 8.24-8.23 (1H, m), 8.14 (1H, dd, J = 9.0, 2.4 Hz), 8.01 (1H, d, J = 9.0 Hz), 7.55-7.50 (1H, m), 7.45-7.38 (2H, m), 7.19-7.14 (1H, m).

### (48b) 6-[5-Ethyl-3-(2,4-difluorophenoxy)pyridin-2-ylamino]nicotinonitrile

The compound (0.30 g, 0.74 mmol) obtained in Example (48a) and [1,1'-bis(diphenylphosphino)ferrocene]palladium dichloride dichloromethane adduct (II) (30 mg, 0.037 mmol) were dissolved in 1,4-dioxane (5 mL) under a nitrogen stream, and 1.09M diethyl zinc hexane solution (1.4 mL) was added dropwise, followed by stirring at room temperature for 1 hour. Further, heating to reflux was carried out for 2 hours. After quenching the reaction by saturated brine, extraction was carried out with ethyl acetate, and the resulting organic layer was dried over anhydrous sodium sulfate and subsequently concentrated under reduced pressure to give a residue. The resulting residue was purified using silica gel column chromatography (dichloromethane-methanol) to afford the desired title compound (0.24 g, 92 %).
¹H-NMR (DMSO-d₆, 400 MHz) δ: 9.40 (1H, s), 8.60 (1H, dd, J = 2.4, 0.8 Hz), 8.06 (1H, dd, J = 9.0, 2.4 Hz), 8.00 (1H, d, J = 2.0 Hz), 7.93 (1H, dd, J = 9.0, 0.8 Hz), 7.52-7.46 (1H, m), 7.31-7.28 (1H, m), 7.16-7.11 (2H, m), 2.55 (2H, q, J = 7.8 Hz), 1.12 (3H, t, J = 7.8 Hz).

### (48c) [5-Ethyl-3-(2,4-difluorophenoxy)pyridin-2-yl]-[5-(1H-tetrazol-5-yl)pyridin-2-yl]amine

Analogously to Example (1e), the desired title compound (91 mg, 68 %) was obtained from the compound (0.12 g, 0.34 mmol) obtained in Example (48b).
¹H-NMR (DMSO-d₆, 400 MHz) δ: 9.03 (1H, brs), 8.84 (1H, s), 8.31-8.28 (1H, m), 8.21 (1H, d, J = 8.8 Hz), 8.00 (1H, s), 7.54-7.49 (1H, m), 7.38-7.33 (1H, m), 7.18-7.14 (1H, m), 7.10 (1H, s), 2.58-2.53 (2H, m), 1.13 (3H, t, J = 7.8 Hz);
LCMS (ESI, m/z): 396 (M+H)⁺, retention time: 1.9 min.

### (Example 49)

### 3-{6-[5-Ethyl-3-(2,4-difluorophenoxy)pyridin-2-ylamino]pyridin-3-yl}-4H-[1,2,4]oxadiazol-5-one

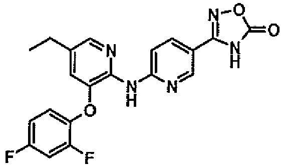

### (49a) 6-[5-Ethyl-3-(2,4-difluorophenoxy)pyridin-2-ylamino]-N-hydroxynicotineamidine

Analogously to Example (39a), a crude product (0.10 g, 78 %) of the desired title compound was obtained from the compound (0.12 g, 0.34 mmol) obtained in Example (48b).

### (49b) 3-{6-[5-Ethyl-3-(2,4-difluorophenoxy)pyridin-2-ylamino]pyridin-3-yl}-4H-[1,2,4]oxadiazol-5-one

Analogously to Example (39b), the desired title compound (76 mg, 72 %) was obtained from the compound (0.10 g, 0.26 mmol) obtained in Example (49a).
¹H-NMR (DMSO-d₆, 400 MHz) δ: 12.9 (1H, brs), 9.15 (1H, s), 8.58 (1H, s), 8.10 (1H, d, J = 8.8 Hz), 8.07-8.05 (1H, m), 7.99 (1H, s), 7.53-7.48 (1H, m), 7.35-7.30 (1H, m), 7.16-7.13 (1H, m), 7.10 (1H, s), 2.57-2.52 (2H, q, J = 7.3 Hz), 1.12 (3H, t, J = 7.3 Hz);
LCMS (ESI, m/z): 412 (M+H)⁺, retention time: 2.0 min.

### (Example 50)

### 3-{6-[3-(3,4-Difluorophenoxy)-5-(3-methoxypropylsulfanyl)pyridin-2-ylamino]pyridin-3-yl}-4H-[1,2,4]oxadiazol-5-one

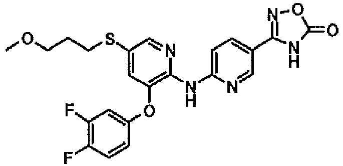

### (50a) 6-[3-(3,4-Difluorophenoxy)-5-(3-methoxypropylsulfanyl)pyridin-2-ylamino]-N-hydroxynicotineamidine

Analogously to Example (39a), a crude product (0.17 g, 85 %) of the desired title compound was obtained from the compound (0.19 g, 0.44 mmol) obtained in Example (28a).

### (50b) 3-{6-[3-(3,4-Difluorophenoxy)-5-(3-methoxypropylsulfanyl)pyridin-2-ylamino]pyridin-3-yl}-4H-[1,2,4]oxadiazol-5-one

Analogously to Example (39b), the desired title compound (0.14 g, 79 %) was obtained from the compound (0.17 g, 0.37 mmol) obtained in Example (50a).
¹H-NMR (DMSO-d₆, 400 MHz) δ: 12.9 (1H, brs), 9.27 (1H, s), 8.59 (1H, dd, J = 2.0, 0.8 Hz), 8.14 (1H, d, J = 2.0 Hz), 8.09 (1H, d, J = 1.8 Hz), 8.08 (1H, d, J = 2.0 Hz), 7.52-7.45 (1H, m), 7.40 (1H, d, J = 2.4 Hz), 7.32 (1H, ddd, J = 11.7, 6.7, 2.7 Hz), 6.98-6.94 (1H, m), 3.37 (2H, t, J = 5.9 Hz), 3.19 (3H, s), 2.93 (2H, t, J = 7.0 Hz), 1.77-1.70 (2H, m);
LCMS (ESI, m/z): 488 (M+H)⁺, retention time: 2.1 min.

### (Example 51)

### [3-(4-Fluorophenoxy)-5--(3-methoxy-1-methylpropylsulfanyl)pyridin-2-yl]-[5-(1H-tetrazol-5-yl)pyridin-2-yl]amine

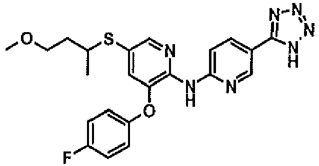

### (51a) 6-[5-(3-Methoxy-1-methylpropylsulfanyl)-3-(4-fluorophenoxy)pyridin-2-ylamino]nicotinonitrile

Analogously to Example (17a), the desired title compound (165 mg, 17 %) was obtained as a pale yellow oil from the compound (1.20 g, 2.30 mmol) obtained in Example (26b).
¹H-NMR (CDCl₃, 400 MHz) δ: 8.74 (1H, d, J = 9.0 Hz), 8.54 (1H, d, J = 1.6 Hz), 8.25 (1H, brs), 8.12 (1H, d, J = 2.0 Hz), 7.92 (1H, dd, J = 9.0, 2.4 Hz), 7.16-7.04 (5H, m), 3.53-3.41 (2H, m), 3.29 (3H, s), 3.17-3.11 (1H, m), 1.81-1.67 (2H, m), 1.23 (3H, d, J = 7.0 Hz).

### (51b) [3-(4-Fluorophenoxy)-5-(3-methoxy-1-methylpropylsulfanyl)pyridin-2-yl]-[5-(1H-tetrazol-5-yl)pyridin-2-yl]amine

Analogously to Example (1e), the desired title compound (125 mg, 67 %) was obtained as a pale yellow solid from the compound (165 mg, 0.39 mmol) obtained in Example (51a).
¹H-NMR (DMSO-d₆, 400 MHz) δ: 8.88 (1H, brs), 8.86 (1H, brs), 8.35-8.31 (2H, m), 8.12 (1H, d, J = 1.9 Hz), 7.33-7.20 (5H, m), 3.44-3.37 (2H, m), 3.19-3.14 (1H, m), 3.17 (3H, s), 1.70-1.62 (2H, m), 1.18 (3H, d, J = 6.6 Hz);
MS (FAB, m/z): 468 (M+H)⁺.

### (Example 52)

### [3-(2,4-Difluorophenoxy)-5-(4-methoxybutyl)pyridin-2-yl]-[5-(1H-tetrazol-5-yl)pyridin-2-yl]amine

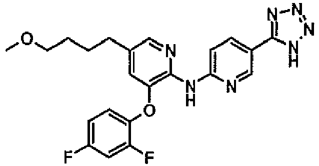

### (52a) 2-Amino-3-(2,4-difluorophenoxy)-5-(4-methoxybut-1-ynyl)pyridine

2-Amino-5-bromo-3-(2,4-difluorophenoxy)pyridine (602 mg, 2.0 mmol) obtained in Example (37b), 3-butynylmethyl ether (235 mg, 2.8 mmol), 10% palladium-carbon catalyst (water content 52%)(205 mg), copper iodide (38 mg, 0.2 mmol), triphenylphosphine (53 mg, 0.2 mmol), and diisopropylamine (0.34 mL, 2.4 mmol) were dissolved in a mixed solvent (12 mL) of N,N-dimethylacetamide and water (10:1) under a nitrogen stream, followed by stirring at 80°C for 8 hours. The reaction solution was filtered through Celite, and then poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane:ethyl acetate = 2:1) to afford the desired title compound (407 mg, 67 %) as a pale yellow solid.
¹H-NMR (CDCl₃, 500 MHz) δ: 7.91(1H, s), 7.12-7.07 (1H, m), 7.01-6.97 (1H, m), 6.92-6.88 (1H, m), 6.79 (1H, s), 4.88 (2H, brs), 3.54 (2H, t, J = 6.8 Hz), 3.39 (3H, s), 2.64 (2H, t, J = 6.8 Hz).

### (52b) 2-Amino-3-(2,4-difluorophenoxy)-5-(4-methoxybutyl)pyridine

To a methanol solution (8 mL) of the compound (406 mg, 1.33 mmol) obtained in Example (52a), 10% palladium-carbon catalyst (40 mg) was added under a hydrogen stream, followed by stirring at room temperature for 2 hours. The reaction solution was filtered through Celite to afford the desired title compound (387 mg, 100 %) as a colorless oil.
¹H-NMR (CDCl₃, 400 MHz) δ: 7.60 (1H, s), 7.09 (1H, dt, J = 13.6, 4.5 Hz), 7.03-6.98 (1H, m), 6.94-6.89 (1H, m), 6.73 (1H, s), 5.22 (2H, brs), 3.37-3.34 (2H, m), 3.31 (3H, s), 2.47-2.44 (2H, m), 1.57-1.53 (4H, m).

### (52c) 6-[3-(2,4-Difluorophenoxy)-5-(4-methoxybutyl)pyridin-2-ylamino]nicotinonitrile

Analogously to Example (4d), the desired title compound (368 mg, 72 %) was obtained as a pale yellow solid from the compound (386 mg, 1.25 mmol) obtained in Example (52b).
¹H-NMR (CDCl₃, 400 MHz) δ: 8.71 (1H, d, J = 9.0 Hz), 8.53 (1H, s), 8.18 (1H, brs), 7.89 (1H, d, J = 1.9 Hz), 7.89 (1H dd, J = 9.0, 2.3 Hz), 7.16 (1H, dt, J = 13.6, 4.5 Hz), 7.06-7.00 (1H, m), 6.97-6.95 (1H, m), 6.76 (1H, s), 3.37 (2H, t, J = 6.1 Hz), 3.31 (3H, s), 2.53 (2H, t, J = 7.0 Hz), 1.60-1.57 (4H, m).

### (52d) [3-(2,4-Difluorophenoxy)-5-(4-methoxybutyl)pyridin-2-yl]-[5-(1H-tetrazol-5-yl)pyridin-2-yl]amine

Analogously to Example (1e), the desired title compound (316 mg, 78 %) was obtained from the compound (368 mg, 0.90 mmol) obtained in Example (52c).
¹H-NMR (DMSO-d₆, 400 MHz) δ: 9.00 (1H, brs), 8.85 (1H, d, J = 2.0 Hz), 8.30 (1H, dd, J = 9.0, 2.4 Hz), 8.24 (1H, d, J = 8.6 Hz), 7.97 (1H, d, J = 1.9 Hz), 7.53 (1H, dt, J = 11.2, 4.3 Hz), 7.39-7.33 (1H, m), 7.19-7.15 (1H, m), 7.08 (1H, d, J = 1.6 Hz), 3.30 (2H, t, J = 6.2 Hz), 3.19 (3H, s), 2.53 (2H, t, J = 5.7 Hz), 1.55-1.46 (4H, m);
MS (ESI, m/z): 454 (M+H)⁺.

### (Example 53)

### [3-(4-Fluorophenoxy)-5-(4-methoxybutyl)pyridin-2-yl]-[5-(1H-tetrazol-5-yl)pyridin-2-yl]amine

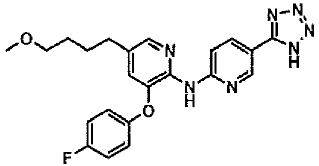

### (53a) 2-Amino-3-(4-fluorophenoxy)-5-(4-methoxybut-1-ynyl)pyridine

Analogously to Example (52a), the desired title compound (312 mg, 55 %) was obtained as a pale yellow solid from 2-amino-5-bromo-3-(4-fluorophenoxy)pyridine (566 mg, 2.0 mmol) obtained in Example (13c).
¹H-NMR (CDCl₃, 500 MHz) δ: 7.92 (1H, s), 7.06 (2H, d, J = 9.1 Hz), 6.99 (2H, dd, J = 9.1, 4.6 Hz), 6.91 (1H, s), 4.82 (2H, brs), 3.55 (2H, t, J = 6.8 Hz), 3.39 (3H, s), 2.64 (2H, t, J = 6.6 Hz).

### (53b) 2-Amino-3-(4-fluorophenoxy)-5-(4-methoxybutyl)pyridine

Analogously to Example (52b), the desired title compound (307 mg, 97 %) was obtained as a pale yellow oil from the compound (312 mg, 1.09 mmol) obtained in Example (53a).
¹H-NMR (CDCl₃, 400 MHz) δ: 7.69 (1H, d, J = 1.9 Hz), 7.08-7.04 (2H, m), 6.98-6.95 (2H, m), 6.83 (1H, d, J = 1.9 Hz), 4.61 (2H, brs), 3.38-3.35 (2H, m), 3.31 (3H, s), 2.48-2.45 (2H, m), 1.59-1.55 (4H, m).

### (53c) 6-[3-(4-Fluorophenoxy)-5-(4-methoxybutyl)pyridin-2-ylamino]nicotinonitrile

Analogously to Example (4d), the desired title compound (273 mg, 66 %) was obtained as a colorless solid from the compound (307 mg, 1.06 mmol) obtained in Example (53b).
¹H-NMR (CDCl₃, 400 MHz) δ: 8.72 (1H, d, J = 8.9 Hz), 8.51 (1H, d, J = 2.3 Hz), 8.15 (1H, brs), 7.90 (1H, d, J = 1.9 Hz), 7.88 (1H, dd, J = 9.1, 2.5 Hz), 7.14-7.09 (2H, m), 7.06-7.02 (2H, m), 6.89 (1H, d, J = 1.9 Hz), 3.37 (2H, t, J = 5.6 Hz), 3.31 (3H, s), 2.53 (2H, t, J= 7.0 Hz), 1.60-1.58 (4H, m).

### (53d) [3-(4-Fluorophenoxy)-5-(4-methoxybutyl)pyridin-2-yl]-[5-(1H-tetrazol-5-yl)pyridin-2-yl]amine

Analogously to Example (1e), the desired title compound (301 mg, 100 %) was obtained from the compound (272 mg, 0.69 mmol) obtained in Example (53c).
¹H-NMR (DMSO-d₆, 400 MHz) δ: 8.84 (1H, brs), 8.82 (1H, d, J = 2.0 Hz), 8.29 (1H, dd, J = 9.0, 2.4 Hz), 8.23 (1H, d, J = 9.0 Hz), 7.99 (1H, d, J = 1.9 Hz), 7.29-7.25 (2H, m), 7.18-7.14 (2H, m), 7.15 (1H, d, J = 1.9 Hz), 3.31 (2H, t, J = 6.2 Hz), 3.20 (3H, s), 2.54 (2H, t, J = 7.8 Hz), 1.55-1.49 (4H, m);
MS (ESI, m/z): 436 (M+H)⁺.

### (Example 54)

### [5-(2-Cyclopropylethyl)-3-(4-fluorophenoxy)pyridin-2-yl]-[5-(1H-tetrazol-5-yl)pyridin-2-yl]amine

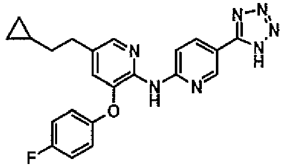

### (54a) 2-Amino-5-(cyclopropylethynyl)-3-(4-fluorophenoxy)pyridine

Analogously to Example (52a), the desired title compound (475 mg, 65 %) was obtained as a brown oil from 2-amino-5-bromo-3-(4-fluorophenoxy)pyridine (770 mg, 2.72 mmol) obtained in Example (13c) and cyclopropylethyne (252 mg, 3.81 mmol).
¹H-NMR (CDCl₃, 400 MHz) δ: 7.90 (1H, d, J = 1.5 Hz), 7.09-7.04 (2H, m), 7.02-6.98 (2H, m), 6.89 (1H, d, J = 1.5 Hz), 4.81 (2H, brs), 1.42-1.36 (1H, m), 0.85-0.81 (2H, m), 0.78-0.72 (2H, m).

### (54b) 2-Amino-5-(2-cyclopropylethyl)-3-(4-fluorophenoxy)pyridine

Analogously to Example (52b), the desired title compound (464 mg, 97 %) was obtained as a colorless oil from the compound (474 mg, 1.77 mmol) obtained in Example (54a).
¹H-NMR (CDCl₃, 500 MHz) δ: 7.72 (1H, s), 7.07-7.04 (2H, m), 6.98-6.95 (2H, m), 6.85 (1H, s), 4.58 (2H, brs), 2.55 (2H, t, J = 7.5 Hz), 1.41 (2H, q, J = 7.1 Hz), 0.66-0.63 (1H, m), 0.42-0.39 (2H, m), 0.03-0.00 (2H, m).

### (54c) 6-[5-(2-Cyclopropylethyl)-3-(4-fluorophenoxy)pyridin-2-ylamino]nicotinonitrile

Analogously to Example (4d), the desired title compound (357 mg, 56 %) was obtained as a colorless solid from the compound (464 mg, 1.70 mmol) obtained in Example (54b).
¹H-NMR (CDCl₃, 400 MHz) δ: 8.73 (1H, d, J = 9.0 Hz), 8.51 (1H, d, J = 1.5 Hz), 8.14 (1H, brs), 7.92 (1H, d, J = 2.0 Hz), 7.88 (1H, dd, J = 9.0, 2.4 Hz), 7.14-7.09 (2H, m), 7.06-7.02 (2H, m), 6.90 (1H, d, J = 1.9 Hz), 2.62 (2H, t, J = 7.6 Hz), 1.43 (2H, q, J = 7.0 Hz), 0.66-0.60 (1H, m), 0.43-0.39 (2H, m), 0.03-0.00 (2H, m).

### (54d) [5-(2-Cyclopropylethyl)-3-(4-fluoxophenoxy)pyridin-2-yl]-[5-(1H-tetrazol-5-yl)pyridin-2-yl]amine

Analogously to Example (1e), the desired title compound (396 mg, 100 %) was obtained from the compound (356 mg, 0.95 mmol) obtained in Example (54c).
¹H-NMR (DMSO-d₆, 400 MHz) δ: 8.85 (1H, brs), 8.82 (1H, d, J = 2.0 Hz), 8.29 (1H, dd, J = 9.0, 2.4 Hz), 8.23 (1H, d, J = 8.6 Hz), 8.01 (1H, d, J = 1.6 Hz), 7.29-7.25 (2H, m), 7.18 (1H, d, J = 1.6 Hz), 7.18-7.14 (2H, m), 2.62 (2H, t, J = 7.6 Hz), 1.43 (2H, q, J = 7.1 Hz), 0.68-0.64 (1H, m), 0.39-0.34 (2H, m), 0.04-0.00 (2H, m);
MS (ESI, m/z): 418 (M+H)⁺.

### (Example 55)

### [5-(2-Cyclopropylethyl)-3-(2,4-difluorophenoxy)pyridin-2-yl]-[5-(1H-tetrazol-5-yl)pyridin-2-yl]amine

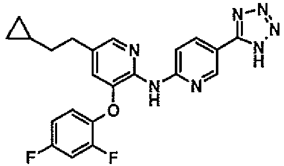

### (55a) 2-Amino-5-(cyclopropylethynyl)-3-(2,4-difluorophenoxy)pyridine

Analogously to Example (52a), the desired title compound (537 mg, 94 %) was obtained as a pale yellow solid from 2-amino-5-bromo-3-(2,4-difluorophenoxy)pyridine (602 mg, 2.0 mmol) obtained in Example (37b) and cyclopropylethyne (185 mg, 2.0 mmol).
¹H-NMR (CDCl₃, 400 MHz) δ: 7.88 (1H, d, J = 1.9 Hz), 7.09 (1H, dt, J = 13.8, 4.5 Hz), 7.01-6.96 (1H, m), 6.92-6.87 (1H, m), 6.76 (1H, d, J = 1.9 Hz), 4.88 (2H, brs), 1.42-1.35 (1H, m), 0.85-0.79 (2H, m), 0.78-0.72 (2H, m).

### (55b) 2-Amino-5-(2-cyclopropylethyl)-3-(2,4-difluorophenoxy)pyridine

Analogously to Example (52b), the desired title compound (491 mg, 90 %) was obtained as a colorless solid from the compound (536 mg, 1.87 mmol) obtained in Example (55a).
¹H-NMR (CDCl₃, 400 MHz) δ: 7.71 (1H, s), 7.06 (1H, dt, J = 13.6, 4.3 Hz), 7.04-6.98 (1H, m), 6.93-6.90 (1H, m), 6.74 (1H, s), 4.71 (2H, brs), 2.55 (2H, t, J = 7.4 Hz), 1.41 (2H, q, J = 7.0 Hz), 0.65-0.63 (1H, m), 0.43-0.38 (2H, m), 0.03-0.00 (2H, m).

### (55c) 6-[5-(2-Cyclopropylethyl)-3-(2,4-difluorophenoxy)pyridin-2-ylamino]nicotinonitrile

Analogously to Example (4d), the desired title compound (302 mg, 46 %) was obtained as a colorless solid from the compound (490 mg, 1.69 mmol) obtained in Example (55b).
¹H-NMR (CDCl₃, 400 MHz) δ: 8.72 (1H, d, J = 8.6 Hz), 8.53 (1H, d, J = 2.4 Hz), 8.18 (1H, brs), 7.91 (1H, d, J = 2.0 Hz), 7.88 (1H, dd, J = 6.2, 2.4 Hz), 7.16 (1H, dt, J = 13.8, 4.5 Hz), 7.05-7.00 (1H, m), 6.97-6.92 (1H, m), 6.76 (1H, d, J = 2.0 Hz), 2.60 (2H, t, J = 7.4 Hz), 1.42 (2H, q, J = 7.1 Hz), 0.62-0.60 (1H, m), 0.42-0.37 (2H, m), 0.01-0.02 (2H, m).

### (55d) [5-(2-Cyclopropylethyl)-3-(2,4-difluorophenoxy)pyridin-2-yl]-[5-(1H-tetrazol-5-yl)pyridin-2-yl]amine

Analogously to Example (1e), the desired title compound (314 mg, 94 %) was obtained from the compound (302 mg, 0.77 mmol) obtained in Example (55c).
¹H-NMR (DMSO-d₆, 400 MHz) δ: 9.00 (1H, brs), 8.86 (1H, d, J = 1.5 Hz), 8.32 (1H, dd, J = 8.6, 2.3 Hz), 8.25 (1H, d, J = 9.4 Hz), 8.00 (1H, d, J = 2.0 Hz), 7.57-7.51 (1H, m), 7.37 (1H, dt, J = 14.2, 4.5 Hz), 7.21-7.15 (1H, m), 7.09 (1H, d, J = 2.0 Hz), 2.62 (2H, t, J = 7.6 Hz), 1.42 (2H, q, J = 7.0 Hz), 0.67-0.63 (1H, m), 0.39-0.34 (2H, m), 0.04-0.00 (2H, m);
MS (ESI, m/z): 436 (M+H)⁺.

### (Example 56)

### [3-(2,4-Difluorophenoxy)-5-pentylpyridin-2-yl]-[5-(1H-tetrazol-5-yl)pyridin-2-yl]amine

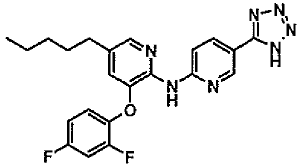

### (56a) 2-Amino-3-(2,4-difluorophenoxy)-5-(pent-1-ynyl)pyridine

Analogously to Example (52a), the desired title compound (570 mg, 99 %) was obtained as a pale yellow solid from 2-amino-5-bromo-3-(2,4-difluorophenoxy)pyridine (602 mg, 2.0 mmol) obtained in Example (37b) and 1-pentyne (191 mg, 2.8 mmol). ¹H-NMR (CDCl₃, 500 MHz) δ: 7.90 (1H, s), 7.13-7.07 (1H, m), 7.00-6.96 (1H, m), 6.92-6.86 (1H, m), 6.78 (1H, s), 4.87 (2H, brs), 2.32 (2H, t, J = 7.1 Hz), 1.60-1.54 (2H, m), 1.00 (3H, t, J = 7.6 Hz).

### (56b) 2-Amino-3-(2,4-difluorophenoxy)-5-pentylpyridine

Analogously to Example (52b), the desired title compound (300 mg, 52 %) was obtained as a colorless oil from the compound (569 mg, 1.97 mmol) obtained in Example (56a).
¹H-NMR (CDCl₃, 400 MHz) δ: 7.60 (1H, d, J = 1.6 Hz), 7.08 (1H, dt, J = 13.8, 4.5 Hz), 7.03-6.98 (1H, m), 6.93-6.89 (1H, m), 6.72 (1H, d, J = 1.6 Hz), 5.14 (2H, brs), 2.42 (2H, t, J = 7.8 Hz), 1.52-1.44 (2H, m), 1.32-1.22 (4H, m), 0.86 (3H, t, J = 7.1 Hz).

### (56c) 6-[3-(2,4-Difluorophenoxy)-5-pentylpyridin-2-ylamino]nicotinonitrile

Analogously to Example (4d), the desired title compound (224 mg, 55 %) was obtained as a colorless solid from the compound (299 mg, 1.02 mmol) obtained in Example (56b).
¹H-NMR (CDCl₃, 500 MHz) δ: 8.71 (1H, d, J = 8.8 Hz), 8.51 (1H, s), 8.17 (1H, brs), 7.88-7.87 (2H, m), 7.15 (1H, dt, J = 12.6, 4.5 Hz), 7.04-7.00 (1H, m), 6.96-6.93 (1H, m), 6.74 (1H, s), 2.49 (2H, t, J = 7.8 Hz), 1.53-1.49 (2H, m), 1.32-1.26 (4H, m), 0.87 (3H, t, J = 7.1 Hz).

### (56d) [3-(2,4-Difluorophenoxy)-5-pentylpyridin-2-yl]-[5-(1H-tetrazol-5-yl)pyridin-2-yl]amine

Analogously to Example (1e), the desired title compound (213 mg, 86 %) was obtained from the compound (223 mg, 0.57 mmol) obtained in Example (56c).
¹H-NMR (DMSO-d₆, 400 MHz) δ: 9.00 (1H, brs), 8.85 (1H, d, J = 1.6 Hz), 8.30 (1H, dd, J = 8.8, 2.6 Hz), 8.24 (1H, d, J = 8.6 Hz), 7.97 (1H, d, J = 2.0 Hz), 7.56-7.50 (1H, m), 7.36 (1H, dt, J = 14.1, 4.5 Hz), 7.19-7.15 (1H, m), 7.07 (1H, d, J = 2.0 Hz), 2.52 (2H, t, J = 7.0 Hz), 1.54-1.48 (2H, m), 1.31-1.18 (4H, m), 0.84 (3H, t, J = 7.1 Hz);
MS (ESI, m/z): 438 (M+H)⁺.

### (Example 57)

### [3-(4-Fluorophenoxy)-5-(trifluoromethyl)pyridin-2-yl]-[5-(1H-tetrazol-5-yl)pyridin-2-yl]amine

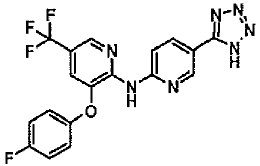

### (57a) [3-(4-Fluorophenoxy)-5-(trifluoromethyl)pyridine-2-carbonitrile

Analogously to Example (38a), the desired title compound (1.6 g, 93 %) was obtained from 4-fluorophenol (0.67 g, 6.0 mmol).
¹H-NMR (CDCl₃, 400 MHz) δ: 8.66-8.64 (1H, m), 7.34 (1H, s), 7.23-7.19 (2H, m), 7.16-7.12 (2H, m).

### (57b) [3-(4-Fluorophenoxy)-5-(trifluoromethyl)pyridine-2-carboxylic acid amide

Analogously to Example (38b), the desired title compound was quantitatively obtained from the compound (1.55 g, 5.5 mmol) obtained in Example (57a), which was used in the next step without further purification.

### (57c) 2-Amino-[3-(4-fluorophenoxy)-5-(trifluoromethyl)pyridine

Analogously to Example (1c), the desired title compound (1.3 g, 85 %) was obtained from the compound (1.7 g, 5.5 mmol) obtained in Example (57b).
¹H-NMR (CDCl₃, 400 MHz) δ: 8.08 (1H, dd, J = 0.8, 2.0 Hz), 7.13-7.08 (2H, m), 7.04-6.99 (3H, m), 5.14 (2H, brs).

### (57d) 6-[3-(4-Fluorophenoxy)-5-(trifluoromethyl)pyridin-2-ylamino]nicotinonitrile

Analogously to Example (1d), the desired title compound (0.90 g, 56 %) was obtained from the compound (1.2 g, 4.3 mmol) obtained in Example (57c).
¹H-NMR (DMSO-d₆, 400 MHz) δ: 9.76 (1H, s), 8.73 (1H, dd, J = 0.8, 2.4 Hz), 5.48-5.47 (1H, m), 8.28 (1H, dd, J = 0.8, 9.0 Hz), 8.22 (1H, dd, J = 2.4, 9.0 Hz), 7.42 (1H, d, J = 2.0 Hz), 7.33-7.24 (4H, m).

### (57e) [3-(4-Fluorophenoxy)-5-(trifluoromethyl)pyridin-2-yl]-[5-(1H-tetrazol-5-yl)pyridin-2-yl]amine

Analogously to Example (1e), the desired title compound (0.41 g, 52 %) was obtained from the compound (0.70 g, 1.9 mmol) obtained in Example (57d).
¹H-NMR (DMSO-d₆, 500 MHz) δ: 9.47 (1H, brs), 8.93 (1H, dd, J = 0.8, 2.4 Hz), 8.47-8.44 (2H, m), 8.39 (1H, dd, J = 2.4, 9.0 Hz), 7.39 (1H, d, J = 2.0 Hz), 7.34-7.27 (4H, m).
LCMS (ESI, m/z): 418 (M+H)⁺, retention time: 2.0 min.

### (Example 58)

### [3-(2,4-Difluorophenoxy)-5-cyclopropylpyridin-2-yl]-[5-(1H-tetrazol-5-yl)pyridin-2-yl]amine

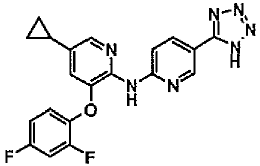

### (58a) 2-Amino-3-(2,4-difluorophenoxy)-5-cyclopropylpyridine

2-Amino-5-bromo-3-(2,4-difluorophenoxy)pyridine (0.30 g, 1.0 mmol) obtained in Example (37b) was dissolved in toluene-water (10:1, v/v, 6.6 mL), and cyclopropylboric acid (0.17 g, 2 mmol), sodium carbonate (0.32 g, 3 mmol) and tetrakis(triphenylphosphine)palladium(0) (0.12 g, 0.1 mmol) were added under nitrogen atmosphere, followed by heating to reflux for 7.5 hours. The reaction solution was poured into a saturated aqueous ammonium chloride solution, extraction was carried out with ethyl acetate, and the extract was washed with water and brine sequentially. The resulting organic layer was dried over anhydrous sodium sulfate and subsequently concentrated under reduced pressure to give a residue. The resulting residue was purified using silica gel column chromatography (hexane-ethyl acetate) to afford the desired title compound (0.18 g, 70 %).
¹H-NMR (CDCl₃, 400 MHz) δ: 7.68 (1H, d, J = 1.6 Hz), 7.04-6.95 (2H, m), 6.89-6.84 (1H, m), 6.59-6.57 (1H, m), 4.60 (2H, brs), 1.77-1.70 (1H, m), 0.87-0.82 (2H, m), 0.52-0.48 (2H, m).

### (58b) 6-[3-(2,4-Difluorophenoxy)-5-cyclopropylpyridin-2-ylamino]nicotinonitrile

Analogously to Example (1d), the desired title compound (0.12 g, 49 %) was obtained from the compound (0.17 g, 0.65 mmol) obtained in Example (58a).
¹H-NMR (DMSO-d₆, 500 MHz) δ: 9.41 (1H, s), 8.58 (1H, s), 8.06-8.04 (1H, m), 7.95 (1H, s), 7.86 (1H, d, J = 8.8 Hz), 7.50-7.46 (1H, m), 7.28-7.23 (1H, m), 7.13-7.09 (1H, m), 6.96 (1H, s), 1.95-1.89 (1H, m), 0.95-0.91 (2H, m), 0.67-0.64 (2H, m).

### (58c) [3-(2,4-Difluorophenoxy)-5-cyclopropylpyridin-2-yl]-[5-(1H-tetrazol-5-yl)pyridin-2-yl]amine

Analogously to Example (1e), the desired title compound (43 mg, 35 %) was obtained from the compound (0.11 g, 0.30 mmol) obtained in Example (58b).
¹H-NMR (CDCl₃, 400 MHz) δ: 8.92 (1H, brs), 8.81 (1H, dd, J = 0.8, 2.4 Hz), 8.26 (1H, dd, J = 2.4, 8.6 Hz), 8.16 (1H, dd, J = 0.8, 8.6 Hz), 7.93 (1H, d, J = 2.0 Hz), 7.53-7.47 (1H, m), 7.33-7.27 (1H, m), 7.16-7.11 (1H, m), 6.92 (1H, d, J = 2.0 Hz), 1.94-1.88 (1H, m), 0.94-0.89 (2H, m), 0.66-0.62 (2H, m).
MS (ESI, m/z): 408 (M+H)⁺.

### (Example 59)

### [3-(2-Chloro-4-fluorophenoxy)-5-(trifluoromethyl)pyridin-2-yl]-[5-(1H-tetrazol-5-yl)pyridin-2-yl]amine

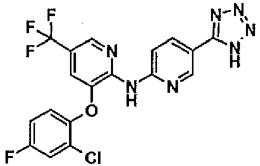

### (59a) [3-(2-Chloro-4-fluorophenoxy)-5-(trifluoromethyl)pyridine-2-carbonitrile

Analogously to Example (38a), the desired title compound (1.5 g, 93 %) was obtained from 2-chloro-4-fluorophenol (0.73 g, 5.0 mmol).
¹H-NMR (CDCl₃, 500 MHz) δ: 8.67 (1H, s), 7.35-7.33 (1H, m), 7.28-7.26 (1H, m), 7.16-7.14 (2H, m).

### (59b) [3-(2-Chloro-4-fluorophenoxy)-5-(trifluoromethyl)pyridine-2-carboxylic acid amide

Analogously to Example (38b), the desired title compound was quantitatively obtained from the compound (1.5 g, 4.6 mmol) obtained in Example (59a), which was used in the next step without further purification.

### (59c) 2-Amino-[3-(2-chloro-4-fluorophenoxy)-5-(trifluoromethyl)pyridine

Analogously to Example (1c), the desired title compound (0.69 g, 52 %) was obtained from the compound (1.5 g, 4.3 mmol) obtained in Example (59b).
¹H-NMR (CDCl₃, 500 MHz) δ: 8.09 (1H, s), 7.29-7.27 (1H, m), 7.12-7.04 (2H, m), 6.82 (1H, s), 5.20 (2H, brs).

### (59d) 6-[3-(2-Chloro-4-fluorophenoxy)-5-(trifluoromethyl)pyridin-2-ylamino]nicotinonitrile

Analogously to Example (1d), synthesis was carried out with the compound (0.69 g, 2.25 mmol) obtained in Example (59c), and further an ether-hexane mixed solvent was added to allow it to be suspended. The solid was purified by filtering off to afford the desired title compound (0.71 g, 77 %).
¹H-NMR (CDCl₃, 400 MHz) δ: 8.80 (1H, d, J = 9.0 Hz), 8.58 (1H, d, J = 2.4 Hz), 8.47 (1H, brs), 8.32 (1H, s), 7.97 (1H, dd, J = 2.4, 9.0 Hz), 7.32 (1H, dd, J = 2.7, 7.8 Hz), 7.21 (1H, dd, J = 4.7, 9.0 Hz), 7.15-7.10 (1H, m), 6.90 (1H, d, J = 2.0 Hz).

### (59e) [3-(2-Chloro-4-fluorophenoxy)-5-(trifluoromethyl)pyridin-2-yl]-[5-(1H-tetrazol-5-yl)pyridin-2-yl]amine

Analogously to Example (1e), the desired title compound (0.32 g, 42 %) was obtained from the compound (0.71 g, 1.7 mmol) obtained in Example (59d).
¹H-NMR (DMSO-d₆, 500 MHz) δ: 9.54 (1H, brs), 8.95 (1H, dd, J = 0.8, 2.4 Hz), 8.48-8.39 (2H, m), 8.41 (1H, dd, J = 2.4, 9.0 Hz), 7.71 (1H, dd, J = 3.1, 8.2 Hz), 7.49 (1H, dd, J = 5.1, 9.0 Hz), 7.37 (1H, ddd, J = 3.1, 8.2, 11.4 Hz), 7.27 (1H, d, J = 2.0 Hz). LCMS (ESI, m/z): 452 (M+H)⁺, retention time: 2.1 min.

### (Example 60)

### [3-(3,4-Difluorophenoxy)-5-(trifluoromethyl)pyridin-2-yl]-[5-(1H-tetrazol-5-yl)pyridin-2-yl]amine

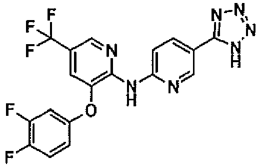

### (60a) [3-(3,4-Difluorophenoxy)-5-(trifluoromethyl)pyridine-2-carbonitrile

Analogously to Example (38a), the desired title compound (1.4 g, 92 %) was obtained from 3,4-difluorophenol (0.65 g, 5.0 mmol).
¹H-NMR (CDCl₃, 500 MHz) δ: 8.70 (1H, s), 7.40 (1H, s), 7.35-7.29 (1H, m), 7.06-7.02 (1H, m), 7.92-7.90 (1H, m).

### (60b) [3-(3,4-Difluorophenoxy)-5-(trifluoromethyl)pyridine-2-carboxylic acid amide

Analogously to Example (38b), the desired title compound was quantitatively obtained from the compound (1.5 g, 4.6 mmol) obtained in Example (60a), which was used in the next step without further purification.

### (60c) 2-Amino-[3-(3,4-difluorophenoxy)-5-(trifluoromethyl)pyridine

Analogously to Example (1c), the desired title compound (0.67 g) was obtained as a mixture of impurities from the compound (1.4 g, 4.2 mmol) obtained in Example (60b), which was used in the next step without further purification.

### (60d) 6-[3-(3,4-Difluorophenoxy)-5-(trifluoromethyl)pyridin-2-ylamino]nicotinonitrile

Analogously to Example (1d), synthesis was carried out with the compound (0.67 g) obtained in Example (60c), and further an ether-hexane mixed solvent was added to allow it to be suspended. The solid was purified by filtering off to afford the desired title compound (0.59 g, 66 %).
¹H-NMR (DMSO-d₆, 400 MHz) δ: 8.79 (1H, dd, J = 0.8, 9.0 Hz), 8.57 (1H, dd, J = 0.8, 2.4 Hz), 8.36-8.35 (2H, m), 7.98-7.95 (1H, m), 7.31-7.24 (1H, m), 7.17 (1H, d, J = 2.0 Hz), 7.00 (1H, ddd, J = 2.4, 6.3, 10.2 Hz), 6.90-6.86 (1H, m).

### (60e) [3-(3,4-Difluorophenoxy)-5-(trifluoromethyl)pyridin-2-yl]-[5-(1H-tetrazol-5-yl)pyridin-2-yl]amine

Analogously to Example (1e), the desired title compound (0.23 g, 35 %) was obtained from the compound (0.59 g, 1.5 mmol) obtained in Example (60d).
¹H-NMR (DMSO-d₆, 500 MHz) δ: 9.55 (1H, s), 8.92 (1H, dd, J = 0.8, 3.1 Hz), 8.50-8.49 (1H, m), 8.42 (1H, dd, J = 1.2, 9.0 Hz), 8.38 (1H, dd, J = 2.0, 8.6 Hz), 7.60 (1H, d, J = 1.6 Hz), 7.57-7.49 (1H, m), 7.48-7.42 (1H, m), 7.09-7.05 (1H, m).
LCMS (ESI, m/z): 436 (M+H)⁺, retention time: 2.1 min.

### (Example 61)

### [5-n-Propyl-3-(2,4-difluorophenoxy)pyridin-2-yl]-[5-(1H-tetrazol-5-yl)pyridin-2-yl]amine

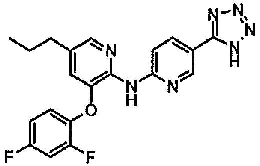

### (61a) 2-Amino-3-(2,4-difluorophenoxy)-5-n-propylpyridine

2-Amino-3-(2,4-difluorophenoxy)-5-bromopyridine (0.90 g, 3.0 mmol) obtained in Example (37b), palladium acetate (II) (67 mg, 0.3 mmol), and 2-dicyclohexylphosphino-2',6'-dimethoxybiphenyl (0.25 g, 0.6 mmol) were dissolved in tetrahydrofuran (12 mL) under nitrogen atmosphere and n-propyl zinc bromide (12 mL, a 0.5M tetrahydrofuran solution, Aldrich) was added dropwise, followed by stirring at room temperature for 18 hours. After the reaction, the reaction was quenched by an aqueous ammonium chloride solution, and subsequently extraction was carried out with ethyl acetate. The resulting organic layer was dried over anhydrous sodium sulfate and subsequently concentrated under reduced pressure to give a residue. The resulting residue was purified using silica gel column chromatography (hexane-ethyl acetate) to afford the desired title compound (0.45 g, 57 %).
¹H NMR (CDCl₃, 400 MHz) δ: 7.67 (1H, d, J = 2.0 Hz), 7.06-6.95 (2H, m), 6.89-6.84 (1H, m), 6.69 (1H, d, J = 1.6 Hz), 4.61 (2H, brs), 2.40 (2H, t, J = 7.4 Hz), 1.56-1.46 (2H, m), 0.88 (3H, t, J = 7.0 Hz).

### (61b) 6-[3-(2,4-Difluorophenoxy)-5-n-propylpyridin-2-ylamino]nicotinonitrile

Analogously to Example (1d), synthesis was carried out with the compound (0.44 g, 1.7 mmol) obtained in Example (61a) to afford the desired title compound (0.44 g, 73 %).
¹H NMR (CDCl₃, 400 MHz) δ: 8.71 (1H, dd, J = 0.8, 9.0 Hz), 8.51 (1H, dd, J = 0.8, 2.4 Hz), 8.17 (1H, s), 7.88 (2H, dt, J = 2.7, 5.1 Hz), 7.15 (1H, dt, J = 5.1, 8.6 Hz), 7.05-6.99 (1H, m), 6.97-6.92 (1H, m), 6.74 (1H, s), 2.48 (2H, t, J = 7.4 Hz), 1.57-1.50 (2H, m), 0.90 (3H, t, J = 7.4 Hz).

### (61c) [5-n-Propyl-3-(2,4-difluorophenoxy)pyridin-2-yl]-[5-(1H-tetrazol-5-yl)pyridin-2-yl]amine

Analogously to Example (1e), the desired title compound (0.26 g, 53 %) was obtained from the compound (0.44 g, 1.2 mmol) obtained in Example (61b).
¹H NMR (DMSO-d₆, 400 MHz) δ: 9.04 (1H, brs), 8.84 (1H, dd, J = 0.8, 2.4 Hz), 8.30 (1H, dd, J = 2.4, 9.0 Hz), 8.23 (1H, dd, J = 0.8, 9.0 Hz), 7.96 (1H, d, J = 1.6 Hz), 7.54-7.49 (1H, m), 7.35 (1H, dt, J = 5.5, 9.0 Hz), 7.18-7.13 (1H, m), 7.03 (1H, d, J = 1.2 Hz), 2.50-2.47 (2H, m), 1.52 (2H, sext, J = 7.4 Hz), 0.86 (3H, t, J = 7.0 Hz).
LCMS (ESI, m/z): 410 (M+H)⁺, retention time: 2.0 min.

### (Example 62)

### [5-(2-Methylpropyl)-3-(2,4-difluorophenoxy)pyridin-2-yl]-[5-(1H-tetrazol-5-yl)pyridin-2-yl]amine

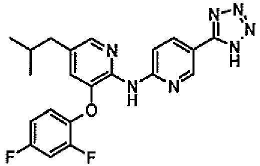

### (62a) 2-Amino-3-(2,4-difluorophenoxy)-5-(2-methylpropyl)pyridine

In a similar manner to Example (61a), the desired title compound (0.56 g, 67 %) was obtained from 2-amino-3-(2,4-difluorophenoxy)-5-bromopyridine (0.90 g, 3.0 mmol) obtained in Example (37b) and 2-methylpropyl zinc bromide (12 mL, a 0.5M tetrahydrofuran solution, Aldrich).
¹H NMR (CDCl₃, 400 MHz) δ: 7.64 (1H, d, J = 2.0 Hz), 7.05-6.95 (2H, m), 6.89-6.84 (1H, m), 6.66 (1H, d, J = 1.2 Hz), 4.61 (2H, brs), 2.29 (2H, d, J = 7.4 Hz), 1.74-1.64 (1H, m), 0.85 (6H, d, J = 6.7 Hz).

### (62b) 6-[3-(2,4-Difluorophenoxy)-5-(2-methylpropyl)pyridin-2-ylamino]nicotinonitrile

Analogously to Example (1d), synthesis was carried out with the compound (0.55 g, 2.0 mmol) obtained in Example (62a) to afford the desired title compound (0.54 g, 71 %).
¹H NMR (CDCl₃, 400 MHz) δ: 8.72 (1H, dd, J = 0.8, 8.6 Hz), 8.51 (1H, dd, J = 0.8, 2.4 Hz), 8.18 (1H, s), 7.88 (1H, ddd, J = 0.8, 2.4, 9.0 Hz), 7.84 (1H, d, J = 2.0 Hz), 7.15 (1H, dt, J = 5.1, 9.0 Hz), 7.04-6.99 (1H, m), 6.97-6.91 (1H, m), 6.71 (1H, s), 2.36 (2H, d, J = 7.0 Hz), 1.78-1.68 (1H, m), 0.87 (6H, d, J = 6.7 Hz).

### (62c) [5-(2-Methylpropyl)-3-(2,4-difluorophenoxy)pyridin-2-yl]-[5-(1H-tetrazol-5-yl)pyridin-2-yl]amine

Analogously to Example (1e), the desired title compound (0.29 g, 48 %) was obtained from the compound (0.53 g, 1.4 mmol) obtained in Example (62b).
¹H NMR (DMSO-d₆, 400 MHz) δ: 9.05 (1H, brs), 8.84 (1H, dd, J = 1.2, 2.4 Hz), 8.30 (1H, dd, J = 2.4, 9.0 Hz), 8.25 (1H, dd, J = 0.8, 8.6 Hz), 7.92 (1H, d, J = 2.0 Hz), 7.55-7.49 (1H, m), 7.35 (1H, dt, J = 5.5, 9.0 Hz), 7.19-7.13 (1H, m), 7.04 (1H, d, J = 1.2 Hz), 2.39 (2H, d, J = 7.0 Hz), 1.79-1.69 (1H, m), 0.83 (6H, d, J = 6.5 Hz).
LCMS (ESI, m/z): 424 (M+H)⁺, retention time: 2.1 min.

### (Example 63)

### [5-(1-Methylethyl)-3-(2,4-difluorophenoxy)pyridin-2-yl]-[5-(1H-tetrazol-5-yl)pyridin-2-yl]amine

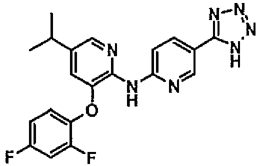

### (63a) 2-Amino-3-(2,4-difluorophenoxy)-5-(1-methylethyl)pyridine

In a similar manner to Example (61a), the desired title compound was obtained as a mixture with the compound of Example (61a) (0.59 g, 75 %, purity 60%)from 2-amino-3-(2,4-difluorophenoxy)-5-bromopyridine (0.90 g, 3.0 mmol) obtained in Example (37b) and 2-propyl zinc bromide (12 mL, a 0.5M tetrahydrofuran solution, Aldrich).
¹H NMR (CDCl₃, 400 MHz) δ: 7.72 (1H, d, J = 2.0 Hz), 7.05-6.95 (2H, m), 6.89-6.85 (1H, m), 6.75 (1H, d, J = 1.6 Hz), 4.69 (2H, brs), 2.82-2.72 (1H, m), 1.15 (6H, d, J = 7.0 Hz).

### (63b) 6-[3-(2,4-Difluorophenoxy)-5-(1-methylethyl)pyridin-2-ylamino]nicotinonitrile

Analogously to Example (1d), synthesis was carried out with the compound (0.59 g, 2.2 mmol) obtained in Example (63a) to afford the desired title compound as a mixture of the compound of Example (61b) (0.60 g, 73 %, purity 60%).
LCMS (ESI, m/z): 367 (M+H)⁺, retention time: 2.9 min.

### (63c) [5-(1-Methylethyl)-3-(2,4-difluorophenoxy)pyridin-2-yl]-[5-(1H-tetrazol-5-yl)pyridin-2-yl]amine

Analogously to Example (1e), the desired title compound was obtained as a mixture of the compound of Example (61c) (0.41 g, 60 %, purity 60%) from the compound (0.60g, 1.6 mmol) obtained in Example (63b).
LCMS (ESI, m/z): 410 (M+H)⁺, retention time: 2.1 min.

### (Example 64)

### [5-Butyl-3-(2,4-difluorophenoxy)pyridin-2-yl]-[5-(1H-tetrazol-5-yl)pyridin-2-yl]amine

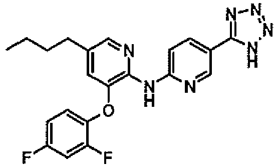

### (64a) 2-Amino-5-butyl-3-(2,4-difluorophenoxy)pyridine

2-Amino-5-bromo-3-(2,4-difluorophenoxy)pyridine (0.50 g, 1.66 mmol) synthesized in Example (37b) and sodium carbonate (351 mg, 3.32 mmol) were dissolved in water (1.6 mL) and toluene (7 mL) under a nitrogen stream, and a 1M diethyl ether solution of tributylboron (3.3 mL, 3.3 mmol) was added dropwise. Further, tetrakis(triphenylphosphine)palladium (30 mg, 0.037 mmol) was added, followed by stirring for 8 hours with heating to reflux. After the reaction was quenched by saturated brine, extraction was carried out with ethyl acetate. The organic layer was dried over anhydrous sodium sulfate and subsequently concentrated under reduced pressure to give a residue. The resulting residue was purified using silica gel column chromatography to afford the desired title compound (269 mg, 58 %).
¹H-NMR (CDCl₃, 400 MHz) δ: 7.69 (1H, d, J = 1.6Hz), 6.96-7.07 (2H, m), 6.65-6.91 (1H, m), 6.69 (1H, s), 4.95 (2H, brs), 2.42 (2H, d, J = 7.8 Hz), 1.42-1.49 (2H, m), 1.24-1.36 (2H, m), 0.88 (3H, t, J = 7.4 Hz).

### (64b) 6-[5-Butyl-3-(2,4-difluorophenoxy)pyridin-2-ylamino]nicotinonitrile

Analogously to Example (1d), the desired title compound (174.4 mg, 31 %) was obtained from the compound (269 mg, 0.967 mmol) obtained in Example (64a).
¹H-NMR (CDCl₃, 400 MHz) δ: 8.71 (1H, d, J = 9.0Hz), 8.51-8.52 (1H, m), 8.17 (1H, s), 7.87-7.89 (2H, m), 7.12-7.18 (1H, m), 6.92-7.05 (2H, m), 6.74 (1H, s), 2.50 (2H, t, J = 7.6 Hz), 1.46-1.55 (2H, m), 1.27-1.36 (2H, m), 0.90 (3H, t, J = 7.4 Hz).

### (64c) [5-Butyl-3-(2,4-difluorophenoxy)pyridin-2-yl]-[5-(1H-tetrazol-5-yl)pyridin-2-yl]amine

Analogously to Example (1e), the desired title compound (37 mg, 60 %) was obtained from the compound (50 mg, 0.15 mmol) obtained in Example (64b).
¹H-NMR (DMSO-d₆, 400 MHz) δ: 9.04 (1H, s), 8.84 (1H, d, J = 2.4Hz), 8.28 (1H, dd, J = 2.4, 9.0Hz), 8.22 (1H, d, J = 9.0Hz), 7.96 (1H, d, J = 1.6Hz), 7.49-7.55 (1H, m), 7.32-7.37 (1H, m), 7.14-7.19 (1H, m), 7.08 (1H, s), 2.51-2.59 (2H, m), 1.48 (2H, quint, J = 7.4Hz), 1.27 (2H, hept, J = 7.4Hz), 0.87 (3H, t, J = 7.4 Hz);
LCMS (ESI, m/z): 435 (M+H)⁺, retention time: 2.1 min.

### (Example 65)

### [3-(2,4-Difluorophenoxy)-5-(3-methoxy-1-methylpropylsulfanyl)pyridin-2-yl]-[5-(1H-tetrazol-5-yl)pyridin-2-yl]amine trifluoroacetate

The compound (101.0 mg) obtained in Example (44b) was separated by a high-pressure liquid chromatography system (SCL-10A/CTO-10AC/LC-10AD) manufactured by Shimadzu Corporation using a Chiralpak AD-H column manufactured by Daicel Chemical Industries, Ltd. (n-hexane:ethanol:diethylamine:trifluoroacetic acid = 80:20:0.1:0.1, 1 mL/min, column size 0.46 cm I.D. x 15 cm, 40°C). After the solvent at peak 1 (retention time: 11.8 minutes) was distilled off under reduced pressure, water and acetic acid were added to the residue, followed by stirring at room temperature. The resulting solid was filtered off, washed with water, and subsequently dried to afford the desired title compound (43.4 mg).
¹H-NMR (DMSO-d₆, 400 MHz) δ: 8.91 (1H, d, J=2.4Hz), 8.38 (1H, dd, J=2.4, 9.0Hz), 8.30 (1H, d, J=9.0Hz), 8.12 (1H, d, J=2.0Hz), 7.54-7.58 (1H, m), 7.41-7.46 (1H, m), 7.22-7.17 (1H, m), 7.19 (1H, d, J=4.0Hz), 3.34-3.45 (2H, m), 3.18-3.24 (1H, m), 3.16 (3H, s), 1.49-1.72 (2H, m), 1.18 (3H, d, J = 7.0 Hz).

The desired title compound (45.3 mg) was obtained from peak 2 (retention time: 14.4 minutes).
¹H-NMR (DMSO-d₆, 400 MHz) δ: 8.91 (1H, d, J=2.4Hz), 8.38 (1H, dd, J=2.4, 9.0Hz), 8.30 (1H, d, J=9.0Hz), 8.12 (1H, d, J=2.0Hz), 7.54-7.58 (1H, m), 7.41-7.46 (1H, m), 7.22-7.17 (1H, m), 7.19 (1H, d, J=4.0Hz), 3.34-3.45 (2H, m), 3.18-3.24 (1H, m), 3.16 (3H, s), 1.49-1.72 (2H, m), 1.18 (3H, d, J = 7.0 Hz).

### (Test Example 1)

### (1) GK Preparation

cDNA encoding human pancreatic GK polypeptide (GenBank Accession No. NM_000162, human glucokinase variant 1) was cloned from a human cDNA library by polymerase chain reaction (PCR), and introduced into a glutathione S-transferase (GST)-fused protein expression vector (GEX4T, GE Healthcare Bioscience).
The vector was introduced into Escherichia coli (such as BL21, Invitrogen), and the transformed E. coli was cultured overnight at 37°C followed by recovery of the cells. After freezing and thawing the recovered cells, the cells were suspended in phosphate buffer containing Triton-X at a final concentration of 1% followed by disrupting the cells with an ultrasonic homogenizer. The supernatant obtained by low-speed centrifugation treatment of the homogenate (10,000 x g, 30 minutes) was further subjected to high-speed centrifugation (100,000 x g, 10 minutes) followed by recovering the supernatant and purifying the fused protein using a GST fused protein purification system (Bulk GST Purification Module, GE Healthcare Bioscience). The GK fused protein was divided into smaller aliquots and stored at -80°C.

### (2) GK Activity Test

GK activity was measured using the GK purified in (1) above. More specifically, an enzyme solution was prepared by adding the purified GK of (1) above and glucose-6-phosphate dehydrogenase (Sigma) to solution 1 of a glucose assay kit (D-Glucose UV Method, Roche Diagnostics). The enzyme solution, a test compound, diluent and glucose (final concentration: 5 mM) were mixed in a 96-well ELISA plate, and allowed to react for 30 minutes at room temperature. Following completion of the reaction, absorbance at a wavelength of 340 nm was measured using SpectraMax Plus (Molecular Probe). Furthermore, unreacted (when glucose was not added) absorbance was used for the background.

GK activation ratios were calculated with values represented by the following numerical formula: (absorbance after reacting for 30 minutes when test compound added)/(absorbance after reacting for 30 minutes when test compound not added). The compounds of Examples 1 to 65 demonstrated GK activation ratios equal to 1.4 times or more at a test compound concentration of 10 µM.

**Preparation Example 1: Capsule**

| | |
|---|---|
| Compound of Example 1 | 50 mg |
| Lactose | 128 mg |
| Cornstarch | 70 mg |
| Magnesium stearate | 2 mg |
| | 250 mg |

Powders of the above formulation were mixed and passed through a 60 mesh sieve followed by filling the powders into a 250 mg gelatin capsule to obtain a capsule.

**Preparation Example 2: Tablet**

| | |
|---|---|
| Compound of Example 1 | 50 mg |
| Lactose | 126 mg |
| Cornstarch | 23 mg |
| Magnesium stearate | 1 mg |
| | 200 mg |

Powders of the above formulation were mixed, granulated using cornstarch paste and dried, followed by forming into tablets with a tableting machine to obtain a 200 mg tablet. This tablet can be provided with a sugar coating as necessary.

### INDUSTRIAL APPLICABILITY

A compound represented by general formula (I) of the present invention, or a pharmacologically acceptable salt thereof, has superior GK activating activity, and is useful as a therapeutic or preventive (and particularly a therapeutic) for diabetes, impaired glucose tolerance, gestational diabetes, chronic complications of diabetes (including diabetic peripheral neuropathy, diabetic nephropathy, diabetic retinopathy and diabetic macroangiopathy) or metabolic syndrome (and particularly diabetes or impaired glucose tolerance) for use in warm-blooded animals (and particularly humans).

## Claims

1. A compound represented by the general formula (I): 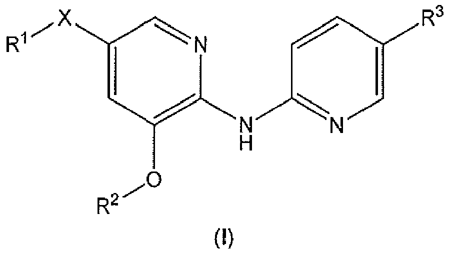 [wherein,
R¹ represents a C₆-C₁₀ aryl group that may be substituted with 1 to 5 group(s) independently selected from Substituent Group A, a heterocyclic group that may be substituted with 1 to 4 group(s) independently selected from Substituent Group A, a C₃-C₆ cycloalkyl group that may be substituted with 1 to 4 group(s) independently selected from Substituent Group A or a C₁-C₆ alkyl group that may be substituted with 1 to 5 group(s) independently selected from Substituent Group B,
X represents a single bond, an oxygen atom, a sulfur atom or a group represented by the formula -N(R⁴)- (wherein R⁴ represents a hydrogen atom or a C₁-C₆ alkyl group),
with the proviso that the case in which X represents a single bond, and in which R¹ represents a C₆-C₁₀ aryl group that may be substituted with 1 to 5 group(s) independently selected from Substituent Group A or a heterocyclic group that may be substituted with 1 to 4 group(s) independently selected from Substituent Group A is excluded,
R² represents a C₆-C₁₀ aryl group that may be substituted with 1 to 5 group(s) independently selected from Substituent Group A or a heterocyclic group that may be substituted with 1 to 4 group(s) independently selected from Substituent Group A,
R³ represents a 1H-tetrazol-5-yl group or a 5-oxo-4,5-dihydro-[1,2,4]oxadiazol-3-yl group,
Substituent Group A represents the group of substituents selected from a halogen atom, a C₁-C₆ alkyl group, a C₁-C₆ halogenated alkyl group, a hydroxy group, a C₁-C₆ hydroxyalkyl group, a C₁-C₆ alkoxy group, a carboxyl group, a C₂-C₇ alkylcarbonyl group, a C₂-C₇ alkoxycarbonyl group, a C₂-C₇ alkylcarbonyloxy group, a cyano group, a nitro group, an amino group, a mono-C₁-C₆ alkylamino group, a di-(C₁-C₆ alkyl)amino group, a C₁-C₆ alkylsulfanyl group, a C₁-C₆ alkylsulfinyl group, a C₁-C₆ alkylsulfonyl group, a group represented by the formula-C(=O)-NR⁵R⁶ (wherein R⁵ and R⁶ may be the same or different and respectively represent a hydrogen atom or a C₁-C₆ alkyl group), and a group represented by the formula -NR⁷R⁸ (wherein R⁷ represents a hydrogen atom or a C₁-C₆ alkyl group, and R⁸ represents a C₂-C₇ alkylcarbonyl group, a C₁-C₆ alkylsulfinyl group or a C₁-C₆ alkylsulfonyl group), and
Substituent Group B represents the group of substituents selected from a halogen atom, a C₃-C₆ cycloalkyl group that may be substituted with one C₁-C₆ hydroxyalkyl group, a hydroxy group, a C₁-C₆ alkoxy group, a C₂-C₇ alkylcarbonyl group, a C₂-C₇ alkoxycarbonyl group, an amino group, a mono-C₁-C₆ alkylamino group, a di-(C₁-C₆ alkyl)amino group, a C₆-C₁₀ aryl group that may be substituted with 1 to 5 group(s) independently selected from Substituent Group A, and a heterocyclic group that may be substituted with 1 to 4 group(s) independently selected from Substituent Group A]
or a pharmacologically acceptable salt thereof.

2. The compound or pharmacologically acceptable salt thereof according to claim 1, wherein Substituent Group A is the group of substituents selected from a halogen atom, a C₁-C₆ alkyl group, a C₁-C₆ halogenated alkyl group, a C₁-C₆ hydroxyalkyl group, a C₂-C₇ alkoxycarbonyl group, a C₁-C₆ alkylsulfonyl group, and a group represented by the formula -C(=O)-NR⁵R⁶.

3. The compound or pharmacologically acceptable salt thereof according to claim 1 or 2, wherein Substituent Group B is the group of substituents selected from a halogen atom, a C₃-C₆ cycloalkyl group that may be substituted with one C₁-C₆ hydroxyalkyl group, a hydroxy group, a C₁-C₆ alkoxy group, a C₂-C₇ alkoxycarbonyl group, and a di-(C₁-C₆ alkyl)amino group.

4. The compound or pharmacologically acceptable salt thereof according to any one of claims 1 to 3, wherein R¹ is a pyridyl group that may be substituted with 1 to 3 group(s) independently selected from Substituent Group A, a pyrimidinyl group that may be substituted with 1 to 3 group(s) independently selected from Substituent Group A or a C₁-C₆ alkyl group that may be substituted with 1 to 5 group(s) independently selected from Substituent Group B.

5. The compound or pharmacologically acceptable salt thereof according to any one of claims 1 to 3, wherein R¹ is a 2-pyridyl group that may be substituted with 1 or 2 group(s) independently selected from the group consisting of (a C₁-C₆ alkyl group, a C₁-C₆ halogenated alkyl group, a C₁-C₆ hydroxyalkyl group and a C₂-C₇ alkoxycarbonyl group), a 2-pyrimidinyl group, or a C₁-C₆ alkyl group that may be substituted with 1 to 3 group(s) independently selected from the group consisting of (a halogen atom, a C₃-C₆ cycloalkyl group which may be substituted with one C₁-C₆ hydroxyalkyl group, a hydroxy group, a C₁-C₆ alkoxy group, a C₂-C₇ alkoxycarbonyl group and a di-(C₁-C₆ alkyl) amino group).

6. The compound or pharmacologically acceptable salt thereof according to any one of claims 1 to 3, wherein R¹ is a 2-pyridyl group, a 5-hydroxymethyl-2-pyridyl group, a 5-ethoxycarbonyl-2-pyridyl group, a 2-pyrimidinyl group, an isopropyl group, a cyclopropylmethyl group, a trifluoromethyl group, a 3-hydroxypropyl group, a 3-methoxypropyl group, a 3-methoxy-1-methylpropyl group, a 2-fluoro-3-methoxypropyl group or a 4-methoxybutyl group.

7. The compound or pharmacologically acceptable salt thereof according to any one of claims 1 to 3, wherein R¹ is a cyclopropyl group or a 2-cyclopropylethyl group.

8. The compound or pharmacologically acceptable salt thereof according to any one of claims 1 to 7, wherein X is a single bond or a sulfur atom.

9. The compound or pharmacologically acceptable salt thereof according to any one of claims 1 to 8, wherein R² is a phenyl group that may be substituted with 1 to 3 group(s) independently selected from Substituent Group A.

10. The compound or pharmacologically acceptable salt thereof according to any one of claims 1 to 8, wherein R² is a phenyl group that is substituted with 1 or 2 group(s) independently selected from the group consisting of (a halogen atom, a C₁-C₆ halogenated alkyl group, a C₁-C₆ alkylsulfonyl group and a group represented by the formula -C(=O)-NR⁵R⁶).

11. The compound or pharmacologically acceptable salt thereof according to any one of claims 1 to 8, wherein R² is a 4-fluorophenyl group, a 2,4-difluorophenyl group, a 3,4-difluorophenyl group or a 2-chloro-4-fluorophenyl group.

12. The compound or pharmacologically acceptable salt thereof according to any one of claims 1 to 11, wherein R³ is a 1H-tetrazol-5-yl group.

13. A compound or a pharmacologically acceptable salt thereof, which is:
[3-(2,4-difluorophenoxy)-5-(pyridin-2-ylsulfanyl)pyridin-2-yl]-[5-(1H-tetrazol-5-yl)pyridin-2-yl]amine,
ethyl 6-{5-(3,4-difluorophenoxy)-6-[5-(1H-tetrazol-5-yl)pyridin-2-ylamino]pyridin-3-ylsulfanyl}nicotinate,
6-{5-(3,4-difluorophenoxy)-6-[5-(1H-tetrazol-5-yl)pyridin-2-ylamino]pyridin-3-ylsulfanyl}pyridin-3-ylmethanol,
[3-(4-fluorophenoxy)-5-[(3-methoxypropylsulfanyl)pyridin-2-yl]-[5-(1H-tetrazol-5-yl)pyridin-2-yl]amine,
[5-(cyclopropylmethylsulfanyl)-3-(4-fluorophenoxy)pyridin-2-yl]-[5-(1H-tetrazol-5-yl)pyridin-2-yl]amine,
[3-(3,4-difluorophenoxy)-5-(3-methoxypropylsulfanyl)pyridin-2-yl]-[5-(1H-tetrazol-5-yl)pyridin-2-yl]amine,
[3-(2,4-difluorophenoxy)-5-trifluoromethylpyridin-2-yl]-[5-(1H-tetrazol-5-yl)pyridin-2-yl]amine,
[3-(2,4-difluorophenoxy)-5-(3-methoxypropylsulfanyl)pyridin-2-yl]-[5-(1H-tetrazol-5-yl)pyridin-2-yl]amine, or
[3-(2,4-difluorophenoxy)-5-(3-methoxy-1-methylpropylsulfanyl)pyridin-2-yl]-[5-(1H-tetrazol-5-yl)pyridin-2-yl]amine.

14. A compound or a pharmacologically acceptable salt thereof, which is:
[3-(2,4-difluorophenoxy)-5-(4-methoxybutyl)pyridin-2-yl]-[5-(1H-tetrazol-5-yl)pyridin-2-yl]amine,
[5-(2-cyclopropylethyl)-3-(2,4-difluorophenoxy)pyridin-2-yl]-[5-(1H-tetrazol-5-yl)pyridin-2-yl]amine,
[3-(4-fluorophenoxy)-5-(trifluoromethyl)pyridin-2-yl]-[5-(1H-tetrazol-5-yl)pyridin-2-yl]amine,
[3-(2,4-difluorophenoxy)-5-cyclopropylpyridin-2-yl]-[5-(1H-tetrazol-5-yl)pyridin-2-yl]amine,
[3-(2-chloro-4-fluorophenoxy)-5-(trifluoromethyl)pyridin-2-yl]-[5-(1H-tetrazol-5-yl)pyridin-2-yl]amine, or
[3-(3,4-difluorophenoxy)-5-(trifluoromethyl)pyridin-2-yl]-[5-(1H-tetrazol-5-yl)pyridin-2-yl]amine.

15. A pharmaceutical composition containing as an active ingredient thereof a compound or pharmacologically acceptable salt thereof according to any one of claims 1 to 14.

16. The pharmaceutical composition according to claim 15, wherein the pharmaceutical composition has glucokinase activating activity.

17. The pharmaceutical composition according to claim 15, wherein the pharmaceutical composition is for treating and/or
preventing a disease that is treatable and/or preventable by glucokinase activating activity.

18. The pharmaceutical composition according to claim 15, wherein the pharmaceutical composition is for treating and/or preventing a disease for which the symptoms thereof are treated, improved, diminished and/or prevented by activation of glucokinase and maintenance of glucose homeostasis or regulation of blood glucose level.

19. The pharmaceutical composition according to claim 15, wherein the pharmaceutical composition is for treating and/or preventing diabetes, impaired glucose tolerance, gestational diabetes, chronic complications of diabetes (including diabetic peripheral neuropathy, diabetic nephropathy, diabetic retinopathy and diabetic macroangiopathy) or metabolic syndrome.

20. The pharmaceutical composition according to claim 15, wherein the pharmaceutical composition is for treating and/or preventing diabetes or impaired glucose tolerance.

21. A glucokinase activator containing as an active ingredient thereof a compound or pharmacologically acceptable salt thereof according to any one of claims 1 to 14.

22. A use of a compound or pharmacologically acceptable salt thereof according to any one of claims 1 to 14, for producing a pharmaceutical composition.

23. The use according to claim 22, wherein the pharmaceutical composition is a composition for activating glucokinase.

24. The use according to claim 22, wherein the pharmaceutical composition is a composition for treating and/or preventing diabetes, impaired glucose tolerance, gestational diabetes, chronic complications of diabetes (including diabetic peripheral neuropathy, diabetic nephropathy, diabetic retinopathy and diabetic macroangiopathy) or metabolic syndrome.

25. A glucokinase activation method, comprising administering a pharmacologically effective amount of a compound or pharmacologically acceptable salt thereof according to any one of claims 1 to 14 to a warm-blooded animal.

26. A method for treating and/or preventing a disease, comprising administering a pharmacologically effective amount of a compound or pharmacologically acceptable salt thereof according to any one of claims 1 to 14 to a warm-blooded animal.

27. The method according to claim 26, wherein the disease is diabetes, impaired glucose tolerance, gestational diabetes, chronic complications of diabetes (including diabetic peripheral neuropathy, diabetic nephropathy, diabetic retinopathy and diabetic macroangiopathy) or metabolic syndrome.

28. The method according to any one of claims 25 to 27, wherein the warm-blooded animal is a human.
